# EUROPEAN PATENT APPLICATION

(11) **EP 4 403 191 A1**
(43) Date of publication of application: **24.07.2024**
(21) Application number: 22870023.3
(22) Date of filing: 15.09.2022
(51) Int. Cl.: A61K 48/00, A61P 43/00, A61K 47/54, A61K 47/64, C12N 15/113, A61K 31/713, A61P 25/00

(54) **HETERONUCLEIC ACID INCLUDING 2'-MODIFIED NUCLEOSIDE**

(30) Priority: 15.09.2021 JP 2021150310
(71) Applicant: National University Corporation Tokyo Medical and Dental University, Tokyo 113-8510 (JP)
(72) Inventor: YOKOTA Takanori, Tokyo 113-8510 (JP); YOSHIOKA Kotaro, Tokyo 113-8510 (JP)
(74) Representative: Mewburn Ellis LLP
(86) International application number: PCT/JP2022/034531
(87) International publication number: WO 2023/042876

(57) **Abstract**

A problem to be addressed is to provide a double-stranded nucleic acid complex with reduced central nervous system toxicity. Provided is a double-stranded nucleic acid complex comprising a first nucleic acid strand and a second nucleic acid strand, wherein: the first nucleic acid strand is capable of hybridizing to at least part of a target gene or a transcription product thereof, and has an antisense effect on the target gene or a transcription product thereof, and the second nucleic acid strand comprises a base sequence complementary to the first nucleic acid strand, and comprises one or more 2'-modified nucleoside.

## Description

### Technical Field

The present invention relates to a double-stranded nucleic acid complex comprising a 2'-modified nucleoside, and a pharmaceutical composition comprising the same as an active ingredient, and the like.

### Background Art

In recent years, an oligonucleotide has been drawing attention in the ongoing development of a pharmaceutical called nucleic acid medicine, and in particular, development of nucleic acid medicine utilizing the antisense method is actively carried out from the viewpoint of high selectivity on the target gene and low toxicity. The antisense method is a method comprising selectively modifying or inhibiting the expression of a protein encoded by a target gene or the activity of miRNA by introducing into a cell an oligonucleotide complementary to a target sense strand that is a partial sequence of mRNA or miRNA transcribed from the target gene (antisense oligonucleotide, herein often referred to as "ASO").

As a nucleic acid utilizing the antisense method, the present inventors have developed a double-stranded nucleic acid complex (heteroduplex oligonucleotide, HDO) in which an antisense oligonucleotide and a complementary strand thereto are annealed (Patent Literature 1, Non-Patent Literature 1 and 2). A double-stranded nucleic acid complex has a high antisense effect, and provides an epoch-making technology that can control the central nervous system beyond the blood brain barrier.

Meanwhile, clinical development of nucleic acid medicine has advanced in recent years, and the results of preclinical tests have been accumulated. As a result, it has been revealed that nucleic acid medicine such as an ASO has problems, such as toxicity and an adverse event, in not a few cases. For example, nearly half of the causes for the suspension of the development of nucleic acid medicine were toxicity and an adverse event from 2013 to 2016 (Non-Patent Literature 3). Accordingly, a technology for avoiding the toxicity of nucleic acid medicine is demanded.

### Citation List

### Patent Literature

Patent Literature 1: WO2013/089283
Patent Literature 2: WO2014/192310

### Non-Patent Literature

Non-Patent Literature 1: Nishina K, et. al., "DNA/RNA heteroduplex oligonucleotide for highly efficient gene silencing", Nature Communication, 2015, 6:7969.
Non-Patent Literature 2: Asami Y, et al., "Drug delivery system of therapeutic oligonucleotides", Drug Discoveries & Therapeutics. 2016; 10(5):256-262.
Non-Patent Literature 3: Harrison R.K., "Phase II and phase III failures: 2013-2015", Nature Reviews Drug Discovery, 2016; 15:817-818.

### Summary of Invention

### Technical Problem

A problem to be addressed is to provide a double-stranded nucleic acid complex with reduced central nervous system toxicity.

### Solution to Problem

When a single-stranded gapmer antisense nucleic acid is intraventricularly administered to a mouse, hypoactivity and motor dysfunction that are due to central nervous system toxicity are observed in the mouse within several hours after the administration. The present inventors have studied vigorously to seek a novel technology capable of reducing the central nervous system toxicity of nucleic acid medicine, and introduced a 2'-modified nucleoside into a double-stranded nucleic acid complex. As a result, the present inventors have found that the introduction of a 2'-modified nucleoside can dramatically reduce or eliminate the central nervous system toxicity of the double-stranded nucleic acid complex. This toxicity reduction effect is a surprising effect much higher than expected. The present invention is based on the above-described findings, and provides the following.
(1) A double-stranded nucleic acid complex comprising a first nucleic acid strand and a second nucleic acid strand, wherein: said first nucleic acid strand is capable of hybridizing to at least part of a target gene or a transcription product thereof, and has an antisense effect on said target gene or a transcription product thereof, and said second nucleic acid strand comprises a base sequence complementary to said first nucleic acid strand, and comprises one or more 2'-modified nucleoside.
(2) The double-stranded nucleic acid complex of (1), wherein said first nucleic acid strand is gapmer.
(3) The double-stranded nucleic acid complex of (1), wherein all of nucleosides in said first nucleic acid strand are modified nucleosides.
(4) The double-stranded nucleic acid complex of (3), wherein all of nucleosides in said first nucleic acid strand are 2'-modified nucleosides.
(5) The double-stranded nucleic acid complex of (4), wherein all of nucleosides in said first nucleic acid strand are 2'-O-methoxyethyl-modified nucleosides.
(6) The double-stranded nucleic acid complex of any one of (1) to (5), wherein said second nucleic acid strand comprises one or consecutive two to ten 2'-modified nucleosides at the 5' end, and/or one or consecutive two to ten 2'-modified nucleosides at the 3' end.
(7) The double-stranded nucleic acid complex of any one of (1) to (6), wherein said second nucleic acid strand comprises one to seven 2'-modified nucleosides at positions other than 5' end and 3' end.
(8) The double-stranded nucleic acid complex of (2), wherein said first nucleic acid strand comprises:
   (1) a central region which comprises at least four consecutive deoxyribonucleosides,
   (2) a 5'-wing region which comprises a non-natural nucleoside, positioned on the 5' end side of said central region, and
   (3) a 3'-wing region which comprises a non-natural nucleoside, positioned on the 3' end side of said central region.
(9) The double-stranded nucleic acid complex of (8), wherein said second nucleic acid strand comprises a 2'-modified nucleoside in a region which consists of a base sequence complementary to the 5'-wing region and/or the 3'-wing region in said first nucleic acid strand.
(10) The double-stranded nucleic acid complex of (9), wherein all of the nucleosides in a region in said second nucleic acid strand which consists of a base sequence complementary to the 5'-wing region and/or the 3'-wing region in said first nucleic acid strand are 2'-modified nucleosides.
(11) The double-stranded nucleic acid complex of (10), wherein all of the nucleosides which comprise a purine base in a region in said second nucleic acid strand which consists of a base sequence complementary to the central region in said first nucleic acid strand are ribonucleosides.
(12) The double-stranded nucleic acid complex of (11), wherein all of the nucleosides which comprise a pyrimidine base in a region in said second nucleic acid strand which consists of a base sequence complementary to the central region in said first nucleic acid strand are deoxyribonucleosides.
(13) The double-stranded nucleic acid complex of (12), wherein said 2'-modified nucleosides in said second nucleic acid strand are 2'-O-methoxyethyl-modified nucleosides or 2'-O-methyl-modified nucleosides.
(14) The double-stranded nucleic acid complex of any one of (8) to (10), wherein all of nucleosides in a region in said second nucleic acid strand which consists of a base sequence complementary to the central region in said first nucleic acid strand are:
   (a) deoxyribonucleosides,
   (b) deoxyribonucleosides and ribonucleosides,
   (c) deoxyribonucleosides and 2'-modified nucleosides,
   (d) ribonucleosides and 2'-modified nucleosides, or
   (e) deoxyribonucleosides, ribonucleosides, and 2'-modified nucleosides.
(15) The double-stranded nucleic acid complex of (14), wherein, in said second nucleic acid strand, all of nucleosides in the regions which consist of a base sequence complementary to the 5'-wing region and the 3'-wing region in said first nucleic acid strand are 2'-modified nucleosides, and all of nucleosides in the region which consists of a base sequence complementary to the central region in said first nucleic acid strand are deoxyribonucleosides.
(16) The double-stranded nucleic acid complex of any one of (8) to (10), wherein said second nucleic acid strand comprises at least four consecutive ribonucleosides complementary to the at least four consecutive deoxyribonucleosides in said central region in said first nucleic acid strand.
(17) The double-stranded nucleic acid complex of any one of (1) to (16), wherein the nucleosides in said second nucleic acid strand which are complementary to:
   (i) at least one guanosine nucleoside in said first nucleic acid strand,
   (ii) the nucleoside which is adjacent to said guanosine nucleoside at the 5' end side,
   (iii) the nucleoside which is adjacent to said guanosine nucleoside at the 3' end side,
      or
   (iv) any combination of (i) to (iii)
   are 2'-modified nucleosides.
(18) The double-stranded nucleic acid complex of any one of (1) to (17), wherein the nucleosides in said second nucleic acid strand which are complementary to:
   (i) at least one guanosine nucleoside in the 5'-wing region of said first nucleic acid strand,
   (ii) the nucleoside which is adjacent to said guanosine nucleoside at the 5' end side,
   (iii) the nucleoside which is adjacent to said guanosine nucleoside at the 3' end side,
      or
   (iv) any combination of (i) to (iii)
   are 2'-modified nucleosides.
(19) The double-stranded nucleic acid complex of any one of (1) to (18), wherein at least one nucleoside comprising adenine base or pyrimidine base in said second nucleic acid strand is a 2'-modified nucleoside and/or deoxyribonucleoside.
(20) The double-stranded nucleic acid complex of any one of (1) to (19), wherein at least one nucleoside comprising pyrimidine base in said second nucleic acid strand is 2'-modified nucleoside and/or deoxyribonucleoside.
(21) The double-stranded nucleic acid complex of any one of (1) to (20), wherein said second nucleic acid strand does not comprise a natural ribonucleoside comprising pyrimidine base.
(22) The double-stranded nucleic acid complex of (21), wherein all of the nucleosides comprising pyrimidine base in said second nucleic acid strand are 2'-modified nucleosides and/or deoxyribonucleosides.
(23) The double-stranded nucleic acid complex of any one of (1) to (22), wherein 20% or more of nucleosides in said second nucleic acid strand are 2'-modified nucleosides.
(24) The double-stranded nucleic acid complex of any one of (1) to (23), wherein all of the nucleosides other than 2'-modified nucleosides in said second nucleic acid strand are deoxyribonucleosides.
(25) The double-stranded nucleic acid complex of any one of (1) to (24), wherein all of the nucleosides in a region in said second nucleic acid strand which consists of a base sequence complementary to the first nucleic acid strand are 2'-modified nucleosides.
(26) The double-stranded nucleic acid complex of any one of (1) to (25), wherein said 2'-modified nucleosides in said second nucleic acid strand are 2'-O-methoxyethyl-modified nucleosides and/or 2'-O-methyl-modified nucleosides.
(27) The double-stranded nucleic acid complex of any one of (1) to (26), wherein said second nucleic acid strand comprises at least one 2'-O-methyl-modified nucleoside, scpBNA nucleoside, AmNA nucleoside, or BNA-NC.
(28) The double-stranded nucleic acid complex of (1), wherein said first nucleic acid strand is mixmer.
(29) The double-stranded nucleic acid complex of any one of (1) to (28), wherein said second nucleic acid strand comprises at least one bulge structure consisting of a base sequence not complementary to said first nucleic acid strand.
(30) The double-stranded nucleic acid complex of any one of (1) to (29), wherein said second nucleic acid strand comprises a non-complementary base, and/or an insertion sequence and/or a deletion of one or more bases, with respect to said first nucleic acid strand.
(31) The double-stranded nucleic acid complex of (30), wherein said second nucleic acid strand comprises one to three said non-complementary bases.
(32) The double-stranded nucleic acid complex of (30) or (31), wherein said insertion sequence consists of 1 to 8 bases.
(33) The double-stranded nucleic acid complex of any one of (30) to (32), wherein said deletion consists of consecutive 1 to 4 bases.
(34) The double-stranded nucleic acid complex of any one of (30) to (33), wherein said non-complementary base in said second nucleic acid strand forms a bulge structure, or wherein said second nucleic acid strand comprises, at the position of said deletion, a bulge structure consisting of a base sequence which is not complementary to said first nucleic acid strand.
(35) The double-stranded nucleic acid complex of (29) or (34), wherein said bulge structure comprises a sugar-unmodified nucleoside, or all of nucleosides in said bulge structure are sugar-unmodified nucleosides.
(36) The double-stranded nucleic acid complex of (29), (34), or (35), wherein said bulge structure is 1 to 10 bases in length.
(37) The double-stranded nucleic acid complex of any one of (29) and (34) to (36), wherein, in said second nucleic acid strand, all of the nucleosides other than said bulge structure are 2'-modified nucleosides.
(38) The double-stranded nucleic acid complex of any one of (1) to (37), wherein said second nucleic acid strand is at least 8 bases in length.
(39) The double-stranded nucleic acid complex of any one of (1) to (38), wherein the base length of said second nucleic acid strand is shorter than the base length of the first nucleic acid strand.
(40) The double-stranded nucleic acid complex of any one of (1) to (39), wherein said second nucleic acid strand comprises at least one overhang region on the 5' end side and/or 3' end side of a region consisting of a base sequence complementary to said first nucleic acid strand.
(41) The double-stranded nucleic acid complex of (40), wherein said overhang region is 1 to 20 bases in length.
(42) The double-stranded nucleic acid complex of (40) or (41), wherein said overhang region comprises at least one deoxyribonucleoside and/or non-natural nucleoside.
(43) The double-stranded nucleic acid complex of any one of (1) to (42), wherein said first nucleic acid strand and said second nucleic acid strand are bound via a linker.
(44) The double-stranded nucleic acid complex of (43), wherein: the 5' end of said first nucleic acid strand and the 3' end of said second nucleic acid strand, and/or the 3' end of said first nucleic acid strand and the 5' end of said second nucleic acid strand are bound by said linker.
(45) The double-stranded nucleic acid complex of (43) or (44), wherein said linker is a cleavable or uncleavable linker.
(46) The double-stranded nucleic acid complex of any one of (43) to (45), wherein said linker consists of nucleic acids comprising natural nucleosides and/or non-natural nucleosides, or polyethylene glycol.
(47) The double-stranded nucleic acid complex of (46), wherein said linker consisting of nucleic acids are 2 to 10 bases in length.
(48) The double-stranded nucleic acid complex of any one of (1) to (47), wherein said second nucleic acid strand is bound to a ligand.
(49) The double-stranded nucleic acid complex of (48), wherein said ligand is any one or more selected from the group consisting of a small molecule, a peptide, a lipid, and a nucleic acid aptamer.
(50) The double-stranded nucleic acid complex of (49), wherein said lipid is cholesterol or an analog thereof, or tocopherol or an analog thereof.
(51) The double-stranded nucleic acid complex of any one of (48) to (50), wherein said ligand is bound to the 5' end and/or 3' end of said second nucleic acid strand.
(52) The double-stranded nucleic acid complex of any one of (1) to (47), which is not bound to a ligand.
(53) The double-stranded nucleic acid complex of any one of (1) to (52), wherein all or a part of the internucleoside linkages in said first nucleic acid strand and/or said second nucleic acid strand are modified internucleoside linkages.
(54) The double-stranded nucleic acid complex of (53), wherein said modified internucleoside linkage is a phosphorothioate linkage.
(55) A pharmaceutical composition comprising the double-stranded nucleic acid complex of any one of (1) to (54) as an active ingredient.
(56) The pharmaceutical composition of (55) for treating central nervous system disease in a subject.
(57) The pharmaceutical composition of (55) or (56), wherein the pharmaceutical composition is administered by intraventricular administration or intrathecal administration.
(58) The pharmaceutical composition of (57), wherein said intrathecal administration is suboccipital puncture or lumbar puncture.
(59) The pharmaceutical composition of (57) or (58), wherein 0.1 mg to 200 mg of said double-stranded nucleic acid complex is administered.
(60) The pharmaceutical composition of (55) or (56), wherein the pharmaceutical composition is administered by intravenous administration or subcutaneous administration.
(61) The pharmaceutical composition of (60), wherein said double-stranded nucleic acid complex is administered at 0.1 mg/kg to 100 mg/kg.
(62) The pharmaceutical composition of any one of (55) to (61), wherein central nervous system toxicity is reduced.

The contents of the disclosures in Japanese Patent Application No. 2021-150310 which form the basis for priority of the present application are incorporated herein.

### Advantageous Effects of Invention

The present invention provides a double-stranded nucleic acid complex with reduced central nervous system toxicity.

### Brief Description of Drawings

[Figure 1] Figure 1 shows the structures of various bridged nucleic acids.

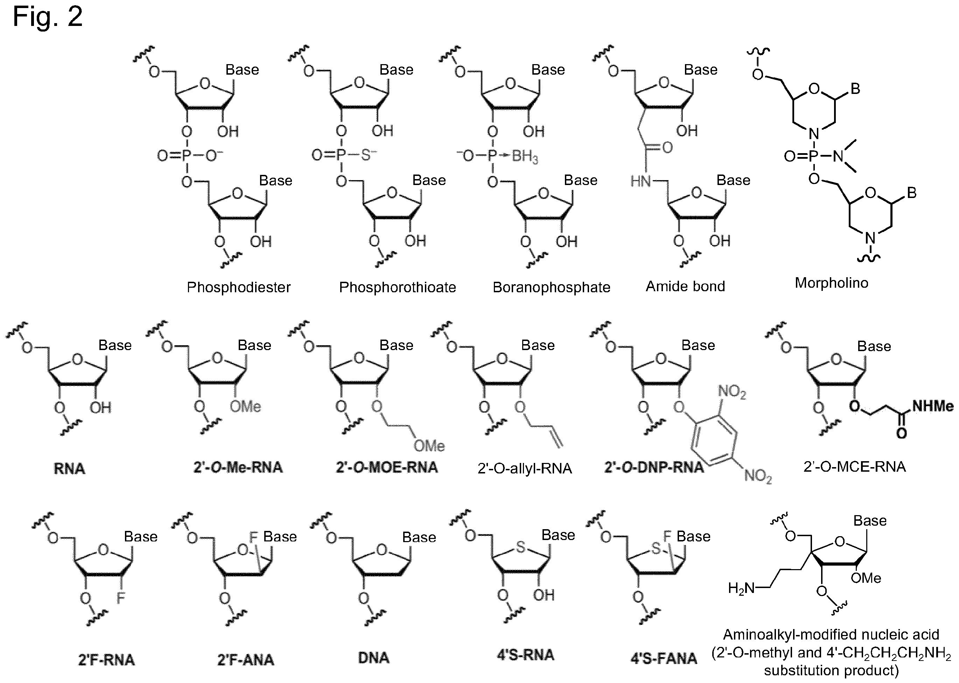

[Figure 2] Figure 2 shows the structures of various natural nucleotides or non-natural nucleotides.
[Figure 3] Figure 3 shows a scoring system for evaluating central nervous system toxicity in mice to which nucleic acid agents were administered.
[Figure 4] Figure 4 shows the structures of nucleic acids used in Example 1. Figure 4A shows the structure of an ASO targeting the Mapt gene. Figure 4B shows the structure of HDO (all RNA). Figure 4C shows the structure of HDO (all DNA). Figure 4D shows the structure of HDO (6MOE wing). Figure 4E shows the structure of HDO (all MOE).
[Figure 5] Figure 5 shows the acute tolerability scores of mice at 30 minutes to 4 hours after various nucleic acid agents were intraventricularly administered to the mice. The error bars indicate standard errors.
[Figure 6] Figure 6 shows the motor function of mice at one hour after various nucleic acid agents were intraventricularly administered to the mice. Figure 6A shows the total movement distance for 5 minutes. Figure 6B shows the maximum moving speed. The error bars indicate standard errors.
[Figure 7] Figure 7 shows the Mapt mRNA expression level in the hippocampus of mice to which various nucleic acid agents were intraventricularly administered. The error bars indicate standard errors.
[Figure 8] Figure 8 shows the structures of nucleic acids used in Example 2. Figure 8A shows the structure of an ASO targeting the BACE1 gene. Figure 8B shows the structure of HDO (all RNA). Figure 8C shows the structure of HDO (all DNA). Figure 8D shows the structure of HDO (5MOE wing).
[Figure 9] Figure 9 shows the acute tolerability scores of mice at 30 minutes to 4 hours after various nucleic acid agents were intraventricularly administered to the mice. The error bars indicate standard errors.
[Figure 10] Figure 10 shows the motor function of mice at one hour after various nucleic acid agents were intraventricularly administered to the mice. Figure 10A shows the total movement distance for 5 minutes. Figure 10B shows the maximum moving speed. The error bars indicate standard errors.
[Figure 11] Figure 11 shows the structures of nucleic acids used in Example 3. Figure 11A shows the structure of an ASO targeting the Malat1 gene. Figure 11B shows the structure of HDO (all RNA). Figure 11C shows the structure of HDO (all DNA). Figure 11D shows the structure of HDO (10MOE wing).
[Figure 12] Figure 12 shows the acute tolerability scores of mice at 30 minutes to 4 hours after various nucleic acid agents were intraventricularly administered to the mice. The error bars indicate standard errors.
[Figure 13] Figure 13 shows the motor function of mice at one hour after various nucleic acid agents were intraventricularly administered to the mice. Figure 13A shows the total movement distance for 5 minutes. Figure 13B shows the maximum moving speed. The error bars indicate standard errors.
[Figure 14] Figure 14 shows the structures of nucleic acids used in Example 4. Figure 14A shows the structure of HDO (all DNA) comprising an ASO targeting the Mapt gene. Figure 14B shows the structure of HDO (RNA 6MOE wing). Figure 14C shows the structure of HDO (6MOE wing). Figure 14D shows the structure of HDO (6OMe wing). Figure 14E shows the structure of HDO (6F wing).
[Figure 15] Figure 15 shows the acute tolerability scores of mice at 30 minutes to 4 hours after various nucleic acid agents were intraventricularly administered to the mice. The error bars indicate standard errors.
[Figure 16] Figure 16 shows the motor function of mice at one hour after various nucleic acid agents were intraventricularly administered to the mice. Figure 16A shows the total movement distance for 5 minutes. Figure 16B shows the maximum moving speed. The error bars indicate standard errors.
[Figure 17] Figure 17 shows the motor function of mice at 3 hours after various nucleic acid agents were intraventricularly administered to the mice. Figure 17A shows the total movement distance for 5 minutes. Figure 17B shows the maximum moving speed. The error bars indicate standard errors.
[Figure 18] Figure 18 shows the structures of nucleic acids used in Example 5. Figure 18A shows the structure of an ASO targeting the Mapt gene. Figure 18B shows the structure of HDO (6MOE wing). Figure 18C shows the structure of HDO (G^{MOE}). Figure 18D shows the structure of HDO (G^{RNA}). Figure 18E shows the structure of HDO (inosine).
[Figure 19] Figure 19 shows the acute tolerability scores of mice at 30 minutes to 4 hours after various nucleic acid agents were intraventricularly administered to the mice. The error bars indicate standard errors.
[Figure 20] Figure 20 shows the structures of nucleic acids used in Example 6. Figure 20A shows the structure of HDO (all DNA) comprising an ASO targeting the Mapt gene. Figure 20B shows the structure of HDO (6MOE-5'&3'). Figure 20C shows the structure of HDO (6MOE-5'). Figure 20D shows the structure of HDO (6MOE-3'). Figure 20E shows the structure of HDO (10MOE-5'). Figure 20F shows the structure of HDO (10MOE-3').
[Figure 21] Figure 21 shows the acute tolerability scores of mice at 30 minutes to 4 hours after various nucleic acid agents were intraventricularly administered to the mice. The error bars indicate standard errors.
[Figure 22] Figure 22 shows the motor function of mice at one hour after various nucleic acid agents were intraventricularly administered to the mice. Figure 22A shows the total movement distance for 5 minutes. Figure 22B shows the maximum moving speed. The error bars indicate standard errors.
[Figure 23] Figure 23 shows the motor function of mice at 3 hours after various nucleic acid agents were intraventricularly administered to the mice. Figure 23A shows the total movement distance for 5 minutes. Figure 23B shows the maximum moving speed. The error bars indicate standard errors.
[Figure 24] Figure 24 shows the Mapt mRNA expression level in the hippocampus of mice to which various nucleic acid agents were intraventricularly administered. The error bars indicate standard errors.
[Figure 25] Figure 25 shows the structures of nucleic acids used in Example 7. Figure 25A shows the structure of HDO (all DNA) comprising an ASO targeting the BACE1 gene. Figure 25B shows the structure of HDO (5MOE-5'). Figure 25C shows the structure of HDO (5MOE-3'). Figure 25D shows the structure of HDO (8MOE-5'). Figure 25E shows the structure of HDO (8MOE-3'). Figure 25F shows the structure of HDO (10MOE-5'). Figure 25G shows the structure of HDO (10MOE-3').
[Figure 26] Figure 26 shows the acute tolerability scores of mice at 30 minutes to 4 hours after various nucleic acid agents were intraventricularly administered to the mice. The error bars indicate standard errors.
[Figure 27] Figure 27 shows the motor function of mice at one hour after various nucleic acid agents were intraventricularly administered to the mice. Figure 27A shows the total movement distance for 5 minutes. Figure 27B shows the maximum moving speed. The error bars indicate standard errors.
[Figure 28] Figure 28 shows the structures of nucleic acids used in Example 8. Figure 28A shows the structure of HDO (all DNA) comprising an ASO targeting the Mapt gene. Figure 28B shows the structure of HDO (6MOE wing). Figure 28C shows the structure of HDO (9MOE wing). Figure 28D shows the structure of HDO (11MOE wing). Figure 28E shows the structure of HDO (13MOE wing). Figure 28F shows the structure of HDO (15MOE wing).
[Figure 29] Figure 29 shows the acute tolerability scores of mice at 30 minutes to 4 hours after various nucleic acid agents were intraventricularly administered to the mice. The error bars indicate standard errors.
[Figure 30] Figure 30 shows the motor function of mice at one hour after various nucleic acid agents were intraventricularly administered to the mice. Figure 27A shows the total movement distance for 5 minutes. Figure 27B shows the maximum moving speed. The error bars indicate standard errors.
[Figure 31] Figure 31 shows the structures of nucleic acids used in Example 9. Figure 31A shows the structure of HDO (all DNA) comprising an ASO targeting the Mapt gene. Figure 31B shows the structure of HDO (A^{MOE}). Figure 31C shows the structure of HDO (G^{MOE}). Figure 31D shows the structure of HDO (C^{MOE}). Figure 31E shows the structure of HDO (T^{MOE}).
[Figure 32] Figure 32 shows the acute tolerability scores of mice at 30 minutes to 4 hours after various nucleic acid agents were intraventricularly administered to the mice. The error bars indicate standard errors.
[Figure 33] Figure 33 shows the motor function of mice at one hour after various nucleic acid agents were intraventricularly administered to the mice. Figure 33A shows the total movement distance for 5 minutes. Figure 33B shows the maximum moving speed. The error bars indicate standard errors.
[Figure 34] Figure 34 shows the structures of nucleic acids used in Example 10. Figure 34A shows the structure of HDO (all DNA) comprising an ASO targeting the Mapt gene. Figure 34B shows the structure of HDO (C^{MOE}). Figure 34C shows the structure of HDO (2C^{MOE}-5). Figure 34D shows the structure of HDO (2C^{MOE}-3). Figure 34E shows the structure of HDO (3C^{MOE}).
[Figure 35] Figure 35 shows the acute tolerability scores of mice at 30 minutes to 4 hours after various nucleic acid agents were intraventricularly administered to the mice. The error bars indicate standard errors.
[Figure 36] Figure 36 shows the motor function of mice at one hour after various nucleic acid agents were intraventricularly administered to the mice. Figure 36A shows the total movement distance for 5 minutes. Figure 36B shows the maximum moving speed. The error bars indicate standard errors.

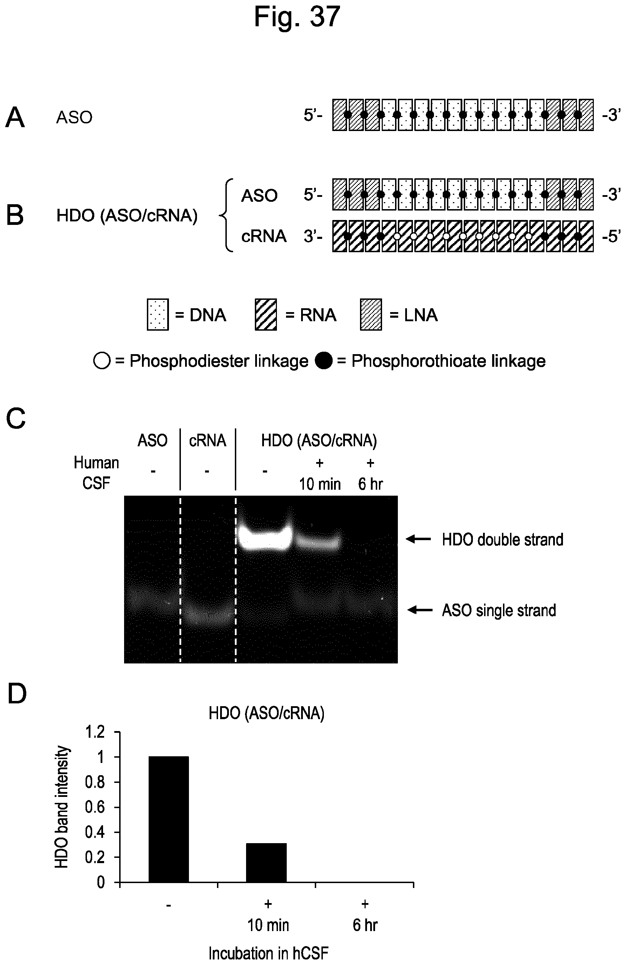

[Figure 37] Figure 37 shows the stability of various nucleic acid agents in the human cerebrospinal fluid. Figures 37A and 37B show the structures of nucleic acids used in Example 11. Figure 37A shows the structure of an ASO targeting the Mapt gene. Figure 37B shows the structure of HDO (ASO/cRNA). Figure 37C shows the results of electrophoresis for examining the stability of the various nucleic acid agents that were mixed with human cerebrospinal fluid (human CSF; hCSF) for 10 minutes or 6 hours. Figure 37D shows the results of quantification of the band intensity of the HDO double strand in HDO (ASO/cRNA).

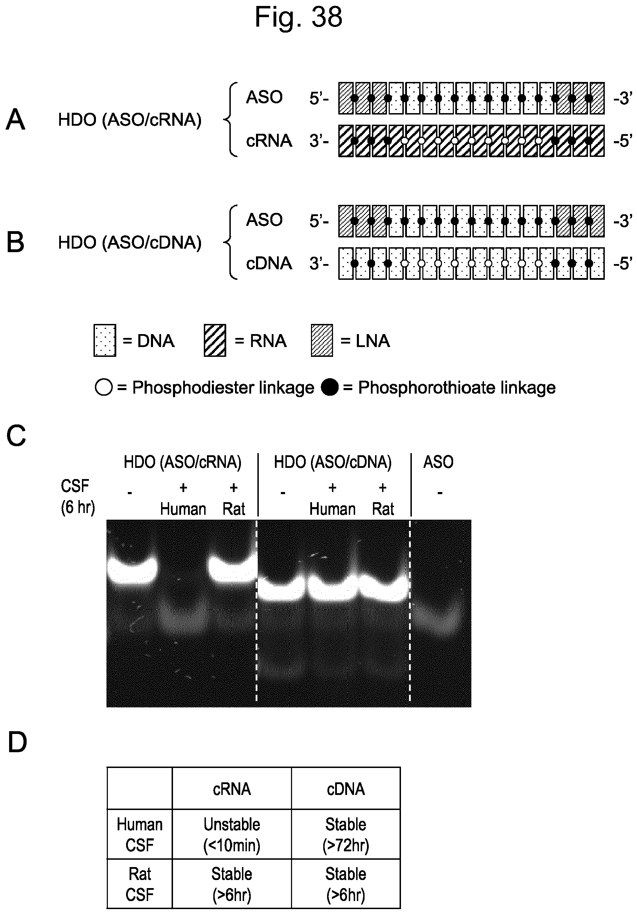

[Figure 38] Figure 38 shows the stability of various nucleic acid agents in the cerebrospinal fluid of a human and a rat. Figures 38A and 38B show the structures of nucleic acids used in Example 11. Figure 38A shows the structure of HDO (ASO/cRNA) comprising an ASO targeting the Mapt gene. Figure 38B shows the structure of HDO (ASO/cDNA). Figure 38C shows the results of electrophoresis for examining the stability of the various nucleic acid agents that were mixed with the cerebrospinal fluid of a human or a rat for 6 hours. Figure 38D shows the stability of the second nucleic acid strands (cRNA and cDNA) of HDO (ASO/cRNA) and HDO (ASO/cDNA) in the cerebrospinal fluid of a human and a rat.
[Figure 39] Figure 39 shows the stability of various nucleic acid agents in the cerebrospinal fluid of a mouse, rat, monkey, and human. Figures 39A and 39B show the structures of nucleic acids used in Example 12. Figure 39A shows the structure of HDO (all RNA) comprising an ASO targeting the Mapt gene. Figure 39B shows the structure of HDO (all DNA). Figure 39C shows the results of electrophoresis for examining the stability of the various nucleic acid agents that were mixed with the cerebrospinal fluid of a mouse, rat, monkey, and human for 6 hours.
[Figure 40] Figure 40 shows the stability of various nucleic acid agents in the cerebrospinal fluid of a mouse, rat, monkey, and human. Figures 40A and 40B show the structures of nucleic acids used in Example 12. Figure 40A shows the structure of HDO (cRNA 6MOE wing) comprising an ASO targeting the Mapt gene. Figure 40B shows the structure of HDO (cDNA 6MOE wing). Figure 40C shows the results of electrophoresis for examining the stability of the various nucleic acid agents that were mixed with the cerebrospinal fluid of a mouse, rat, monkey, and human for 6 hours.
[Figure 41] Figure 41 shows the structures of nucleic acids used in Example 13. Figure 41A shows the structure of HDO (all DNA) comprising an ASO targeting the Mapt gene. Figure 41B shows the structure of HDO (A^{RNA}). Figure 41C shows the structure of HDO (G^{RNA}). Figure 41D shows the structure of HDO (C^{RNA}). Figure 41E shows the structure of HDO (U^{RNA}).
[Figure 42] Figure 42 shows the results of electrophoresis for examining the stability of the various nucleic acid agents that were mixed with the human cerebrospinal fluid for 6 hours.
[Figure 43] Figure 43 shows the structures of nucleic acids used in Example 14. Figure 43A shows the structure of HDO (all DNA) comprising an ASO targeting the Mapt gene. Figure 43B shows the structure of HDO (GA^{RNA}). Figure 43C shows the structure of HDO (CU^{RNA}). Figure 43D shows the structure of HDO (C^{RNA}). Figure 43E shows the structure of HDO (U^{RNA}).
[Figure 44] Figure 44 shows the results of electrophoresis for examining the stability of the various nucleic acid agents that were mixed with the human cerebrospinal fluid for 1 hour or 6 hours.
[Figure 45] Figure 45 shows the structures of nucleic acids used in Example 15. Figure 45A shows the structure of HDO (all DNA) comprising an ASO targeting the Malat1 gene. Figure 45B shows the structure of HDO (A^{RNA}). Figure 45C shows the structure of HDO (G^{RNA}). Figure 45D shows the structure of HDO (C^{RNA}). Figure 45E shows the structure of HDO (U^{RNA}).
[Figure 46] Figure 46 shows the results of electrophoresis for examining the stability of various nucleic acid agents that were mixed with the human cerebrospinal fluid for 6 hours.

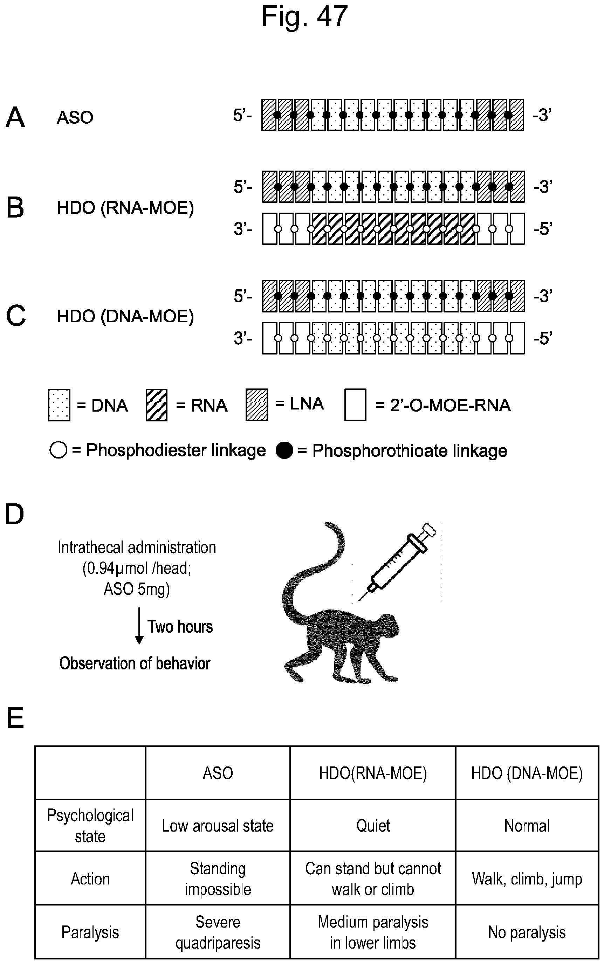

[Figure 47] Figure 47 shows the results of evaluation of the central nervous system toxicity of nucleic acid agents in monkeys. Figures 47A to 47C show the structures of nucleic acids used in Example 16. Figure 47A shows the structure of an ASO targeting the Mapt gene. Figure 47B shows the structure of HDO (RNA-MOE). Figure 47C shows the structure of HDO (DNA-MOE). Figure 47D shows the procedures for evaluation of the central nervous system toxicity to monkeys in Example 16. Figure 47E shows the results of evaluation of the central nervous system toxicity of various nucleic acid agents in monkeys.
[Figure 48] Figure 48 shows the structures of nucleic acids used in Example 17. Figure 48A shows the structure of HDO (all DNA). Figure 48B shows the structure of HDO (all MOE). Figure 48C shows the structure of HDO (bulge1). Figure 48D shows the structure of HDO (bulge2).
[Figure 49] Figure 49 shows the Mapt mRNA expression level and LDH activity of human neuroblastoma-derived cells into which various nucleic acid agents were introduced. Figure 49A shows a relative Mapt mRNA level. Figure 49B shows a relative LDH release level in a supernatant. The error bars indicate standard errors.
[Figure 50] Figure 50 shows the structures of nucleic acids used in Example 18. Figure 50A shows the structure of HDO (bulge). Figure 50B shows the structure of HDO (ssHDO). Figure 50C shows the structure of PEG linker ssHDO. Figure 50D shows the structure of Bulge plus ssHDO.
[Figure 51] Figure 51 shows the results of evaluation of the motor function of mice at one hour after various nucleic acid agents were intraventricularly administered to the mice. Figure 51A shows the total movement distance for 5 minutes. Figure 51B shows the maximum moving speed. The error bars indicate standard errors.
[Figure 52] Figure 52 shows the Malat1 RNA expression level in the brain of mice to which various nucleic acid agents were intraventricularly administered. Figure 52A shows the results of the left frontal cortex. Figure 52B shows the results of the right frontal cortex. The error bars indicate standard errors.
[Figure 53] Figure 53 shows the results of electrophoresis for evaluating the efficiency of dissociation of double strands after various nucleic acid agents were incubated in the brain tissue homogenate for 7 days.
[Figure 54] Figure 54 shows the acute tolerability scores of mice at 30 minutes to 4 hours after various nucleic acid agents were intraventricularly administered to the mice. The error bars indicate standard errors.
[Figure 55] Figure 55 shows the results of evaluation of the motor function of mice at one hour after various nucleic acid agents were intraventricularly administered to the mice. Figure 55A shows the total movement distance for 5 minutes. Figure 55B shows the maximum moving speed. The error bars indicate standard errors.
[Figure 56] Figure 56 shows the acute tolerability scores of mice at 30 minutes to 4 hours after various nucleic acid agents were intraventricularly administered to the mice. The error bars indicate standard errors.
[Figure 57] Figure 57 shows the results of evaluation of the motor function of mice at one hour after various nucleic acid agents were intraventricularly administered to the mice. Figure 57A shows the total movement distance for 5 minutes. Figure 57B shows the maximum moving speed. The error bars indicate standard errors.
[Figure 58] Figure 58 shows the results of measurement of the body weight of mice to which various nucleic acid agents were intraventricularly administered. The error bars indicate standard errors. The error bars indicate standard errors.
[Figure 59] Figure 59 shows the results of evaluation of the motor function of mice at one day or later after various nucleic acid agents were intraventricularly administered to the mice.
[Figure 60] Figure 60 shows the Mapt mRNA expression level in the right frontal lobe of mice to which various nucleic acid agents were intraventricularly administered. The error bars indicate standard errors.
[Figure 61] Figure 61 shows the results of electrophoresis for evaluation of the efficiency of dissociation of double strands after various nucleic acid agents were incubated in the brain tissue homogenate for 7 days.
[Figure 62] Figure 62 shows the acute tolerability scores of mice at 30 minutes to 4 hours after various nucleic acid agents were intraventricularly administered to the mice. The error bars indicate standard errors.
[Figure 63] Figure 63 shows the results of evaluation of the motor function of mice at one hour after various nucleic acid agents were intraventricularly administered to the mice. Figure 63A shows the total movement distance for 5 minutes. Figure 63B shows the maximum moving speed. The error bars indicate standard errors.
[Figure 64] Figure 64 shows the LDH activity and the Bace1 mRNA expression level of mouse neuroblastoma-derived cells (Neuro 2a cell line) into which various nucleic acid agents were introduced. Figure 64A shows a relative LDH release level in a supernatant. Figure 64B shows a relative Bace1 mRNA level. The error bars indicate standard errors.
[Figure 65] Figure 65 shows an evaluation method by the modified FOB scores.
[Figure 66] Figure 66 shows the results of evaluation by the modified FOB scores for monkeys to which various nucleic acid agents were intrathecally administered.
[Figure 67] Figure 67 shows the results of a three-minute video for measuring the spontaneous locomotion time and number of jumps in monkeys to which various nucleic acid agents were intrathecally administered.

### Description of Embodiments

### 1. Double-stranded nucleic acid complex

### 1-1. Overview

A first aspect of the present invention is a double-stranded nucleic acid complex. The double-stranded nucleic acid complex of the present invention comprises a first nucleic acid strand and a second nucleic acid strand, and comprises one or more 2'-modified nucleosides. The double-stranded nucleic acid complex of the present invention is stable in the cerebrospinal fluid of a primate including a human, and has reduced toxicity of *e.g.,* central nervous system toxicity.

### 1-2. Definition of terms

A "transcription product" of a target gene means herein any RNA that is synthesized by an RNA polymerase and is a direct target of the nucleic acid complex of the present invention. Specifically, mRNA transcribed from a target gene (comprising, e.g., mature mRNA, mRNA precursor, and mRNA without base modification), non-coding RNA (ncRNA) such as miRNA, long non-coding RNA (lncRNA), and natural antisense RNA can be included.

A "target gene" means herein a gene wherein the expression level of a transcription product or a translation product thereof can be reduced or increased by the antisense effect of the double-stranded nucleic acid complex of the present invention; a gene wherein the function of a transcription product or a translation product thereof can be inhibited by the effect; or a gene for which steric blocking, splicing switching, RNA editing, exon skipping, or exon inclusion can be induced by the effect. There is no particular restriction on the kind of target gene, as long as it is expressed in vivo. Examples thereof include a gene which is derived from an organism into which a double-stranded nucleic acid complex of the present invention is to be introduced, such as a gene whose expression is increased in various diseases. Specific examples thereof include a scavenger receptor B 1 (often referred to herein as "SR-B1") gene, and a metastasis associated lung adenocarcinoma transcript 1 (herein often referred to as "Malat1") gene, a microtubule-associated protein tau (herein often referred to as "Mapt") gene, β-secretase 1 (herein often referred to as "BACE1") gene, a DMPK (dystrophia myotonica-protein kinase) gene, and a dystrophin gene.

A "target transcription product" means herein any RNA that is synthesized by an RNA polymerase and is a direct target of the nucleic acid complex of the present invention. In general, it is a "transcription product of a target gene". Specifically, mRNA transcribed from a target gene (comprising, e.g., mature mRNA, mRNA precursor, and mRNA without base modification), non-coding RNA (ncRNA) such as miRNA, long non-coding RNA (IncRNA), and natural antisense RNA can be included. Examples of a transcription product of a target gene may comprise SR-B1 mRNA which is a transcription product of the SR-B1 gene, Mapt mRNA which is a transcription product of the Mapt gene, BACE1 mRNA which is a transcription product of the BACE1 gene, Malat 1 non-coding RNA which is a transcription product of the Malat1 gene, DMPK mRNA which is a transcription product of a DMPK gene, and dystrophin mRNA which is a transcription product of the dystrophin gene or a precursor thereof (pre-mRNA).

Specific examples of a target transcription product include the exon 23/intron 23 boundary region of Dystrophin pre-mRNA (GenBank accession number: NC_000086.7), e.g., positions 83803482-83803566, e.g., 83803512-83803536. As other specific examples of a target transcription product, the base sequence of a murine DMPK mRNA is shown in SEQ ID NO: 7, and the base sequence of a human DMPK mRNA is shown in SEQ ID NO: 8. In this regard, in all of SEQ ID NOs: 7 to 8, the base sequences of mRNA are replaced with the base sequences of DNA. The base sequence information for these genes and transcription products can be obtained from publicly known databases, such as the database of NCBI (The U.S. National Center for Biotechnology Information).

An "antisense oligonucleotide (ASO)" or "antisense nucleic acid" herein refers to a single-stranded oligonucleotide that comprises a base sequence capable of hybridizing (i.e., complementary) to at least a part of a target transcription product (mainly, a transcription product of a target gene), and can produce an antisense effect on the target transcription product. In the double-stranded nucleic acid complex of the present invention, the first nucleic acid strand functions as ASO, and its target region may comprise 3'UTR, 5'UTR, exon, intron, coding region, translation initiation region, translation termination region, or any other nucleic acid region. The target region of a target transcription product may be at least 8 bases in length, e.g., 10 to 35 bases in length, 12 to 25 bases in length, 13 to 20 bases in length, 14 to 19 bases in length, 15 to 18 bases in length, 13 to 22 bases in length, 16 to 22 bases in length, or 16 to 20 bases in length.

An "antisense effect" means an effect of regulating expression or editing of a target transcription product by hybridization of ASO to the target transcription product (e.g. RNA sense strand). The phrase "regulating expression or editing of a target transcription product" includes suppression or reduction of the expression of a target gene or the expression amount of a target transcription product ("expression amount of a target transcription product" is herein often referred to as "expression level of a target transcription product"), inhibition of translation, RNA editing, a splicing function modifying effect (e.g., splicing switching, exon inclusion, and exon skipping), or degradation of a transcription product. For example, in the case of post-transcriptional inhibition of a target gene, when an RNA oligonucleotide is introduced into a cell as ASO, the ASO forms a partial double strand by annealing to mRNA which is a transcription product of a target gene. This partial double strand serves as a cover to prevent translation by ribosomes, so as to inhibit the expression of the target protein encoded by the target gene at the translation level (steric blocking). Meanwhile, when an oligonucleotide comprising DNA is introduced into a cell as ASO, a partial DNA-RNA heteroduplex is formed. This heteroduplex structure is recognized by RNase H, and as a result mRNA of the target gene is degraded and the expression of the protein encoded by the target gene is inhibited at the expression level. In addition, an antisense effect can also be produced for an intron in an mRNA precursor as a target. Furthermore, an antisense effect can also be produced for miRNA as a target. In this case, as a result of functional inhibition of the miRNA, the expression of the gene whose expression is normally regulated by the miRNA may be increased. In one embodiment, expression regulation of a target transcription product may be a decrease in the amount of a target transcription product.

The antisense effect can be measured, e.g., as follows: a subject nucleic acid compound is administered to a subject (e.g., a mouse); and, e.g., after several days (e.g., after 2 to 7 days), a measurement is made of the expression amount of a target gene or the level (amount) of the target transcription product (e.g., the amount of mRNA, the amount of RNA such as microRNA, the amount of cDNA, the amount of protein, or the like), wherein the expression of the target gene is regulated by the antisense effect provided by the subject nucleic acid compound.

For example, when the expression amount measured of the target gene or the level measured of the target transcription product is decreased by at least 10%, at least 20%, at least 25%, at least 30%, or at least 40%, compared with a negative control (e.g., vehicle administration), the measurement shows that the subject nucleic acid compound can produce an antisense effect (e.g., a decrease in the amount of the target transcription product).

The number, kind, and position of a non-natural nucleotide in a nucleic acid strand may influence the antisense effect and the like provided by the nucleic acid complex. The choice of a modification may vary depending on the sequence of a target gene or the like, but those skilled in the art can determine a suitable embodiment by referring to the descriptions in the literature related to the antisense method (e.g., WO 2007/143315, WO 2008/043753, and WO 2008/049085). Furthermore, when the antisense effect of a nucleic acid complex after the modification is measured and the obtained measured value is not significantly lower than the measured value of the nucleic acid complex before the modification (e.g., when the measured value obtained after the modification is 70% or more, 80% or more, or 90% or more of the measured value of the nucleic acid complex before the modification), a relevant modification may be evaluated.

A "translation product of the target gene" means herein any polypeptide or protein that is synthesized by translation of a target transcription product or a transcription product of a target gene which is a direct target of a nucleic acid complex of the present invention.

Herein, an "aptamer" refers to a nucleic acid molecule that specifically binds to a particular target molecule which is intracellular, on the cell membrane, or extracellular, such as a target molecule on the cell membrane or an extracellular target molecule. An aptamer can be produced by a method known in the art, e.g., an in vitro sorting method using the SELEX (systematic evolution of ligands by exponential enrichment) method.

Herein, "decoy" refers to a nucleic acid having the sequence of a binding site of a transcription factor (e.g., NF-kB) or a similar sequence, which is introduced into a cell as a "decoy" to inhibit the function of a transcription factor (inhibit transcription in the case of a transcription activator or promote transcription in the case of a transcription repressor). A decoy nucleic acid can be easily designed based on information about a binding sequence of a target transcription factor.

Herein, "bait" refers to a nucleic acid molecule that specifically binds to a particular target molecule in a cell and modifies a function of the target molecule. A target that interacts with a bait is also referred to as a "prey".

The term "nucleic acid" or "nucleic acid molecule" used herein may refer to a nucleotide or nucleoside of a monomer, and may mean an oligonucleotide consisting of a plurality of monomers, or means a polynucleotide in the case of a polymer. A "natural nucleic acid" refers to a naturally-occurring nucleic acid. Examples of the natural nucleic acid include a natural nucleoside, natural nucleotide, and the like, as described below. A "non-natural nucleic acid" or "artificial nucleic acid" refers to any nucleic acid other than a natural nucleic acid. Examples of the non-natural nucleic acid or the artificial nucleic acid include a non-natural nucleoside, non-natural nucleotide, and the like, as described below.

A "nucleic acid strand" or simply "strand" herein means two or more nucleosides linked via an internucleoside linkage, and may be, e.g., an oligonucleotide or a polynucleotide. A full-length strand or a partial length strand of a nucleic acid strand can be produced, e.g., by a chemical synthesis using an automated synthesizer, or by an enzymatic step using a polymerase, a ligase, or a restricted reaction. A nucleic acid strand may comprise a natural nucleotide and/or a non-natural nucleotide.

A "nucleoside" generally means a molecule consisting of a combination of a base and a sugar. The sugar moiety of a nucleoside is usually, but not limited to, composed of pentofuranosyl sugar, and specific examples thereof include ribose and deoxyribose. The base moiety of nucleoside (nucleobase) is usually a heterocyclic base moiety. Without limitation, examples thereof include adenine, cytosine, guanine, thymine, or uracil as well as other modified nucleobases (modified bases).

A "nucleotide" refers to a molecule in which a phosphate group is covalently bound to the sugar moiety of the nucleoside. In the case of a nucleotide comprising pentofuranosyl sugar, a phosphate group is usually linked to a hydroxyl group at the 2', 3', or 5' position of the sugar.

An "oligonucleotide" refers to a linear oligomer formed by linking several to dozens of neighboring nucleotides through a covalent bond between a hydroxyl group and a phosphate group in the sugar moiety. Furthermore, a "polynucleotide" refers to a linear polymer formed by linking with covalent bonds dozens or more, preferably hundreds or more of nucleotides, namely more nucleotides than in an oligonucleotide. It is considered that the phosphate group generally forms an internucleoside linkage inside the structure of an oligonucleotide or a polynucleotide.

A "natural nucleoside" refers herein to a nucleoside that exists in nature. Examples thereof include a ribonucleoside consisting of a ribose and the aforementioned base such as adenine, cytosine, guanine, or uracil, or a deoxyribonucleoside consisting of a deoxyribose and the aforementioned base such as adenine, cytosine, guanine, or thymine. In this regard, a ribonucleoside found in RNA, and a deoxyribonucleoside found in DNA are herein often referred to as "DNA nucleoside" and "RNA nucleoside", respectively.

A "natural nucleotide" means herein a nucleotide that exists in nature, namely a molecule in which a phosphate group is covalently bound to the sugar moiety of the aforementioned natural nucleoside. Examples thereof include a ribonucleotide which is known as a constituent of RNA, and in which a phosphate group is bound to a ribonucleoside, and a deoxyribonucleotide, which is known as a constituent of DNA, and in which a phosphate group is bound to a deoxyribonucleoside.

A "non-natural nucleotide" means herein any nucleotide other than a natural nucleotide. For example, it comprises a modified nucleotide and a nucleotide mimic. A "modified nucleotide" means herein a nucleotide having any one or more of a modified sugar moiety, a modified internucleoside linkage, and a modified nucleobase. The term "nucleotide mimic" herein comprises a structure used to substitute a nucleoside and a linkage at one or more positions in an oligomer compound. Examples of the nucleotide mimic comprise a peptide nucleic acid, and a morpholino nucleic acid (morpholino linked with -N(H)-C(=O)-O- or other non-phosphodiester linkages). The peptide nucleic acid (PNA) is a nucleotide mimic having a main chain in which N-(2-aminoethyl)glycine in place of a sugar is linked with an amide linkage. A nucleic acid strand comprising a non-natural oligonucleotide herein has in many cases preferable properties, such as enhanced cellular uptake, enhanced affinity for a target nucleic acid, increased stability in the presence of a nuclease, and increase in inhibitory activity. Therefore, it is more preferable than a natural nucleotide.

A "non-natural nucleoside" means herein any nucleoside other than a natural nucleoside. For example, it comprises a modified nucleoside and a nucleoside mimic. A "modified nucleoside" means herein a nucleoside having a modified sugar moiety and/or a modified nucleobase.

A "mimic" refers herein to a functional group that substitutes a sugar, a nucleobase, and/or an internucleoside linkage. In general, a mimic is used in place of a sugar or a combination of a sugar-internucleoside linkage, and a nucleobase is maintained for hybridization to a target to be selected. The term "nucleoside mimic" used herein comprises a structure to be used for substituting a sugar at one or more positions of an oligomer compound, or substituting a sugar and a base, or substituting a bond between monomer subunits constituting an oligomer compound. An "oligomer compound" means a polymer composed of linked monomer subunits that can at least hybridize to a region of a nucleic acid molecule. Examples of a nucleoside mimic comprise a morpholino, cyclohexenyl, cyclohexyl, tetrahydropyranyl, bicyclic or tricyclic sugar mimic, such as a nucleoside mimic having a non-furanose sugar unit.

A "modified sugar" refers to a sugar in which a natural sugar moiety (i.e., sugar moiety found in DNA(2'-H) or RNA(2'-OH)) has undergone a substitution and/or any change. "Sugar modification" refers to substitution and/or any change from a natural sugar moiety. A nucleic acid strand may comprise in some cases one or more modified nucleosides comprising a modified sugar. A "sugar-modified nucleoside" means a nucleoside having a modified sugar moiety. Such a sugar-modified nucleoside can confer a beneficial biological property such as enhanced nuclease stability, increased binding affinity, or the like to a nucleic acid strand. In a specific embodiment, a nucleoside comprises a chemically modified ribofuranose ring moiety. Examples of a chemically modified ribofuranose ring comprise, but are not limited to, addition of a substituent (comprising 5' and 2' substituents), formation of a bicyclic nucleic acid (bridged nucleic acid, BNA) through bridge formation of a non-geminal ring atom, substitution of a ribosyl ring oxygen atom with S, N(R), or C(R1)(R2) (wherein R, R1, and R2 independently represent H, a C₁-C₁₂ alkyl, or a protecting group), and a combination thereof.

Examples of the sugar-modified nucleoside comprise, but are not limited to, a nucleoside comprising a substituent such as 5'-vinyl, 5'-methyl (R or S), 5'-allyl (R or S), 4'-S, 2'-F(2'-fluorogroup), 2'-OCH₃ (2'-O-Me group or 2'-O-methyl group), 2'-O-[2-(N-methylcarbamoyl)ethyl] (2'-O-MCE group), and 2'-O-methoxyethyl (2'-O-MOE or 2-O(CH₂)₂OCH₃). A substituent at the 2' position may be selected from allyl, amino, azide, thio, -O-allyl, -O-C₁-C₁₀ alkyl, -OCF₃, -O(CH₂)₂SCH₃, -O(CH₂)₂-O-N(Rm)(Rn), and O-CH₂-C(=O)-N(Rm)(Rn), wherein Rm and Rn are independently H or a substituted or unsubstituted C₁-C₁₀ alkyl. A "2'-modified sugar" means a furanosyl sugar modified at the 2' position. A nucleoside comprising a 2'-modified sugar may be referred to as a "2'-modified nucleoside" or a "2'-sugar-modified nucleoside".

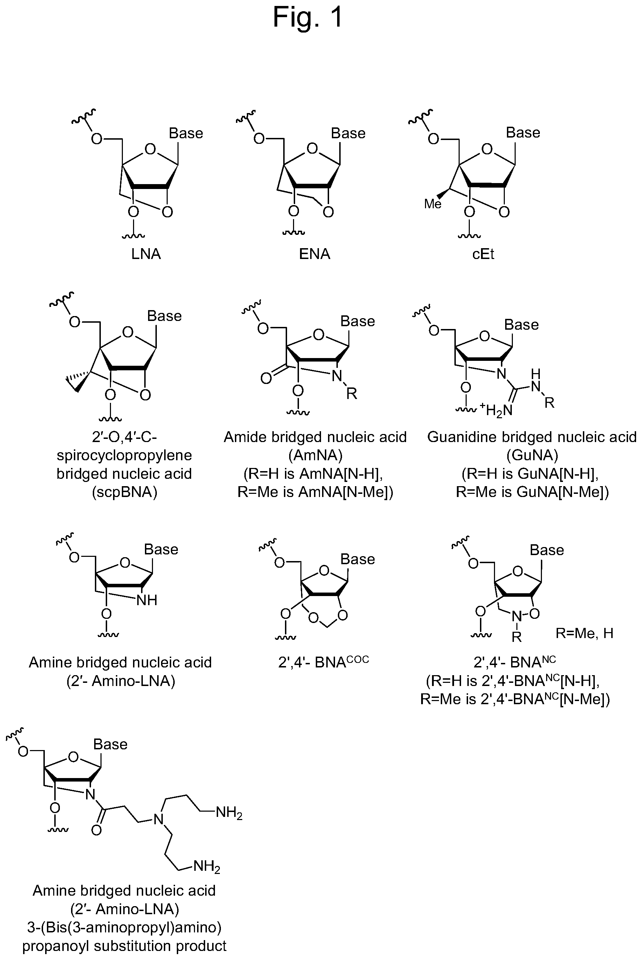

A "bicyclic nucleoside" refers to a modified nucleoside comprising a bicyclic sugar moiety. A nucleic acid comprising a bicyclic sugar moiety is commonly referred to as bridged nucleic acid (BNA). A nucleoside comprising a bicyclic sugar moiety is sometimes referred to as a "bridged nucleoside", "bridged-type non-natural nucleoside", or "BNA nucleoside". Some examples of a bridged nucleic acid are shown in Figure 1.

A bicyclic sugar may be a sugar in which the carbon atom at the 2' position and the carbon atom at the 4' position are bridged with two or more atoms. Examples of a bicyclic sugar are known to those skilled in the art. A subgroup of nucleic acids comprising a bicyclic sugar (BNA) or of BNA nucleosides may be described as having a carbon atom at the 2' position and a carbon atom at the 4' position which are bridged with 4'-(CH₂)ₚ-O-2', 4'-(CH₂)ₚ-CH₂-2', 4'-(CH₂)ₚ-S-2', 4'-(CH₂)ₚ-OCO-2', 4'-(CH₂)ₙ-N(R₃)-O-(CH₂)ₘ-2' [wherein p, m, and n represent integers from 1 to 4, from 0 to 2, and from 1 to 3, respectively; and R₃ represents a hydrogen atom, an alkyl group, an alkenyl group, a cycloalkyl group, an aryl group, an aralkyl group, an acyl group, a sulfonyl group, and a unit substituent (e.g., a fluorescently or chemiluminescently labeled molecule, a functional group with nucleic acid cleavage activity, and an intracellular or nuclear localization signal peptide)]. Furthermore, with respect to a BNA or a BNA nucleoside in a certain embodiment, in the OR₂ substituent of the carbon atom at the 3' position and the OR₁ substituent of the carbon atom at the 5' position, R₁ and R₂ are typically hydrogen atoms, but may be the same or different from each other, or may also be a protecting group for a hydroxyl group for nucleic acid synthesis, an alkyl group, an alkenyl group, a cycloalkyl group, an aryl group, an aralkyl group, an acyl group, a sulfonyl group, a silyl group, a phosphate group, a phosphate group protected by a protecting group for nucleic acid synthesis, or P(R₄)R₅ [wherein R₄ and Rs, may be the same or different from each other, and respectively represent a hydroxyl group, a hydroxyl group protected by a protecting group for nucleic acid synthesis, a mercapto group, a mercapto group protected by a protecting group for nucleic acid synthesis, an amino group, a C₁-C₅ alkoxy group, a C₁-C₅ alkylthio group, a C₁-C₆ cyanoalkoxy group, or an amino group substituted with a C₁-C₅ alkyl group]. Non-limiting examples of such BNA comprise methyleneoxy(4'-CH₂-O-2') BNA (LNA, Locked Nucleic Acid^{®}, also known as 2',4'-BNA) (e.g., α-L-methyleneoxy(4'-CH₂-O-2') BNA or β-D-methyleneoxy(4'-CH₂-O-2') BNA), ethyleneoxy(4'-(CH₂)₂-O-2') BNA (also known as ENA), β-D-thio(4'-CH₂-S-2') BNA, aminooxy(4'-CH₂-O-N(R₃)-₂') BNA, oxyamino(4'-CH₂-N(R₃)-O-2') BNA (also known as 2',4'-BNA^{NC}; R=H is 2',4'-BNA^{NC}[N-H], R=Me is 2',4'-BNA^{NC}[N-Me]), 2',4'-BNAcoc, 3'-amino-2',4'-BNA, 5'-methyl BNA, (4'-CH(CH₃)-O-2') BNA (also known as cEt BNA), (4'-CH(CH₂OCH₃)-O-2') BNA (also known as cMOE BNA), amide BNA (amide-bridged nucleic acid) or (4'-C(O)-N(R)-2') BNA (R=H, or Me) (also known as AmNA; R=H in Figure 1 is AmNA[N-H], R=Me is AmNA[N-Me]), guanidine BNA (also known as GuNA (e.g., R=H is GuNA[N-H], R=Me is GuNA[N-Me]) in Figure 1), amine BNA (also known as 2'-Amino-LNA) (e.g., 3-(Bis(3-aminopropyl)amino)propanoyl substitution product), 2'-O,4'-C-spirocyclopropylene-bridged nucleic acid (also known as scpBNA), and other BNA known to those skilled in the art. Non-limiting examples of such BNA nucleoside comprise methyleneoxy(4'-CH₂-O-2') BNA nucleoside (also known as LNA nucleoside or 2',4'-BNA nucleoside) (e.g., α-L-methyleneoxy(4'-CH₂-O-2') BNA nucleoside, β-D-methyleneoxy(4'-CH₂-O-2') BNA nucleoside), ethyleneoxy(4'-(CH₂)₂-O-2') BNA nucleoside (also known as ENA nucleoside), β-D-thio(4'-CH₂-S-2') BNA nucleoside, aminooxy(4'-CH₂-O-N(R₃)-2') BNA nucleoside, oxyamino(4'-CH₂-N(R₃)-O-2') BNA nucleoside (also known as 2',4'-BNA^{NC} nucleoside; R=H is 2',4'-BNA^{NC}[N-H] nucleoside, and R=Me is 2',4'-BNA^{NC}[N-Me] nucleoside), 2',4'-BNA^{COC} nucleoside, 3'-amino-2',4'-BNA nucleoside, 5'-methyl BNA nucleoside, (4'-CH(CH₃)-O-2') BNA nucleoside (also known as cEt nucleoside), (4'-CH(CH₂OCH₃)-O-2') BNA nucleoside (also known as cMOE nucleoside), amide BNA nucleoside or (4'-C(O)-N(R)-2') BNA nucleoside (R=H, or Me) (also known as AmNA nucleoside; R=H in Figure 1 is AmNA[N-H] nucleoside, and R=Me is AmNA[N-Me] nucleoside)), guanidine BNA nucleoside (GuNA nucleoside (e.g., R=H in Figure 1 is also known as GuNA[N-H] nucleoside, and R=Me is also known as GuNA[N-Me] nucleoside)), amine BNA nucleoside (also known as 2'-Amino-LNA nucleoside) (e.g., 3-(Bis(3-aminopropyl)amino)propanoyl-substituted nucleoside), 2'-O,4'-C-spirocyclopropylene-bridged nucleoside (also known as scpBNA nucleoside), and other BNA nucleosides known to those skilled in the art.

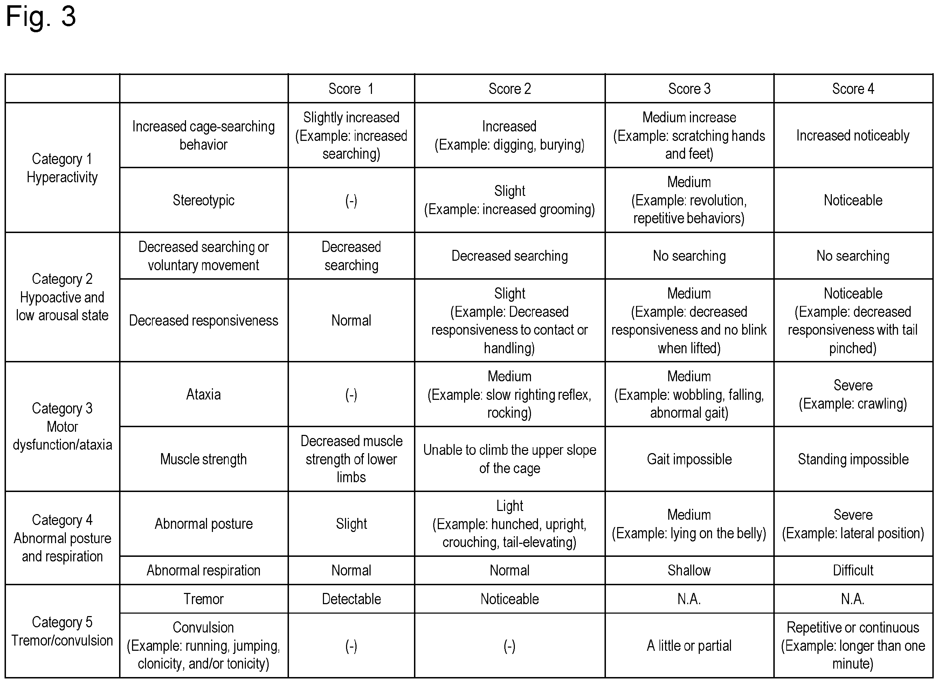

A "cationic nucleoside" herein is a modified nucleoside existing in cationic form, compared with a neutral form (such as the neutral form of ribonucleoside), at a pH (e.g., the physiological pH (approximately 7.4) of a human, a pH of a delivery site (e.g., an organelle, cell, tissue, organ, or organism), or the like). The cationic nucleoside may comprise one or more cationic modified groups at any position of a nucleoside. In one embodiment, the cationic nucleoside is 2'-Amino-LNA nucleoside (e.g., 3-(Bis(3-aminopropyl)amino)propanoyl-substituted nucleoside), aminoalkyl-modified nucleoside (e.g., 2'-O-methyl- and 4'-CH₂CH₂CH₂NH₂-substituted nucleoside), GuNA nucleoside (e.g., R=H in Figure 3 is GuNA[N-H] nucleoside, and R=Me is GuNA[N-Me] nucleoside), or the like. A bicyclic nucleoside having a methyleneoxy(4'-CH₂-O-2') bridge is also referred to as LNA nucleoside.

A "modified internucleoside linkage" means herein an internucleoside linkage that has a substitution or any change from a naturally occurring internucleoside linkage (i.e., phosphodiester linkage). A modified internucleoside linkage comprises an internucleoside linkage that comprises a phosphorus atom, and an internucleoside linkage that does not comprise a phosphorus atom. Typical examples of the phosphorus-containing internucleoside linkage comprise, but are not limited to, a phosphodiester linkage, a phosphorothioate linkage, a phosphorodithioate linkage, a phosphotriester linkage (a methylphosphotriester linkage and an ethylphosphotriester linkage described in U.S. Pat. No. 5,955,599), an alkylphosphonate linkage (e.g., a methylphosphonate linkage described in U.S. Pat. Nos. 5,264,423 and 5,286,717, and a methoxypropylphosphonate linkage described in WO2015/168172), an alkylthiophosphonate linkage, a methylthiophosphonate linkage, a boranophosphate linkage, an internucleoside linkage comprising a cyclic guanidine moiety (e.g., a partial structure represented by the following Formula (I): 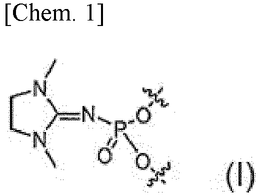 ), an internucleoside linkage comprising a guanidine moiety substituted with one to four C₁₋₆ alkyl groups (e.g., a tetramethyl guanidine (TMG) moiety) (e.g., a partial structure represented by the following Formula (II): 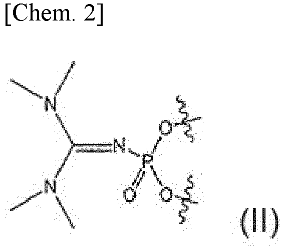 ), and an internucleoside linkage and a phosphoramidate linkage that are to be used for a self-neutralizing nucleic acid (ZON) as described in WO2016/081600. A phosphorothioate linkage refers to an internucleoside linkage in which an unbridged oxygen atom in a phosphodiester linkage is substituted with a sulfur atom. A method for preparing a phosphorus-containing and a phosphorus-free linkage is well known. It is preferable that a modified internucleoside linkage is a linkage having a higher resistance to a nuclease than a naturally occurring internucleoside linkage.

When an internucleoside linkage has a chiral center, the internucleoside linkage may be chirally controlled. The term "chirally controlled" means that a single diastereomer is present with respect to the chiral center, e.g., chirally bound phosphorus. An internucleoside linkage chirally controlled may be completely chirally pure, or may have a high chiral purity, e.g., a chiral purity of 90% de, 95% de, 98% de, 99% de, 99.5% de, 99.8% de, 99.9% de, or more. A "chiral purity" herein refers to the proportion of one diastereomer in a diastereomer mixture, is represented as the diastereomeric excess rate (% de), and is defined as (diastereomer of interest - Other diastereomers)/(Total diastereomer) × 100 (%).

For example, the internucleoside linkage may be a phosphorothioate linkage chirally controlled to an Rp configuration or Sp configuration, an internucleoside linkage comprising a guanidine moiety substituted with one to four C₁₋₆ alkyl groups (e.g., a tetramethyl guanidine (TMG) moiety; see, e.g., Alexander A. Lomzov et al., Biochem Biophys Res Commun., 2019, 513 (4), 807-811), and/or an internucleoside linkage comprising a cyclic guanidine moiety. A method for preparing an internucleoside linkage chirally controlled is publicly known, and a phosphorothioate linkage chirally controlled to an Rp configuration or Sp configuration can be synthesized according to, e.g., a method described in Naoki Iwamoto et al., Angew. Chem. Int. Ed. Engl. 2009, 48(3), 496-9, Natsuhisa Oka et al., J. Am. Chem. Soc. 2003, 125, 8307-8317, Natsuhisa Oka et al., J. Am. Chem. Soc. 2008, 130, 16031-16037, Yohei Nukaga et al., J. Org. Chem. 2016, 81, 2753-2762, or Yohei Nukaga et al., J. Org. Chem. 2012, 77, 7913-7922. A phosphorothioate linkage chirally controlled to an Rp configuration or Sp configuration is also publicly known, and is known to produce an effect described in, e.g., Naoki Iwamoto et al., Nat. Biotechnol,. 2017, 35(9), 845-851, or Anastasia Khvorova et al., Nat. Biotechnol., 2017, 35(3), 238-248. For example, in one embodiment, a chirally controlled phosphorothioate linkage in the Sp configuration is more stable than those in the Rp configuration and/or chirally controlled ASOs in the Sp configuration promote target RNA cleavage by RNase H1, resulting in a more sustained response in vivo. A method for preparing an internucleoside linkage comprising a guanidine moiety substituted with one to four C₁₋₆ alkyl groups (e.g., a TMG moiety) is publicly known, and the internucleoside linkage can be synthesized, e.g., in accordance with the method described in Alexander A. Lomzov et al., Biochem Biophys Res Commun., 2019, 513 (4), 807-811.

The term "nucleobase" or "base" used herein is a base component (heterocyclic moiety) constituting a nucleic acid. As the component, mainly adenine, guanine, cytosine, thymine, and uracil are known. The "nucleobase" or "base" herein encompasses both of a modified and an unmodified nucleobase (base), unless otherwise specified. Accordingly, a purine base may be any of a modified and an unmodified purine base, unless otherwise specified. In addition, a pyrimidine base may be any of a modified and an unmodified pyrimidine base, unless otherwise specified.

A "modified nucleobase" or a "modified base" means any nucleobase other than adenine, cytosine, guanine, thymine, or uracil. The term "unmodified nucleobase" or "unmodified base" (a natural nucleobase) means adenine (A) and guanine (G), which are purine bases, and thymine (T), cytosine (C), and uracil (U), which are pyrimidine bases. Examples of a modified nucleobase comprise, but are not limited to, hypoxanthine, 5-methylcytosine, 5-fluorocytosine, 5-bromocytosine, 5-iodocytosine, and N4-methylcytosine; N6-methyladenine or 8-bromoadenine; 2-thio-thymine; and N2-methylguanine or 8-bromoguanine. The modified nucleobase is preferably 5-methylcytosine.

The term "complementary" as used herein refers to the relationship that nucleobases can form via hydrogen bonds so-called Watson-Crick base pairs (natural base pairs) or non-Watson-Crick base pairs (e.g., Hoogsteen base pairs). In the present invention, the antisense oligonucleotide region of the first nucleic acid strand is not necessarily required to be completely complementary to at least a part of a target transcription product (e.g., the transcription product of a target gene), and it is acceptable if the base sequence has at least 70%, preferably at least 80%, and further preferably at least 90% (e.g., 95%, 96%, 97%, 98%, or 99% or more) of complementarity. The antisense oligonucleotide region in the first nucleic acid strand can hybridize to a target transcription product when the base sequence is complementary (typically when the base sequence is complementary to the base sequence of at least a part of the target transcription product). Similarly, the complementary region in the second nucleic acid strand is not necessarily required to be completely complementary to at least a part of the first nucleic acid strand, and it is acceptable if the base sequence has a complementarity of at least 70%, preferably at least 80%, and further preferably at least 90% (e.g., 95%, 96%, 97%, 98%, or 99% or more). The complementary region in the second nucleic acid strand can be annealed when the base sequence of the region is complementary to the base sequence of at least a part of the first nucleic acid strand. Base sequence complementarity can be determined by using a BLAST program or the like. Those skilled in the art can easily determine the conditions (temperature, salt concentration, and the like) under which two strands can anneal or hybridize, taking into account the degree of complementarity between the strands. Furthermore, those skilled in the art can easily design an antisense nucleic acid complementary to a target transcription product, e.g., based on information about the base sequence of a target gene.

Hybridization conditions may be variously stringent, e.g., low-stringent and high-stringent conditions. Low-stringent conditions may be relatively low temperature and high salt concentration conditions, e.g., 30°C, 2 × SSC, 0.1% SDS. High-stringent conditions may be relatively high temperature and low salt concentration conditions, e.g., 65°C, 0.1 × SSC, 0.1% SDS. The stringency of hybridization can be adjusted by changing conditions such as temperature and salt concentration. In this connection, 1 × SSC comprises 150 mM sodium chloride and 15 mM sodium citrate.

Herein, "toxicity" refers to an effect that induces an objective or subjective symptom or dysfunction that is not preferable to a subject, examples of such an effect including death, pain, tremor, convulsion, movement disorder, cognitive dysfunction, consciousness disorder, general malaise, lassitude, nausea, vomiting, vertigo, numbness, or wobble. The toxicity may be toxicity in any organ. The toxicity may be neurotoxicity.

Herein, "neurotoxicity" refers to an effect that induces damage in a nervous tissue including a central nervous tissue and a peripheral nervous tissue, and prevents the normal activity of the nervous system. Neurotoxicity can induce a symptom selected from death, respiratory abnormality, cardiovascular abnormality, headache, nausea or vomiting, unresponsiveness or low responsiveness, consciousness disorder, psychiatric disorder, character change, hallucination, delusion, cognitive dysfunction, abnormal posture, involuntary movement, shivering, convulsion, hyperactivity motor dysfunction, paralysis, sensory disorder, or autonomic nervous system dysfunction. The neurotoxicity may be acute neurotoxicity. Acute neurotoxicity can be neurotoxicity caused within 1, 3, 6, 9, 12, 24, or 48 hours after administration. For example, as described below in Examples, toxicity can be evaluated with an acute tolerability score, side-effect event rate, or death rate.

Herein, "central nervous system toxicity" refers to an effect that induces damage in at least a central nervous tissue among nervous tissues, and prevents the normal activity of the nervous system.

A "subject" herein refers to the object to which the double-stranded nucleic acid complex or pharmaceutical composition of the present invention is applied. A subject comprises an individual as well as an organ, a tissue, and a cell. When the subject is an individual, any animal including a human may be applicable. For example, in addition to a human, a variety of domestic animals, domestic fowls, pets, and laboratory animals are included. Without limitation, the subject may be an individual in need of a decrease in the expression amount of a target transcription product, or an individual in need of treatment or prevention of a disease.

### 1-3. Configuration

The double-stranded nucleic acid complex of the present invention comprises a first nucleic acid strand and a second nucleic acid strand. The specific configuration of each nucleic acid strand is described below.

In a double-stranded nucleic acid complex of the present invention, the first nucleic acid strand is capable of hybridizing to at least part of a target gene or a transcription product thereof, and has an antisense effect on the target gene or a transcription product thereof, and the second nucleic acid strand comprises a base sequence complementary to the first nucleic acid strand, and comprises one or more 2'-modified nucleoside.

The number of the 2'-modified nucleosides comprised in the second nucleic acid strand is at least one, and equal to or smaller than the number of all the nucleosides constituting the second nucleic acid strand (i.e., the base length of the second nucleic acid strand). The specific number of the 2'-modified nucleosides comprised in the second nucleic acid strand may be, e.g., 2 or more, 3 or more, 4 or more, 5 or more, 6 or more, 7 or more, 8 or more, 9 or more, or 10 or more, and may be 30 or less, 25 or less, 20 or less, 15 or less, 10 or less, 9 or less, 8 or less, 7 or less, 6 or less, 5 or less, 4 or less, 3 or less, or 2 or less. For example, the number of the 2'-modified nucleosides in the second nucleic acid strand may be 1 to 30, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9, 1 to 8, 1 to 7, or 1 to 6. For example, the number may be 1, 2, 3, 4, 5, or 6.

In one embodiment, 1% or more, 2% or more, 3% or more, 4% or more, 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 99% or more and/or 100% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, or 5% or less of the nucleosides in the second nucleic acid strand are 2'-modified nucleosides. For example, 10% to 90%, 20% to 80%, 30% to 70%, or 40% to 60% may be 2'-modified nucleosides.

A nucleoside other than a 2'-modified nucleoside comprised in the second nucleic acid strand may be a natural nucleoside, a non-natural nucleoside such as a bridged nucleoside, or any combination thereof. The number of the nucleosides comprised in the second nucleic acid strand, and other than a 2'-modified nucleoside is not limited, and may be, e.g., 1 or more, 2 or more, 3 or more, 4 or more, 5 or more, 6 or more, 7 or more, 8 or more, 9 or more, or 10 or more, may be, e.g., 1 to 40, 1 to 30, 1 to 20, 1 to 15, 1 to 12, 1 to 10, 1 to 8, or 1 to 6, may be, e.g., 1 to 5, or may be, e.g., 1, 2, 3, 4, or 5.

In one embodiment, the second nucleic acid strand comprises one 2'-modified nucleoside. In another embodiment, all of the nucleosides constituting the second nucleic acid strand are 2'-modified nucleosides. For example, in the second nucleic acid strand, all of the nucleosides in a region which consists of a base sequence complementary to the first nucleic acid strand may be 2'-modified nucleosides. In a further embodiment, the second nucleic acid strand comprises one or more 2'-modified nucleosides which are less than all of the nucleosides of the strand. Herein, that the second nucleic acid strand "comprises ... 2'-modified nucleosides which are less than all of the nucleosides" means that the second nucleic acid strand comprises at least any one nucleoside other than a 2'-modified nucleoside.

In one embodiment, the second nucleic acid strand comprises one or a plurality of consecutive 2'-modified nucleosides at the 5' end. Herein, the phrase "comprises ... a plurality of consecutive 2'-modified nucleosides" means comprising a plurality of 2'-modified nucleosides linked via any internucleoside linkage. For example, the second nucleic acid strand may comprise 1 or consecutive 2 to 12, 2 to 10, 2 to 8, 2 to 6, 2 to 5, 2 to 4, or 2 to 3, e.g., 2, 3, or 4 2'-modified nucleosides at the 5' end.

In one embodiment, the second nucleic acid strand comprises one or a plurality of consecutive 2'-modified nucleosides at the 3' end. For example, the second nucleic acid strand may comprise 1 or consecutive 2 to 12, 2 to 10, 2 to 8, 2 to 6, 2 to 5, 2 to 4, or 2 to 3, e.g., 2, 3, or 4 2'-modified nucleosides at the 3' end.

In a further embodiment, the second nucleic acid strand comprises one or a plurality of consecutive 2'-modified nucleosides at the 5' end and one or a plurality of consecutive 2'-modified nucleosides at the 3' end.

In one embodiment, the second nucleic acid strand comprises a 2'-modified nucleoside at a position other than the 5' end and the 3' end. Herein, that the second nucleic acid strand "comprises a 2'-modified nucleoside at a position other than the 5' end and the 3' end" means that the second nucleic acid strand comprises a 2'-modified nucleoside at a position other than the positions of the above-described one or a plurality of consecutive 2'-modified nucleosides at the 5' end and one or a plurality of consecutive 2'-modified nucleosides at the 3' end. For example, the second nucleic acid strand comprises 1 to 30, 1 to 25, 1 to 20, 1 to 15, 1 to 12, 1 to 10, 1 to 9, 1 to 8, 1 to 7, 1 to 6, 1 to 5, 1 to 4, or 1 to 3, e.g., 1 or 2 2'-modified nucleosides at a position(s) other than the 5' end and the 3' end.

In one embodiment, the second nucleic acid strand comprises one or more nucleosides other than a 2'-modified nucleoside. In a further embodiment, in the second nucleic acid strand (e.g., in the region consisting of a base sequence complementary to the first nucleic acid strand), all of the nucleosides other than 2'-modified nucleosides can be deoxyribonucleosides.

In one embodiment, the 2'-modified nucleoside(s) is/are a 2'-O-methoxyethyl-modified nucleoside and/or a 2'-O-methyl-modified nucleoside. The 2'-O-methoxyethyl-modified nucleoside is represented by the following Formula (III): 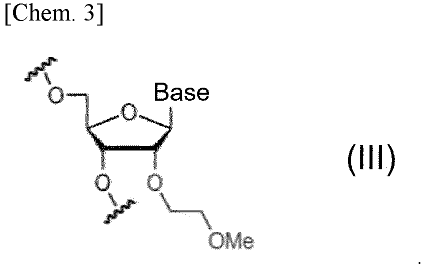 In addition, the 2'-O-methyl-modified nucleoside is represented by the following Formula (IV): 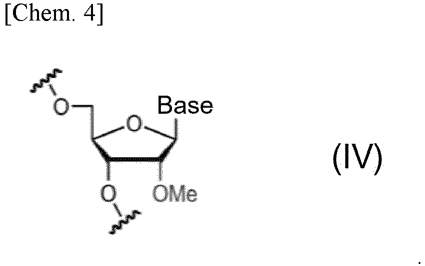 In one embodiment, the 2'-modified nucleoside is a 2'-O-methyl-modified nucleoside. In a more preferable embodiment, the 2'-modified nucleoside is a 2'-O-methoxyethyl-modified nucleoside.

In one embodiment, all of the nucleosides in the first nucleic acid strand may be non-natural nucleosides or modified nucleosides. In a further embodiment, all of the nucleosides in the first nucleic acid strand may be 2'-modified nucleosides. In a further embodiment, all of the nucleosides in the first nucleic acid strand may be 2'-O-methoxyethyl-modified nucleosides.

The first nucleic acid strand can comprise at least four, at least five, at least six, or at least seven consecutive nucleosides that are recognized by RNase H, when hybridized to a target transcription product. Typically, the nucleosides may be a region comprising 4 to 20, 5 to 16, or 6 to 12 consecutive nucleosides. As a nucleoside recognized by RNase H, e.g., a natural deoxyribonucleoside can be used. The modified deoxyribonucleoside and a suitable nucleoside comprising another base are well known in the art. In addition, it is also known that, as the nucleoside, a nucleoside having a hydroxy group at the 2' position, such as a ribonucleoside, is unsuitable. In connection with use for this region comprising "at least four consecutive nucleosides", the compatibility of a nucleoside can be readily determined. In one embodiment, the first nucleic acid strand can comprise at least four consecutive deoxyribonucleosides.

In one embodiment, the nucleosides in the first nucleic acid strand comprise or consist of a deoxyribonucleoside. For example, 70% or more, 80% or more, 90% or more, or 95% or more of the nucleosides in the first nucleic acid strand are deoxyribonucleosides.

In one embodiment, the first nucleic acid strand may be a gapmer. Herein, the "gapmer" refers to a single-stranded nucleic acid that, in principle, consists of a central region (DNA gap region) and wing regions positioned directly at the 5' end and 3' end of the central region (the wing regions are referred to as a 5'-wing region and a 3'-wing region respectively). The length of the DNA gap region may be 13 to 22 bases in length, 16 to 22 bases in length, 16 to 20 bases in length, 4 to 20 bases in length, 5 to 18 bases in length, 6 to 16 bases in length, 7 to 14 bases in length, or 8 to 12 bases in length. The central region in the gapmer comprises at least three or at least four consecutive deoxyribonucleosides, and the wing region comprises at least one non-natural nucleoside. Without limitation, a non-natural nucleoside comprised in the wing region usually has a higher bonding force to RNA than a natural nucleoside, and has high resistance to a nucleic acid-degrading enzyme (nuclease or the like). When a non-natural nucleoside constituting the wing region comprises or consists of a bridged nucleoside, the gapmer is specifically referred to as a "BNA/DNA gapmer". The number of bridged nucleosides comprised in the 5'-wing region and the 3'-wing region may be at least one, e.g., two or three. The bridged nucleosides comprised in the 5'-wing region and the 3'-wing region may be consecutively or inconsecutively present in the 5'-wing region and the 3'-wing region. The bridged nucleoside may further comprise a modified nucleobase (e.g., 5-methylcytosine). When the bridged nucleoside is an LNA nucleoside, the gapmer is referred to as an "LNA/DNA gapmer". When a non-natural nucleoside constituting the 5'-wing region and the 3'-wing region comprises or consists of a peptide nucleic acid, the gapmer is specifically referred to as a "peptide nucleic acid gapmer". When a non-natural nucleoside constituting the 5'-wing region and the 3'-wing region comprises or consists of a morpholino nucleic acid, the gapmer is specifically referred to as a "morpholino nucleic acid gapmer". The base length of each of the 5'-wing region and the 3'-wing region may be independently at least 2 bases in length, e.g., 2 to 10 bases in length, 2 to 7 bases in length, or 3 to 5 bases in length. In one embodiment, the 5'-wing region and/or the 3'-wing region may comprise at least one kind of non-natural nucleoside, and may further comprise a natural nucleoside. The 5'-wing region and the 3'-wing region may be, e.g., non-natural nucleosides linked via a modified internucleoside linkage such as a phosphorothioate linkage, examples of the nucleosides including: bridged nucleosides such as LNA nucleosides; and 2'-modified nucleosides such as 2'-O-methyl-modified nucleosides.

The central region (DNA gap region) in the gapmer comprises at least three or at least four consecutive deoxyribonucleosides, and the wing regions (5'-wing region and 3'-wing region) comprise at least one non-natural nucleoside. The position of the boundary between the central region (DNA gap region) and the wing region in the gapmer can be easily determined from the sequence of the nucleoside by those skilled in the art. For example, the central region can be functionally defined as a region recognizable by RNase H1. Here, the phrase "recognizable by RNase H1" means that, when a gapmer is bound to a target RNA, the sequence that is in the target RNA, and paired with the gapmer can be cleaved by RNase H1. Accordingly, in the gapmer, a region recognizable by RNase H1 can be regarded as the central region, and a region not recognizable by RNase H1 can be regarded as a wing region (5'-wing region and 3'-wing region), so that the position of the boundary can be determined. Herein, in the 5'-wing region and the 3'-wing region, the nucleosides adjacent to the central region are non-natural nucleosides, and in the central region, the nucleoside adjacent to the 5'-wing region or the 3'-wing region is a natural nucleoside.

The first nucleic acid strand constituting the gapmer may be composed of bridged nucleosides having 2 to 7 bases in length or 3 to 5 bases in length (e.g., 2 or 3 bases in length), ribonucleosides or deoxyribonucleosides having 4 to 15 bases in length or 8 to 12 bases in length (e.g., 8 or 10 bases in length), and bridged nucleosides 2 to 7 bases in length or 3 to 5 bases in length (e.g., 2 or 3 bases in length) in this order from the 5' end.

In this regard, a nucleic acid strand having a wing region on only one of the 5' end side or 3' end side is referred to as a "hemi-gapmer" in the art, but herein, the hemi-gapmer is also encompassed in the gapmer.

In one embodiment, the second nucleic acid strand comprises a 2'-modified nucleoside in a region which consists of a base sequence complementary to the 5'-wing region and/or the 3'-wing region in the first nucleic acid strand. In a further embodiment, in the second nucleic acid strand, all of the nucleosides in a region which consists of a base sequence complementary to the 5'-wing region and/or the 3'-wing region in the first nucleic acid strand can be 2'-modified nucleosides. The 2'-modified nucleoside may be, e.g., a 2'-O-methoxyethyl-modified nucleoside or a 2'-O-methyl-modified nucleoside.

In one embodiment, in the second nucleic acid strand, a nucleoside which comprises a purine base in a region which consists of a base sequence complementary to the central region in the first nucleic acid strand may be a ribonucleoside. For example, in the second nucleic acid strand, all of the nucleosides which comprise a purine base in a region which consists of a base sequence complementary to the central region in the first nucleic acid strand can be ribonucleosides.

In one embodiment, in the second nucleic acid strand, a nucleoside which comprises a pyrimidine base in a region which consists of a base sequence complementary to the central region in the first nucleic acid strand may be a deoxyribonucleoside. For example, in the second nucleic acid strand, all of the nucleosides which comprise a pyrimidine base in a region which consists of a base sequence complementary to the central region in the first nucleic acid strand can be deoxyribonucleosides.

In a further embodiment, in the second nucleic acid strand, all of the nucleosides comprising a purine base and all of the nucleosides comprising a pyrimidine base, in a region consisting of a base sequence complementary to the central region in the first nucleic acid strand, can be ribonucleosides and deoxyribonucleosides respectively.

In a preferable embodiment, in the second nucleic acid strand, all of the nucleosides in a region consisting of a base sequence complementary to the 5'-wing region and/or the 3'-wing region in the first nucleic acid strand can be 2'-modified nucleosides (e.g., 2'-O-methoxyethyl-modified nucleosides or 2'-O-methyl-modified nucleosides), and furthermore, in the second nucleic acid strand, all of the nucleosides comprising a purine base and all of the nucleosides comprising a pyrimidine base, in a region consisting of a base sequence complementary to the central region in the first nucleic acid strand, can be ribonucleosides and deoxyribonucleosides respectively.

In one embodiment, in the second nucleic acid strand, a nucleoside complementary to (i) at least one guanosine nucleoside in the first nucleic acid strand, (ii) a nucleoside adjacent to the 5' end side of said guanosine nucleoside, (iii) a nucleoside adjacent to the 3' end side of said guanosine nucleoside, or (iv) any combination of (i) to (iii) above may be a 2'-modified nucleoside. In a further embodiment, in the second nucleic acid strand, nucleosides complementary to (i) all guanosine nucleosides in the first nucleic acid strand, (ii) nucleosides adjacent to the 5' end side of said guanosine nucleosides, (iii) nucleosides adjacent to the 3' end side of said guanosine nucleosides, or (iv) any combination of (i) to (iii) above may be 2'-modified nucleosides.

In one embodiment, in the second nucleic acid strand, a nucleoside complementary to (i) at least one guanosine nucleoside in the 3'-wing region and/or the 5'-wing region of the first nucleic acid strand, (ii) a nucleoside adjacent to the 5' end side of said guanosine nucleoside, (iii) a nucleoside adjacent to the 3' end side of said guanosine nucleoside, or (iv) any combination of (i) to (iii) above may be a 2'-modified nucleoside. In a further embodiment, in the second nucleic acid strand, nucleosides complementary to (i) all guanosine nucleosides in the 3'-wing region and/or the 5'-wing region of the first nucleic acid strand, (ii) nucleosides adjacent to the 5' end side of said guanosine nucleosides, (iii) nucleosides adjacent to the 3' end side of said guanosine nucleosides, or (iv) any combination of (i) to (iii) above may be 2'-modified nucleosides.

In one embodiment, in the second nucleic acid strand, all of the nucleosides in a region consisting of a base sequence complementary to the central region of the first nucleic acid strand are (a) deoxyribonucleosides, (b) deoxyribonucleosides and ribonucleosides, (c) deoxyribonucleosides and 2'-modified nucleosides, (d) ribonucleosides and 2'-modified nucleosides, or (e) deoxyribonucleosides, ribonucleosides, and 2'-modified nucleosides.

In one embodiment, all of the nucleosides in the regions which consist of a base sequence complementary to the 5'-wing region and the 3'-wing region in the first nucleic acid strand are 2'-modified nucleosides, and all of the nucleosides in the region which consists of a base sequence complementary to the central region in the first nucleic acid strand can be deoxyribonucleosides.

In one embodiment, at least one nucleoside comprising a pyrimidine base in the second nucleic acid strand may be a 2'-modified nucleoside and/or a deoxyribonucleoside. In a further embodiment, the second nucleic acid strand does not comprise a natural ribonucleoside comprising a pyrimidine base, and for example, all of the nucleosides comprising a pyrimidine base in the second nucleic acid strand may be 2'-modified nucleosides and/or deoxyribonucleosides.

In one embodiment, the second nucleic acid strand may comprise a modified nucleoside other than a 2'-modified nucleoside, in addition to a 2'-modified nucleoside. For example, the second nucleic acid strand may comprise at least one 2'-O-methyl-modified nucleoside represented by the above-described Formula (IV), an scpBNA nucleoside represented by the below-described Formula (V), an AmNA nucleoside represented by the below-described Formula (VI), or an oxyamino (4'-CH₂-N(R₃)-O-2') BNA nucleoside (also known as a 2',4'-BNA^{NC} nucleoside; R=H is a 2',4'-BNA^{NC}[N-H] nucleoside, and R=Me is a 2',4'-BNA^{NC}[N-Me] nucleoside). 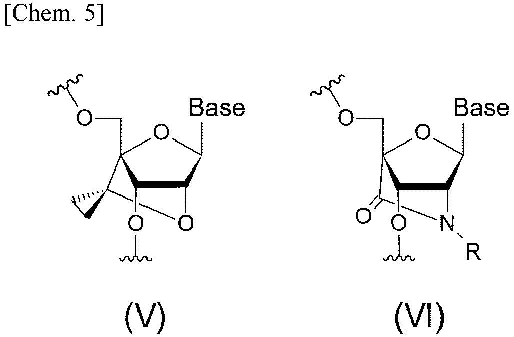 (wherein R represents a hydrogen atom or a methyl group).

A bridged non-natural nucleoside represented by the above-described Formula (V) is a 2'-O,4'-C-spirocyclopropylene bridged nucleic acid, and is herein mainly referred to as "scpBNA". In addition, a bridged non-natural nucleoside represented by the above-described Formula (VI) is amide BNA (an amide bridged nucleic acid), and can be referred to as (4'-C(O)-N(R)-2')BNA(R=H, Me), but is herein mainly referred to as "AmNA". In the above-described Formula (VI), R may be either a hydrogen atom or a methyl group. Unless otherwise specified herein, R may be either a hydrogen atom or a methyl group, but to distinguish between both, R can be referred to as AmNA[N-H] when it is a hydrogen atom, and R can be referred to as AmNA[N-Me] when it is a methyl group.

In a double-stranded nucleic acid complex of the present invention, the first nucleic acid strand may be a mixmer. The term "mixmer" refers to herein a nucleic acid strand that comprises natural nucleosides and non-natural nucleosides with periodically or randomly alternating segment lengths, and does not comprise four or more consecutive deoxyribonucleosides or ribonucleosides. Among mixmers, a mixmer in which the non-natural nucleoside is a bridged nucleoside, and the natural nucleoside is a deoxyribonucleoside is specifically referred to as a "BNA/DNA mixmer". The bridged nucleoside may be a bridged non-natural nucleoside represented by the above-described Formula (V) or Formula (VI). Among mixmers, a mixmer in which the non-natural nucleoside is a peptide nucleic acid and the natural nucleoside is a deoxyribonucleoside is specifically called a "peptide nucleic acid/DNA mixmer". Among mixmers, a mixmer in which the non-natural nucleoside is a morpholino nucleic acid, and the natural nucleoside is a deoxyribonucleoside is specifically referred to as a "morpholino nucleic acid/DNA mixmer". A mixmer is not restricted to comprise only two kinds of nucleosides. A mixmer may comprise any number of kinds of nucleosides irrespective of a natural or modified nucleoside, or a nucleoside mimic. For example, a mixmer may comprise one or two consecutive deoxyribonucleosides separated by a bridged nucleoside (e.g., an LNA nucleoside or a bridged non-natural nucleoside represented by the above-described Formula (V) or Formula (VI)). The bridged nucleoside may further comprise a modified nucleobase (e.g., 5-methylcytosine).

In one embodiment, the second nucleic acid strand may comprise at least four consecutive ribonucleosides complementary to the above-described at least four consecutive nucleosides (e.g., deoxyribonucleosides) in the central region in the first nucleic acid strand. This is in order that the second nucleic acid strand can form a partial DNA-RNA heteroduplex with the first nucleic acid strand, and be recognized and cleaved by RNase H. The at least four consecutive ribonucleosides in the second nucleic acid strand are linked preferably via a naturally-occurring internucleoside linkage, i.e., a phosphodiester linkage.

In a further embodiment, the second nucleic acid strand may comprise at least two consecutive deoxyribonucleosides in addition to the at least four consecutive ribonucleosides. The at least two consecutive deoxyribonucleosides are complementary to the first nucleic acid strand, and may be comprised in a region complementary to the central region of the first nucleic acid strand. The at least two consecutive deoxyribonucleosides may be positioned on either the 5' side or the 3' side of the at least four consecutive ribonucleosides, and may be positioned on both the 5' side and the 3' side. In addition, the at least two consecutive deoxyribonucleosides may be two, three, four, five, or six or more consecutive deoxyribonucleosides.

At least one, at least two (e.g., two), at least three, or at least four nucleosides from the end (5' end, 3' end, or both ends) of the second nucleic acid strand may be modified nucleosides. Modified nucleosides may comprise modified sugars and/or modified nucleobases. The modified sugar may be a 2'-modified sugar (e.g., a sugar comprising a 2'-O-methyl group). The modified nucleobase may also be 5-methylcytosine.

The second nucleic acid strand may be composed of, in order from the 5' end, modified nucleosides of from 2 to 7 bases in length or from 3 to 5 bases in length (e.g., 2 or 3 bases in length) (e.g., modified nucleosides comprising a 2'-modified sugar), ribonucleosides or deoxyribonucleosides (optionally linked by a modified internucleoside linkage) of from 4 to 15 bases in length or from 8 to 12 bases in length (e.g., 8 or 10 bases in length), and modified nucleosides of from 2 to 7 bases in length or from 3 to 5 bases in length (e.g., 2 or 3 bases in length) (e.g., modified nucleosides comprising a 2'-modified sugar). In this case, the first nucleic acid strand may be a gapmer.

The first nucleic acid strand and/or the second nucleic acid strand may comprise, as a whole or in part, a nucleoside mimic or a nucleotide mimic. The nucleotide mimic may be a peptide nucleic acid and/or a morpholino nucleic acid.

In one embodiment, the second nucleic acid strand may comprise a non-complementary base, and/or one or more insertion sequences and/or deletions, relative to the first nucleic acid strand. The number of non-complementary bases in the second nucleic acid strand is not limited, and may be, e.g., 1 to 10, 1 to 9, 1 to 8, 1 to 7, 1 to 6, 1 to 5, 1 to 4, 1 to 3, 1 to 2, or 1 or 2. The sequence consisting of non-complementary bases may form the below-described bulge structure. The base length of the insertion sequence in the second nucleic acid strand is not limited, and may be, e.g., 1 to 10, 1 to 9, 1 to 8, 1 to 7, 1 to 6, 1 to 5, 1 to 4, 1 to 3, 1 to 2, or 1 or 2. The insertion sequence may be a sequence that forms the below-described bulge structure. The base length of consecutive deletions in the second nucleic acid strand is not limited, and may be, e.g., 1 to 10, 1 to 9, 1 to 8, 1 to 7, 1 to 6, 1 to 5, 1 to 4, 1 to 3, 1 to 2, or 1 or 2. The second nucleic acid strand may comprise the below-described bulge structure at the position of deletion.

The base length of the first nucleic acid strand and the second nucleic acid strand are not particularly limited, and may be at least 8 bases in length, at least 9 bases in length, at least 10 bases in length, at least 11 bases in length, at least 12 bases in length, at least 13 bases in length, at least 14 bases in length, or at least 15 bases in length. Furthermore, the base length of the first nucleic acid strand and the second nucleic acid strand may be 35 bases in length or less, 30 bases in length or less, 25 bases in length or less, 24 bases in length or less, 23 bases in length or less, 22 bases in length or less, 21 bases in length or less, 20 bases in length or less, 19 bases in length or less, 18 bases in length or less, 17 bases in length or less, or 16 bases in length or less. The first nucleic acid strand and the second nucleic acid strand may have identical lengths or different lengths (for example, one of them is shorter or longer by 1 to 3 bases). In one embodiment, the length of the second nucleic acid strand is shorter than the length of the first nucleic acid strand. In this case, the second nucleic acid strand may be bound to the first nucleic acid strand at any position. For example, the second nucleic acid strand may be bound to any of the 5' side region, the central region, and the 3' side region in the first nucleic acid strand. In one embodiment, the second nucleic acid strand may be at least 8 bases in length. The double-stranded structure formed by the first nucleic acid strand and the second nucleic acid strand may comprise a bulge. The length can be determined according to the balance between the strength of the antisense effect and the specificity of the nucleic acid strand to the target, among other factors such as cost, and synthesis yield.

In one embodiment, the second nucleic acid strand may further comprise at least one overhang region located on one or both of the 5' end side and the 3' end side thereof. The term "overhang region" refers to a region adjacent to a region that is in the second nucleic acid strand, and is complementary to the first nucleic acid strand, namely a nucleotide region in the second nucleic acid strand in which the 5' end of the second nucleic acid strand extends beyond the 3' end of the first nucleic acid strand and/or the 3' end of the second nucleic acid strand extends beyond the 5' end of the first nucleic acid strand when the first nucleic acid strand and the second nucleic acid strand are annealed to form a double-stranded structure, or protruding from the double-stranded structure. The overhang region in the second nucleic acid strand may be located at the 5' end side of the complementary region or at the 3' end side. The overhang region in the second nucleic acid strand may be located at the 5' end side and 3' end side of the complementary region.

The length of the overhang region is not limited, and may be 1 to 20 bases in length, and may be, e.g., 2 to 15 bases in length, 2 to 12 bases in length, 2 to 10 bases in length, 2 to 8 bases in length, 2 to 6 bases in length, 2 to 5 bases in length, 2 to 4 bases in length, or 2 to 3 bases in length. In addition, the kind of the nucleosides constituting the overhang region is not limited. For example, the nucleosides may be composed of a natural nucleoside (e.g., a deoxyribonucleoside) or a non-natural nucleoside (e.g., a bridged nucleoside, such as an LNA nucleoside). In addition, all or part of the internucleoside linkages in the overhang region may be modified internucleoside linkages. The modified internucleoside linkage may be, e.g., a phosphorothioate linkage. The overhang region preferably has protein affinity, liposolubility, and/or nuclease resistance, and may be, e.g., composed of deoxyribonucleosides or LNA nucleosides linked by a phosphorothioate linkage. In this regard, the base sequence of the overhang region may be a sequence unrelated to the base sequence of a target gene.

At least one, at least two (e.g., two), at least three, or at least four nucleosides from the end (5' end, 3' end, or both ends) of the second nucleic acid strand may be non-natural nucleosides (modified nucleosides). Modified nucleosides may comprise modified sugars and/or modified nucleobases. The modified sugar may be a 2'-modified sugar (e.g., a sugar comprising a 2'-O-methyl group). The modified nucleobase may also be 5-methylcytosine.

In one embodiment, the second nucleic acid strand may have 1 to 8, 1 to 7, 1 to 6, 1 to 5, 1 to 4, or 1 to 3 (e.g., 1 to 2, or 1) non-complementary bases, 1 to 8, 1 to 7, 1 to 6, 1 to 5, 1 to 4, or 1 to 3 (e.g., 1 to 2, or 1) deleted bases, and/or 1 to 20 (e.g., 1 to 15, 1 to 12, 1 to 10, 1 to 8, 1 to 6, 1 to 4, 1 to 3, or 1) inserted bases, relative to the first nucleic acid strand, as long as the second nucleic acid strand and the first nucleic acid strand can form a double strand. The sequence region consisting of inserted bases may form a bulge structure.

In one embodiment, the second nucleic acid strand comprises at least one bulge structure consisting of a base sequence not complementary to the first nucleic acid strand. Herein, the "bulge structure" refers to a part of the nucleic acids of any one of the nucleic acid strands constituting the double strand in the double-stranded nucleic acid, wherein the part is protruded from the double-stranded structure without base-pairing. The base length of the bulge structure is not limited. For example, the base length is 1 to 50 bases in length, 1 to 40 bases in length, 1 to 30 bases in length, 1 to 20 bases in length, 1 to 15 bases in length, or preferably 1 to 10 bases in length.

In one embodiment, the bulge structure comprises a sugar-unmodified nucleoside. In a further embodiment, all of nucleosides in the bulge structure may be sugar-unmodified nucleosides.

In one embodiment, when the second nucleic acid strand comprises a bulge structure, all of nucleosides other than the bulge structure may be 2'-modified nucleosides.

The internucleoside linkage in the first nucleic acid strand and the second nucleic acid strand may be a naturally occurring internucleoside linkage and/or a modified internucleoside linkage. Without limitation, at least one, at least two, or at least three internucleoside linkages from an end (5' end, 3' end or both the ends) of the first nucleic acid strand and/or the second nucleic acid strand are preferably modified internucleoside linkages. In this regard, e.g., two internucleoside linkages from the end of a nucleic acid strand refers to an internucleoside linkage closest to the end of the nucleic acid strand, and an internucleoside linkage positioned next thereto on the opposite side to the end of the nucleic acid strand. Modified internucleoside linkages in the terminal region of a nucleic acid strand are preferred because they can reduce or inhibit undesired degradation of the nucleic acid strand.

In one embodiment, all or part of the internucleoside linkages of the first nucleic acid strand and/or the second nucleic acid strand may be modified internucleoside linkages. In one embodiment, the first nucleic acid strand and/or the second nucleic acid strand may each comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more modified internucleoside linkages. In one embodiment, the first nucleic acid strand and/or the second nucleic acid strand may each comprise modified internucleoside linkages for at least 5%, at least 10%, at least 20%, at least 30%, at least 40%, at least 50%, at least 60%, at least 70%, at least 80%, at least 90%, at least 93%, at least 95%, at least 98%, or 100%. In one embodiment, the modified internucleoside linkage may be a phosphorothioate linkage or a boranophosphate linkage. In one embodiment, when the nucleic acid in the first nucleic acid strand consists of morpholino nucleic acids, all or part of the internucleoside linkages in the first nucleic acid strand may be phosphorothioate linkages.

In one embodiment, the first nucleic acid strand and/or the second nucleic acid strand may each comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more chirally controlled internucleoside linkages. In one embodiment, the first nucleic acid strand and/or the second nucleic acid strand may each comprise chirally controlled internucleoside linkages for at least 5%, at least 10%, at least 20%, at least 30%, at least 40%, at least 50%, at least 60%, at least 70%, at least 80%, at least 90%, or more.

In one embodiment, the first nucleic acid strand and/or the second nucleic acid strand may each comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more non-negatively charged internucleoside linkages (preferably neutral internucleoside linkages). In one embodiment, the first nucleic acid strand and/or the second nucleic acid strand may each comprise non-negatively charged internucleoside linkages for at least 5%, at least 10%, at least 20%, at least 30%, at least 40%, at least 50%, at least 60%, at least 70%, at least 80%, at least 90%, or more.

In one embodiment, at least one, at least two, or at least three internucleoside linkages from the 5' end of the second nucleic acid strand may be a modified internucleoside linkage(s). At least one, at least two, or at least three internucleoside linkages from the 3' end of the second nucleic acid strand may be a modified internucleoside linkage(s), e.g., a phosphorothioate linkage, an internucleoside linkage comprising a guanidine moiety (e.g., a TMG moiety) substituted with one to four C₁₋₆ alkyl groups, and/or an internucleoside linkage comprising a cyclic guanidine moiety. The modified internucleoside linkage may be chirally controlled to an Rp configuration or Sp configuration.

At least one (e.g., three) internucleoside linkage from the 3' end of the second nucleic acid strand may be a modified internucleoside linkage such as a phosphorothioate linkage having a high resistance to an RNase. It is preferable that the second nucleic acid strand comprises a modified internucleoside linkage such as a phosphorothioate modification at the 3' end, because the gene suppression activity of the double-stranded nucleic acid complex is improved.

In one embodiment, a modified internucleoside linkage of the first nucleic acid strand and/or the second nucleic acid strand comprises a non-negatively charged (neutral or cationic) internucleoside linkage present in a neutral form or cationic form respectively at a pH (e.g., the physiological pH (approximately 7.4) of a human, the pH of a delivery site (e.g., an organelle, cell, tissue, organ, organism, or the like), or the like), compared with the anionic form (e.g., -OP(O)(O⁻)-O- (the anionic form of a natural phosphate linkage), -O-P (O)(S⁻)-O- (the anionic form of a phosphorothioate linkage), or the like). In one embodiment, the modified internucleoside linkage of the first nucleic acid strand and/or the second nucleic acid strand comprises a neutral internucleoside linkage. In one embodiment, the modified internucleoside linkage of the first nucleic acid strand and/or the second nucleic acid strand comprises a cationic internucleoside linkage. In one embodiment, a non-negatively charged internucleoside linkage (e.g., a neutral internucleoside linkage), when in the neutral form thereof, does not have a moiety the pKa of which is less than 8, less than 9, less than 10, less than 11, less than 12, less than 13, or less than 14. In one embodiment, the non-negatively charged internucleoside linkage is, e.g., an internucleoside linkage or the like to be used for a methylphosphonate linkage described in U.S. Pat. Nos. 5,264,423 and 5,286,717, a methylphosphotriester linkage and an ethylphosphotriester linkage described in U.S. Pat. No. 5,955,599, a methoxypropylphosphonate linkage described in WO2015/168172, and a self-neutralizing nucleic acid (ZON) described in WO2016/081600. In one embodiment, the non-negatively charged internucleoside linkage comprises a triazole moiety or an alkyne moiety. In one embodiment, the non-negatively charged internucleoside linkage comprises a cyclic guanidine moiety and/or a guanidine moiety (preferably a TMG moiety) substituted with one to four C₁₋₆ alkyl groups. In one embodiment, the modified internucleoside linkage comprising a cyclic guanidine moiety has a partial structure represented by Formula (I). In one embodiment, the guanidine moiety substituted with one to four C₁₋₆ alkyl groups has a partial structure represented by Formula (II). In one embodiment, the neutral internucleoside linkage comprising a cyclic guanidine moiety and/or a guanidine moiety substituted with one to four C₁₋₆ alkyl groups is chirally controlled. In one embodiment, the present disclosure relates to a composition comprising an oligonucleotide comprising at least one neutral internucleoside linkage and at least one phosphorothioate internucleoside linkage. Without wishing to be bound by any specific theory, but at least in some cases, the neutral internucleotide linkage can improve characteristics and/or activity, e.g., improve delivery, improve resistance to exonuclease and endonuclease, improve cellular uptake, improve endosomal escape, and/or improve nucleic uptake, compared with an equivalent nucleic acid comprising no neutral internucleotide linkage.

In one embodiment, the second nucleic acid strand may be bound to a ligand (herein sometimes referred to as a binder). Examples of the ligand include a small molecule (low-molecular weight ligand), a middle molecule (middle molecular weight ligand), a macromolecule (high molecular weight ligand), a peptide (peptide ligand), a lipid (lipid ligand), and an aptamer (e.g., a nucleic acid aptamer).

A "peptide" herein refers to an amino acid polymer having one or more peptide bonds. The "peptide" is not limited by the number of amino acid residues comprised in the peptide. Accordingly, the "peptide" encompasses an oligopeptide comprising several amino acid residues, such as a dipeptide or a tripeptide, and a polypeptide (protein) comprising many amino acid residues. The peptide can be a linear, branched, or cyclic peptide.

The peptide ligand may be bound to a molecule present on the surface of a cell, in a cell, or in a body fluid.

In one embodiment, the peptide may be an antibody or an active fragment thereof. Examples of the antibody include a monoclonal antibody, a polyclonal antibody, a recombinant antibody such as a chimeric antibody or a humanized antibody, Fab, F(ab')₂, Fab', VHH, and the like. Examples of the active fragment of an antibody include an scFv (single chain Fragment of variable region: single chain antibody), a diabody, a triabody, a tetrabody, or the like.

Examples of the lipid (lipid ligand) include, but are not limited to, tocopherol, cholesterol, fatty acid, phospholipid, and analogs thereof; folic acid, vitamin C, vitamin B1, vitamin B2; estradiol, androstane, and analogs thereof; steroid and an analog thereof; ligand of LDLR, SRBI or LRP1/2; FK-506, and cyclosporine; lipids described in PCT/JP2019/012077, PCT/JP2019/010392, and PCT/JP2020/035117. In addition, the lipid (lipid ligand) may be a tocopherol or an analog thereof and/or a cholesterol or an analog thereof, a substituted or unsubstituted C₁₋₃₀ alkyl group, a substituted or unsubstituted C₂₋₃₀ alkenyl group, or a substituted or unsubstituted C₁₋₃₀ alkoxy group.

"Tocopherol" is herein a methylated derivative of tocorol which is a liposoluble vitamin (vitamin E) having a cyclic structure called chroman. Tocorol has a strong antioxidant effect, and therefore functions in vivo as an antioxidant substance to scavenge free radicals produced by metabolism and protect cells from damage.

A plurality of different types of tocopherol consisting of α-tocopherol, β-tocopherol, γ-tocopherol, and δ-tocopherol are known based on the position of the methyl group bound to chroman. A tocopherol herein may be any types of tocopherol. In addition, examples of the analog of tocopherol comprise various unsaturated analogs of tocopherol, such as α-tocotrienol, β-tocotrienol, γ-tocotrienol, and δ-tocotrienol. Preferably, tocopherol is α-tocopherol.

"Cholesterol" is herein a kind of sterol, also called steroid alcohol, which is especially abundant in animals. Cholesterol exerts an important function in the metabolic process in vivo, and in animal cells, it is also a major constituent of the membrane system of a cell, together with phospholipid. In addition, the cholesterol analog refers to various cholesterol metabolism products and their analogs, which are alcohols having a sterol backbone. Examples thereof include, but are not limited to, cholestanol, lanosterol, cerebrosterol, dehydrocholesterol, and coprostanol.

An "analog" herein refers to a compound having a similar structure and property having the same or a similar basic backbone. The analog comprises, e.g., a biosynthetic intermediate, a metabolism product, and a compound having a substituent. Those skilled in the art can determine whether or not a compound is an analog of another compound based on common general technical knowledge.

The cholesterol analog refers to various cholesterol metabolism products and their analogs, which are alcohols having a sterol backbone. Examples thereof include, but are not limited to, cholestanol, lanosterol, cerebrosterol, dehydrocholesterol, and coprostanol.

In one embodiment, the second nucleic acid strand may be bound to tocopherol or cholesterol, or an analog thereof. The second nucleic acid strand bound to cholesterol or an analog thereof may have a group represented by the following general Formula (VII). 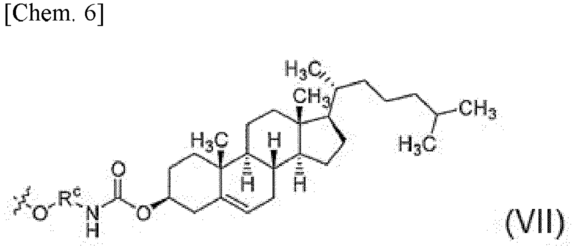 [wherein, R^{c} represents a C₄-C₁₈, preferably C₅-C₁₆, alkylene group optionally having a substituent (here, the substituent is a halogen atom or a C₁-C₃ alkyl group optionally substituted with a hydroxy group, in which such an alkyl group is, e.g., a hydroxymethyl group; and in the alkylene group, a carbon atom not mutually adjacent to another carbon atom may be substituted with an oxygen atom).]

R^{c} is not limited, and may be -(CH₂)₃-O-(CH₂)₂-O-(CH₂)₂-O-(CH₂)₂-O-(CH₂)₂-, -(CH₂)₃-O-(CH₂)₂-O-(CH₂)₂-O-(CH₂)₂-O-CH₂-CH(CH₂OH)-, or -(CH₂)₆-.

A group represented by the above-described general Formula (VII) can be bound to the 5' end or 3' end of the second nucleic acid strand via a phosphoester linkage.

The ligand that is cholesterol, an analog thereof, or the like may be bound to any of the 5' end, the 3' end, and both ends of the second nucleic acid strand. In addition, the ligand that is cholesterol, an analog thereof, or the like may be bound to a nucleotide at an interior position in the second nucleic acid strand.

When the second nucleic acid strand comprises a plurality of cholesterols or analogs thereof, the cholesterols or analogs may be identical or different. In this case, e.g., cholesterol and another cholesterol analog are one each bound to the 5' end and 3' end of the second nucleic acid strand respectively. With regards to the binding position, the cholesterol or an analog thereof may be bound to a plurality of positions in the second nucleic acid strand, and/or may be bound, in the form of one group, to one position. One cholesterol or an analog thereof may be linked to each of the 5' end and 3' end of the second nucleic acid strand.

The bond between the second nucleic acid strand and the ligand may be a direct bond or an indirect bond that is mediated by another substance.

When the second nucleic acid strand and a ligand are directly bound, the ligand can be bound to the second nucleic acid strand, e.g., via a covalent bond, an ionic bond, a hydrogen bond, or the like. A covalent bond is preferable in view of the capability to form a more stable bond.

In one embodiment, the second nucleic acid strand is not bound to a ligand. Herein, being "not bound to a ligand" refers to being not bound to a ligand such as tocopherol or cholesterol. In a further embodiment, the double-stranded nucleic acid complex of the present invention is not bound to a ligand, i.e., neither the first nucleic acid strand nor the second nucleic acid strand is bound to a ligand.

When the second nucleic acid strand and a ligand are indirectly bound to each other, both of them may be bound via a linking group (herein often referred to as a "linker"). The linker may be any one of a cleavable linker and an uncleavable linker.

A "cleavable linker" refers to a linker that can be cleaved under physiological conditions, e.g., in a cell or in an animal body (e.g., in a human body). A cleavable linker is selectively cleaved by an endogenous enzyme such as a nuclease. Examples of a cleavable linker comprise, but are not limited to, an amide, an ester, one or both esters of a phosphodiester, a phosphoester, a carbamate, and a disulfide bond, as well as a natural DNA linker. In one example, cholesterol or an analog thereof may be linked via a disulfide bond.

An "uncleavable linker" refers to a linker that is not cleaved under physiological conditions, e.g., in a cell or in an animal body (e.g., in a human body). Examples of an uncleavable linker comprise, but are not limited to, a phosphorothioate linkage, modified or unmodified deoxyribonucleosides linked by a phosphorothioate linkage, and a linker consisting of modified or unmodified ribonucleosides. There is no particular restriction on the chain length, when a linker is a nucleic acid such as DNA, or an oligonucleotide, however, it may be usually from 2 to 20 bases in length, from 3 to 10 bases in length, or from 4 to 6 bases in length.

Specific examples of the linker comprise a linker represented by the following Formula (VIII). 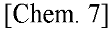 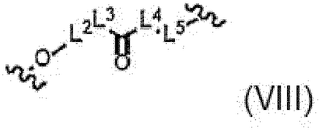 [wherein L² represents a substituted or unsubstituted C₁-C₁₂ alkylene group (e.g., propylene, hexylene, dodecylene), a substituted or unsubstituted C₃-C₈ cycloalkylene group (e.g., cyclohexylene), -(CH₂)₂-O-(CH₂)₂-O-(CH₂)₂-O-(CH₂)₃-, -(CH₂)₂-O-(CH₂)₂-O-(CH₂)₂-O-(CH₂)₂-O-(CH₂)₃-, or CH(CH₂-OH)-CH₂-O-(CH₂)₂-O-(CH₂)₂-O-(CH₂)₂-O-(CH₂)₃-, L³ represents -NH- or a bond, L⁴ represents a substituted or unsubstituted C₁-C₁₂ alkylene group (e.g., ethylene, pentylene, heptylene, or undecylene), a substituted or unsubstituted C₃₋₈ cycloalkylene group (e.g., cyclohexylene), -(CH₂)₂-[O-(CH₂)₂]ₘ-, or a bond, wherein m is an integer of 1 to 25, L⁵ represents -NH-(C=O)-, -(C=O)-, or a bond (here, the substitution is preferably carried out by a halogen atom).]

In one embodiment, regarding a linker represented by Formula (VIII), L² is unsubstituted C₃-C₆ alkylene group (e.g., propylene, hexylene), -CH₂)₂-O-(CH₂)₂-O-(CH₂)₂-O-(CH₂)₃-, or - (CH₂)₂-O-(CH₂)₂-O-(CH₂)₂-O-(CH₂)₂-O-(CH₂)₃-, L³ is -NH-, and L⁴ and L⁵ are bonds.

In addition, other specific examples of the linker comprise a linker represented by the following general Formula (IX). 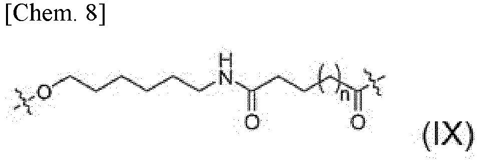 [wherein, n represents 0 or 1.]

The first nucleic acid strand and/or the second nucleic acid strand (preferably the second nucleic acid strand) may further comprise at least one functional moiety bound to a polynucleotide constituting the nucleic acid strand. A "functional moiety" refers to a moiety that provides a desired function to the double-stranded nucleic acid complex and/or the nucleic acid strand to which the functional moiety is bound. Examples of the desired function include a labeling function, a purification function, and the like. Examples of a moiety that provides a labeling function include compounds such as a fluorescent protein, and luciferase. In addition, examples of a moiety that provides a purifying function include compounds such as biotin, avidin, His-tag peptide, GST-tag peptide, and FLAG-tag peptide. In one embodiment, a molecule having an activity for delivery of a double-stranded nucleic acid complex in an embodiment to a target site is preferably bound as a functional moiety to the second nucleic acid strand, from the viewpoint that the first nucleic acid strand is efficiently delivered to a target site at a high specificity and expression of a target gene is very effectively suppressed by the nucleic acid. Examples of a moiety for providing a delivery function to a target include lipid, antibody, aptamer, and a ligand to a particular receptor. In one embodiment, the first nucleic acid strand and/or the second nucleic acid strand (preferably the second nucleic acid strand) is bound to a functional moiety. The linkage between the second nucleic acid strand and the functional moiety may be a direct linkage or an indirect linkage via another substance, and in some embodiments, it is preferable that the second nucleic acid strand and the functional moiety are directly bound by covalent bonding, ionic bonding, hydrogen bonding, or the like, and from the viewpoint of obtaining a more stable bond, covalent bonding is more preferable.

The first nucleic acid strand and the second nucleic acid strand may be linked via a linker. In this case, the first nucleic acid strand and the second nucleic acid strand can be linked via a linker to form a single strand. The double-stranded nucleic acid complex in this case can be referred to as a hinge nucleic acid, a single-stranded HDO, an ssHDO, or the like. However, even in that case, the functional region has the same configuration as the double-stranded nucleic acid complex, and therefore such a single-stranded nucleic acid is herein also encompassed as an embodiment of the double-stranded nucleic acid complex of the present invention.

In one embodiment, the 5' end of the first nucleic acid strand and the 3' end of the second nucleic acid strand are bound by the linker. In another embodiment, the 3' end of the first nucleic acid strand and the 5' end of the second nucleic acid strand are bound by the linker. In a further embodiment, the 5' end of the first nucleic acid strand and the 3' end of the second nucleic acid strand are bound by a linker, and the 3' end of the first nucleic acid strand and the 5' end of the second nucleic acid strand are bound by a linker. In this case, the double-stranded nucleic acid complex has a cyclic structure.

The linker can be any polymer. For example, a linker that links the second nucleic acid strand and a ligand as above-described can be used in addition to a polynucleotide, polypeptide, alkylene, or the like. Specifically, it can be composed of a natural nucleotide or nucleoside such as DNA or RNA, or a non-natural nucleotide or nucleoside such as a peptide nucleic acid or a morpholino nucleic acid. In addition, it may be composed of a polyether such as polyethylene glycol. When a linker consists of a nucleic acid, the chain length of a linker may be at least one base, or, e.g., a chain length of from 2 to 50 bases, from 2 to 40 bases, from 2 to 30 bases, from 2 to 20 bases, from 2 to 15 bases, from 2 to 12 bases, from 3 to 10 bases, or from 4 to 6 bases. It is preferably 4 bases in length. The position of the linker can be either on the 5' side or the 3' side of the first nucleic acid strand. For example, in the case of a configuration in which cholesterol or an analog thereof is bound to the 5' side of the second nucleic acid strand, the 5' end of the first nucleic acid strand and the 3' end of the second nucleic acid strand are linked via a linker. The linker may be either cleavable or uncleavable.

In a further embodiment, the first nucleic acid strand and the second nucleic acid strand are bound via a linker, and the second nucleic acid strand can comprise at least one bulge structure consisting of a base sequence not complementary to the first nucleic acid strand.

In the double-stranded nucleic acid complex of the present invention, the antisense effect on the target transcription product of the first nucleic acid strand can be measured by a method publicly known in the art. For example, after introducing a double-stranded nucleic acid complex into a cell and the like, it can be measured using a publicly known technique such as Northern blotting, quantitative PCR, or Western blotting. By measuring the expression level of a target gene or the level of a target transcription product in specific tissues (e.g., the amount of mRNA, the amount of RNA such as microRNA, the amount of cDNA, and the amount of protein), it can be judged whether or not the target gene expression is suppressed by the double-stranded nucleic acid complex in these sites. For example, regarding exon skipping, the effect can be determined by comparing a product produced by exon skipping with a product produced without exon skipping.

An exemplary embodiment of the double-stranded nucleic acid complex of the present invention has been described above, however, the double-stranded nucleic acid complex of the present invention is not limited to the above exemplary embodiment.

### 1-4. Method for producing a double-stranded nucleic acid complex

Those skilled in the art can produce the double-stranded nucleic acid complex of the present invention by appropriately selecting a publicly known method. Usually, without limitation, firstly each of the first nucleic acid strand and the second nucleic acid strand that constitute a double-stranded nucleic acid complex is designed and prepared. For example, the first nucleic acid strand is designed based on the information on the base sequence of the target transcription product (e.g., the base sequence of the target gene), and the second nucleic acid strand is designed as a complementary strand thereto. Then, based on the information on the designed base sequences, each nucleic acid strand is synthesized using a commercially available automatic nucleic acid synthesizer, such as that from GE Healthcare, Thermo Fisher Scientific, or Beckman Coulter. Thereafter, the prepared oligonucleotides may be purified using a reverse-phase column or the like.

In the case of a double-stranded nucleic acid complex to which a functional moiety is bound, a first nucleic acid strand may be produced according to the above method. Meanwhile, with respect to a second nucleic acid strand to which a functional moiety is bound, it may be produced by performing the aforedescribed synthesis and purification using a nucleic acid species to which a functional moiety has been bound in advance. For example, a second nucleic acid strand may be produced by performing the aforedescribed synthesis and purification using a nucleic acid species to which cholesterol or an analog thereof has been bound in advance. Alternatively, cholesterol or an analog thereof may be joined by a publicly known method to a second nucleic acid strand produced by performing the aforedescribed synthesis and purification. After preparation of each nucleic acid strand, a double-stranded nucleic acid complex to which the functional moiety of interest is bound can be produced by performing annealing for the first nucleic acid strand and the second nucleic acid strand. Specifically, the nucleic acids are mixed in an appropriate buffer solution to be denatured at about 90°C to 98°C for several minutes (e.g., 5 minutes), and then the nucleic acids are annealed in a range of about 30°C to 70°C for about one to eight hours to yield a double-stranded nucleic acid complex of the present invention. The method for linking a functional moiety to a nucleic acid is well known in the art. Furthermore, a nucleic acid strand can be obtained by ordering from various manufacturers (e.g., GeneDesign Inc.) by specifying the base sequence and the modification site and type.

### 1-5. Application of double-stranded nucleic acid complex

In one embodiment, the double-stranded nucleic acid complex of the present invention may be for at least one of the following effects in a subject: the effect of suppressing or increasing the expression level of a transcription product or a translation product of a target gene; the effect of inhibiting a function of a transcription product or a translation product of a target gene; the effect of regulating RNA splicing; and applications for the effect of inhibiting binding of a target gene to a protein, such as exon skipping. For example, the double-stranded nucleic acid complex of the present invention may be for at least one of the following effects: exon skipping, exon inclusion, steric blocking, and increasing RNA expression. The double-stranded nucleic acid complex of the present invention may be used for providing the above-described effect in a particular tissue, e.g., brain, spinal cord, kidney, liver, lung, intestinal tract, spleen, adrenal gland, eye, retina, skin, or peripheral nerves, such as a brain. The brain may be cerebrum, diencephalon, brain stem, or cerebellum, e.g., one or more of the following: cerebrum (e.g., cerebral cortex), brain stem, cerebellum, hippocampus, and striatum. The double-stranded nucleic acid complex of the present invention may be used to provide the above-described effect in a muscle tissue, including heart muscle and skeletal muscle.

In one embodiment, the double-stranded nucleic acid complex of the present invention is for disease or prevention. The disease may be skeletal muscle dysfunction or cardiac dysfunction. Examples of a disease include muscular dystrophies (Duchenne muscular dystrophy, myotonic dystrophy type 1 (DM1), Fukuyama muscular dystrophy, facioscapulohumeral muscular dystrophy, limb-girdle muscular dystrophy, or the like), congenital myopathies, primary age-related tauopathies (PART), Alzheimer's disease (AD), progressive supranuclear palsy (PSP), corticobasal degeneration/corticobasal syndrome (CBD), Pick's disease, frontotemporal dementia, neuroinclusion body disease, spinal muscular atrophy (SMA), amyotrophic lateral sclerosis (ALS), Parkinson's disease, Huntington's disease, hereditary spinocerebellar ataxia (SCA), multiple system atrophy, hereditary spastic paraplegia, multiple sclerosis, stroke, brain tumor, epilepsy, and encephalitis.

### 1-6. Effects

A double-stranded nucleic acid complex of the present invention can reduce or eliminate toxicity such as the central nervous system toxicity of a double-stranded nucleic acid complex without impairing the efficacy of the double-stranded nucleic acid complex. That is, the double-stranded nucleic acid complex has reduced central nervous system toxicity without impairing the antisense effect on a target gene, compared with a conventional double-stranded nucleic acid complex.

### 2. Pharmaceutical composition

### 2-1. Overview

A second aspect of the present invention is a pharmaceutical composition. The pharmaceutical composition of the present invention comprises the double-stranded nucleic acid complex described in said first aspect as an active ingredient. A pharmaceutical composition of the present invention has reduced central nervous system toxicity, and can be intrathecally or intraventricularly administered without involving a side effect.

Each component that a pharmaceutical composition of the present invention may comprise will be described in detail below.

### 2-2. Configuration

### 2-2-1. Active ingredient

The pharmaceutical composition of the present invention comprises as an active ingredient at least a double-stranded nucleic acid complex described in the first aspect. A pharmaceutical composition of the present invention may comprise two or more kinds of double-stranded nucleic acid complexes.

The amount (content) of the double-stranded nucleic acid complex in a pharmaceutical composition varies depending on the kind of the double-stranded nucleic acid complex, the delivery site, the dosage form of the pharmaceutical composition, the dose of the pharmaceutical composition, and the kind of a carrier described below. Therefore, it may be determined as appropriate by taking the respective conditions into consideration. Usually, it may be adjusted so that an effective amount of the double-stranded nucleic acid complex is comprised in a single dose of the pharmaceutical composition. An "effective amount" refers to an amount that is necessary for the double-stranded nucleic acid complex to function as an active ingredient, and to an amount that has little or no adverse side effect on a living body to which the amount is applied. This effective amount can vary depending on various conditions such as information on the subject, the administration route, and number of administrations. Ultimately, it may be determined by the judgment of a physician, veterinarian, pharmacist, or the like. "Information on the subject" is various information on an individual of the living body to which the pharmaceutical composition is applied. For example, when the subject is a human, it comprises age, body weight, gender, dietary habit, health status, stage of progression or grade of severity of the disease, drug sensitivity, and presence of a combined drug.

2-2-2. Carrier

The pharmaceutical composition of the present invention may comprise a pharmaceutically acceptable carrier. A "pharmaceutically acceptable carrier" refers to an additive commonly used in the field of pharmaceutical preparation. Examples thereof include a solvent, a vegetable oil, a base, an emulsifier, a suspending agent, a surfactant, a pH adjuster, a stabilizer, a seasoning, a flavor, an excipient, a vehicle, a preservative, a binder, a diluent, an isotonizing agent, a sedative, a bulking agent, a disintegrating agent, a buffering agent, a coating agent, a lubricant, a colorant, a sweetener, a thickener, a corrective agent, a dissolution aid, and other additives.

The solvent may be any of, e.g., water or other pharmaceutically acceptable aqueous solution, and a pharmaceutically acceptable organic solvent. Examples of an aqueous solution include a physiological saline, an isotonic solution comprising glucose or another additive, a phosphate-buffered saline, and a sodium acetate buffer solution. Examples of the additive include D-sorbitol, D-mannose, D-mannitol, sodium chloride, and further a nonionic surfactant at a low concentration, and polyoxyethylene sorbitan fatty acid ester.

The above carrier is used to avoid or decrease degradation of the double-stranded nucleic acid complex, which is an active ingredient, in vivo by an enzyme and the like, and additionally to facilitate formulation or administration, and to maintain the dosage form and drug efficacy. Therefore, it may be used as appropriate and as needed.

### 2-2-3. Dosage form

There is no particular restriction on the dosage form of the pharmaceutical composition of the present invention as long as the double-stranded nucleic acid complex described in the first aspect, which is an active ingredient, is delivered to a target site without being inactivated by degradation or the like, and the pharmacological effect (an antisense effect on the expression of a target gene) of the active ingredient can be produced in vivo.

The specific dosage form varies depending on the administration method and/or medication conditions. The administration methods can be broadly classified into parenteral administration and oral administration, and the dosage form appropriate for the respective administration methods can be selected.

When the administration method is parenteral administration, the preferred dosage form is liquid formulation which can be administered directly to the target site, or administered systemically via the circulatory system. Examples of the liquid formulation comprise an injectable. An injectable can be formulated by mixing in an appropriate combination with the aforedescribed excipient, elixir, emulsifier, suspending agent, surfactant, stabilizer, pH adjuster, etc. in the form of a unit dose required according to the generally approved pharmaceutical practices. In addition, it may be ointment, plaster, cataplasm, transdermal patch, lotion, inhalant, aerosol, eye drop, and suppository.

When the administration method is oral administration, the preferred dosage form may be a solid preparation or a liquid formulation. Examples include a tablet, capsule, drop, lozenge, pill, granule, dusting powder, powder, oral liquid preparation, emulsion, syrup, pellet, lingusorbs, peptizer, buccal, paste, suspending agent, elixir, coating agent, ointment, plaster, cataplasm, transdermal patch, lotion, inhalant, aerosol, eye drop, injectable, and suppository. In the case of a solid preparation, if necessary, it may take a dosage form with a coating as publicly known in the art, such as a sugar-coated tablet, a gelatin-coated tablet, an enteric-coated tablet, a film-coated tablets, a double layer tablet, and a multilayer tablet.

There is no particular restriction on the specific shape and size of each of the above-mentioned dosage forms, as long as the respective dosage forms are within the ranges of dosage forms publicly known in the art. As for the manufacturing method of the pharmaceutical composition of the present invention, it may be formulated according to the common procedure in the art.

In a specific embodiment, a double-stranded nucleic acid complex of the present invention has excellent quality as a pharmaceutical product having the following: excellent solubility in water, the second fluid for the dissolution test in accordance with the Japanese Pharmacopoeia, or the second fluid for the disintegration test in accordance with the Japanese Pharmacopoeia; excellent pharmacokinetics (e.g., the drug half-life in the blood, intracerebral transitivity, metabolic stability, and CYP inhibition); low toxicity (e.g., superior as a pharmaceutical product from the viewpoint of acute toxicity, chronic toxicity, genotoxicity, reproductive toxicity, cardiotoxicity, drug interaction, carcinogenicity, light toxicity, and the like); less side effects (e.g., suppression of sedation and avoidance of laminar necrosis); and the like.

### 2-3. Dosing form and dose

Herein there is no particular restriction on the preferable dosing form of a pharmaceutical composition. For example, the dosing form may be oral or parenteral administration. Specific examples of the parenteral administration include intramuscular administration, intravenous administration, intraarterial administration, intraperitoneal administration, subcutaneous administration (comprising implantable continuous subcutaneous administration), intradermal administration, trachea/bronchial administration, rectal administration, administration by blood transfusion, intraventricular administration, intrathecal administration, transnasal administration, and intramuscular administration. The intrathecal administration may be, e.g., suboccipital puncture or lumbar puncture.

When a pharmaceutical composition is applied by administration or ingestion, the administered amount or ingested amount may be, e.g., from 0.00001 mg/kg/day to 10000 mg/kg/day, or from 0.001 mg/kg/day to 100 mg/kg/day for the double-stranded nucleic acid complex comprised in the pharmaceutical composition. A pharmaceutical composition may be applied by single-dose administration or multiple dose administration. In the case of multiple dose administration, it may be administered daily or at appropriate time intervals (e.g., at intervals of one day, two days, three days, one week, two weeks, or one month), e.g., for 2 to 20 times. A single dose of the double-stranded nucleic acid complex described above may be, e.g., 0.001 mg/kg or more, 0.005 mg/kg or more, 0.01 mg/kg or more, 0.25 mg/kg or more, 0.5 mg/kg or more, 1 mg/kg or more, 2.5 mg/kg or more, 0.5 mg/kg or more, 1.0 mg/kg or more, 2.0 mg/kg or more, 3.0 mg/kg or more, 4.0 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more. For example, any amount in the range of from 0.001 mg/kg to 500 mg/kg (e.g., 0.001 mg/kg, 0.01 mg/kg, 0.1 mg/kg, 1 mg/kg, 5 mg/kg, 10 mg/kg, 50 mg/kg, 100 mg/kg, or 200 mg/kg) may be selected as appropriate.

The double-stranded nucleic acid complex of the present invention may be administered twice a week for total four times at a dose of from 0.01 to 10 mg/kg (e.g., about 6.25 mg/kg). Alternatively, the double-stranded nucleic acid complex may be administered once or twice a week for total two to four times, e.g., at a frequency of twice a week for total two times, at a dose of from 0.05 to 30 mg/kg (e.g., about 25 mg/kg). By adopting such a dosing regimen (divided administration), the toxicity can be lowered (e.g., avoidance of platelet reduction) compared to a single-dose administration at a higher dose, and the stress to the subject can be reduced.

Even when the pharmaceutical composition is repeatedly administered, its inhibitory effect can be produced additively in a cell. In the case of repeated administration, the efficacy can be improved with certain administration intervals (e.g., half a day or longer).

In one embodiment, a pharmaceutical composition of the present aspect is intraventricularly administered or intrathecally administered. When a pharmaceutical composition of the present aspect is intraventricularly administered or intrathecally administered, the pharmaceutical composition may be administered to a monkey or a human in an amount of 0.01 mg or more, 0.1 mg or more, or 1 mg or more, e.g., 2 mg or more, 3 mg or more, 4 mg or more, 5 mg or more, 10 mg or more, 20 mg or more, 30 mg or more, 40 mg or more, 50 mg or more, 75 mg or more, 100 mg or more, 200 mg or more, 300 mg or more, 400 mg or more, or 500 mg or more, or may be administered in an amount of 0.01 mg to 1000 mg, 0.1 mg to 200 mg, or 1 mg to 20 mg. The pharmaceutical composition may be administered to a mouse in an amount of 1 µg or more.

In one embodiment, a pharmaceutical composition of the present aspect is intravenously administered or subcutaneously administered. When a pharmaceutical composition of the present aspect is intravenously administered or subcutaneously administered, the pharmaceutical composition may be administered in an amount of 0.01 mg/kg or more, 0.1 mg/kg or more, or 1 mg/kg or more, e.g., 2 mg/kg or more, 3 mg/kg or more, 4 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more, or may be administered in an amount of 0.01 mg/kg to 1000 mg/kg, 0.1 mg/kg to 100 mg/kg, or 1 mg/kg to 10 mg/kg.

### 2-4. Disease as subject of application

A disease to which the pharmaceutical composition is applicable is not limited. The disease may be a disease which may be related to a gene for which the antisense effect of a double-stranded nucleic acid complex of the present invention can suppress or increase the expression amount of a transcription product or translation product or can inhibit the function of a transcription product or translation product of the gene, or can induce steric blocking, splicing switching, RNA editing, exon skipping, or exon inclusion. Specific examples of the disease are as described in "1-5. Application of double-stranded nucleic acid complex".

In one embodiment, a pharmaceutical composition in the present aspect can be used for treatment of a central nervous system disease in a subject. Examples of a central nervous system disease for which to use the pharmaceutical composition of the present aspect include, but are not particularly limited to, brain tumor, Alzheimer's disease, Parkinson's disease, amyotrophic lateral sclerosis, multiple sclerosis, Huntington's disease, and the like.

### 2-5. Effects

A pharmaceutical composition of the present invention possesses reduced central nervous system toxicity. Accordingly, a pharmaceutical composition of the present invention can achieve a preventive effect or a therapeutic effect without involving a side effect when intraventricularly or intrathecally administered.

In particular, treating a nervous disease such as Alzheimer's disease requires administering a nucleic acid agent at high doses, and has the risk of inducing a side effect, but a pharmaceutical composition of the present invention can significantly decrease such a side effect.

In addition, provided is a method for treating and/or preventing a disease such as a central nervous system disease, comprising administering the above-described double-stranded nucleic acid complex or pharmaceutical composition to a subject.

In addition, provided is use of a double-stranded nucleic acid complex of the present invention in the production of a medicine for treating and/or preventing a disease.

### Examples

The present invention is described below in more detail by means of Examples. However, the technical scope of the present invention is not limited to these Examples.

### <Example 1: MOE modifications in HDO targeting Mapt gene>

### (Purpose)

Through an in vivo experiment, the toxicity-reducing effect based on MOE modifications is investigated in terms of central nervous system toxicity observed in intraventricular administration of a heteroduplex oligonucleotide (hereinafter referred to as "HDO") comprising: a first nucleic acid strand consisting of an antisense nucleic acid (hereinafter referred to as "ASO") targeting the Mapt gene; and a second nucleic acid strand having a base sequence complementary to the first nucleic acid strand.

### (Method)

### (1) Preparation of nucleic acids

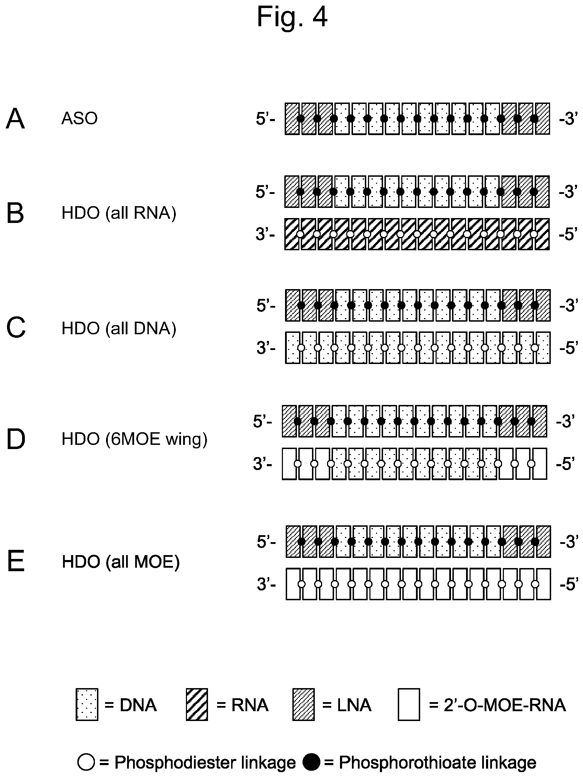

The base sequences and chemical modifications of ASO, and first nucleic acid strands and second nucleic acid strands constituting HDO used in this Example are shown in Table 1 and Figure 4.

**[Table 1]**

| Table 1: ASO and HDO targeting the Mapt gene | | | |
|---|---|---|---|
| | | Sequence (5'-3') | SEQ ID NO |
| ASO | ASO | a^t^a^g^t^c^a^c^t^c^t^g^g^t^g^a | 1 |
| HDO (all RNA) | ASO | a^t^a^g^t^c^a^c^t^c^t^g^g^t^g^a | 1 |
| | c(all RNA) | UCACCAGAGUGACUAU | 2 |
| HDO (all DNA) | ASO | a^t^a^g^t^c^a^c^t^c^t^g^g^t^g^a | 1 |
| | c(all DNA) | tcaccagagtgactat | 3 |
| HDO (6MOE wing) | ASO | a^t^a^g^t^c^a^c^t^c^t^g^g^t^g^a | 1 |
| | c(6MOE wing) | TC(5)AccagagtgacTAT | 4 |
| HDO (all MOE) | ASO | a^t^a^g^t^c^a^c^t^c^t^g^g^t^g^a | 1 |
| | c(all MOE) | TC(5)AC(5)C(5)AGAGTGAC(5)TAT | 5 |

| | | | |
|---|---|---|---|
| Lower case letter: DNA; Upper case letter: RNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is an LNA/DNA gapmer antisense nucleic acid targeting a microtubule-associated protein tau (Mapt) mRNA of a mouse, has a base sequence complementary to part of the Mapt mRNA, and has a structure wherein three LNA nucleosides at the 5' end, three LNA nucleosides at the 3' end, and ten DNA nucleosides therebetween are linked via a phosphorothioate linkage. All of HDO (all RNA), HDO (all DNA), HDO (6MOE wing), and HDO (all MOE) used in this Example comprise the ASO as the first nucleic acid strand, and the second nucleic acid strand has a sequence complementary to the first nucleic acid strand. Specifically, the second nucleic acid strand (c(all RNA)) of HDO (all RNA) has a structure wherein the RNA nucleosides are linked via a phosphodiester linkage. The second nucleic acid strand (c(all DNA)) of HDO (all DNA) has a structure wherein the DNA nucleosides are linked via a phosphodiester linkage. The second nucleic acid strand (c(6MOE wing)) of HDO (6MOE wing) has a structure wherein three 2'-O-MOE-RNA nucleosides at the 5' end, three 2'-O-MOE-RNA nucleosides at the 3' end, and ten DNA nucleosides therebetween are linked via a phosphodiester linkage. The second nucleic acid strand (c(all MOE)) of HDO (all MOE) has a structure wherein the 2'-O-MOE-RNA nucleosides are linked via a phosphodiester linkage.

In this regard, the 2'-O-MOE-RNA nucleoside used in Examples herein is a non-natural nucleoside represented by the following Formula (III):

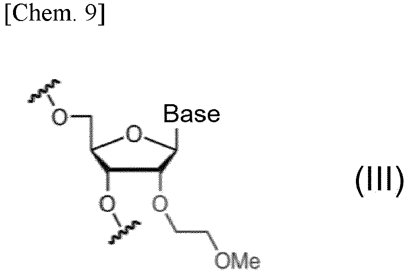

To prepare a double-stranded nucleic acid complex described in Table 1, the first nucleic acid strand and the second nucleic acid strand were mixed in equimolar amounts, and the solution was heated at 95°C for 5 minutes, then cooled to 37°C, and maintained for one hour, allowing the nucleic acid strands to be annealed to thereby prepare a double-stranded nucleic acid complex. The annealed nucleic acids were stored at 4°C or on ice. All the oligonucleotides were synthesized by GeneDesign Inc. (Osaka, Japan) on consignment.

### (2) In vivo experiment

Seven-week-old female ICR mice anesthetized with 2.5 to 4% isoflurane were fixed to a brain stereotaxic apparatus. Then, skin between the ears was cut open 2 to 3 cm anteroposteriorly, and perforated 1 mm leftward and 0.2 mm posteriorly from the bregma using a 1 mm diameter drill. A Hamilton syringe was filled with the nucleic acid agent. Through the perforated site, a needle was inserted approximately 3 mm deep. The nucleic acid agent at a dose of 19 nmol per mouse was administered into the left lateral ventricle at a rate of 2 to 3 µl/min (n = 4 to 7). The skin was sutured with a nylon thread. In addition, a mouse to which only PBS was administered was provided as a negative control group.

### (3) Evaluation of central nervous system toxicity and evaluation of motor function after administration of nucleic acid agents

Evaluation of central nervous system toxicity and evaluation of motor function were performed on mice to which various nucleic acid agents were administered.

In the evaluation of central nervous system toxicity, the behavior was evaluated, using the scoring system shown in Figure 3, at 30 minutes, 1 hour, 2 hours, 3 hours, and 4 hours after the administration of the nucleic acid agents.

According to the scoring system shown in Figure 3, the behaviors classified into five categories were evaluated (Categories 1 to 5 in Figure 3). Each category comprises two behavior evaluation items. Each behavior evaluation item is rated in five stages from 0 to 5 points (Scores 0 to 5 in Figure 3). A normal behavior is given 0 points, and higher toxicity is given a higher score. In each category, the higher score in the two behavior evaluation items is adopted as the score for the category. The total value of the scores for the five categories is defined as an acute tolerability score (0 to 20 points).

In addition, to evaluate the motor function, an open field test was performed at each point of time after the administration of various nucleic acid agents. Specifically, a mouse was placed in the center of a cage (50 cm in width × 50 cm in diameter × 40 cm in height), and the track of the mouse was recorded for 5 minutes. The total movement distance (m) and maximum moving speed (m/s) based on the data recorded were measured using video tracking software (ANY-maze). A significant statistical difference between the treated groups was evaluated in accordance with the Bonferroni test.

### (4) Evaluation of gene suppression effect

A hippocampus was extirpated from the mice at 7 days after the administration of various nucleic acid agents. Using an IsogenI kit (GeneDesign Inc.), RNA was extracted from the left hippocampus extirpated. A cDNA was synthesized using Transcriptor Universal cDNA Master, DNase (Roche Diagnostics) in accordance with the protocol.

Next, the resulting cDNA template was used to perform quantitative RT-PCR, whereby the expression levels of Mapt mRNA and Actb mRNA (the internal standard gene) were measured. Quantitative RT-PCR was performed with TaqMan (Roche Applied Science). Primers used in quantitative RT-PCR were products designed and manufactured by Thermo Fisher Scientific Inc. (formerly known as Life Technologies Corp.). Amplification conditions (temperature and time) were as follows: the cycle, 95°C for 15 seconds, 60°C for 30 seconds, and 72°C for 1 second, was repeated 40 times.

The ratio of the expression level of Mapt mRNA to the expression level of Actb mRNA (internal standard gene) was calculated, and a value standardized with respect to the value of the PBS-treated group was determined as a relative Mapt mRNA level.

### (Results)

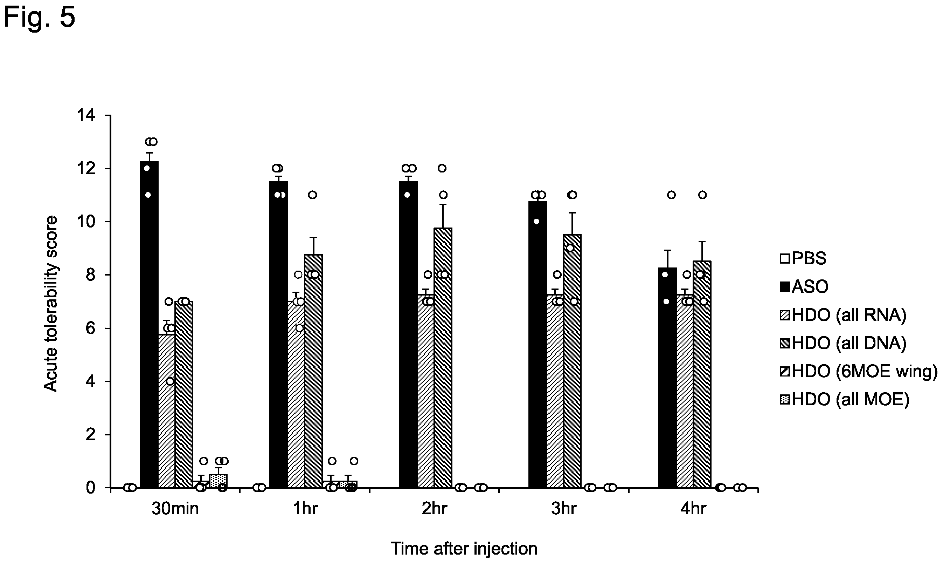

Figure 5 shows the results of evaluation of the central nervous system toxicity in mice to which various nucleic acid agents were intraventricularly administered. At any point of time of 30 minutes, 1 hour, 2 hours, 3 hours, and 4 hours after the administration of the nucleic acid agent, the acute tolerability score was decreased noticeably with the groups treated with HDO (6MOE wing) and HDO (all MOE), compared with the groups treated with ASO, HDO (all RNA), and HDO (all DNA). This result has revealed that the HDOs comprising a 2'-O-MOE-RNA nucleoside achieves a noticeable decrease in central nervous system toxicity.

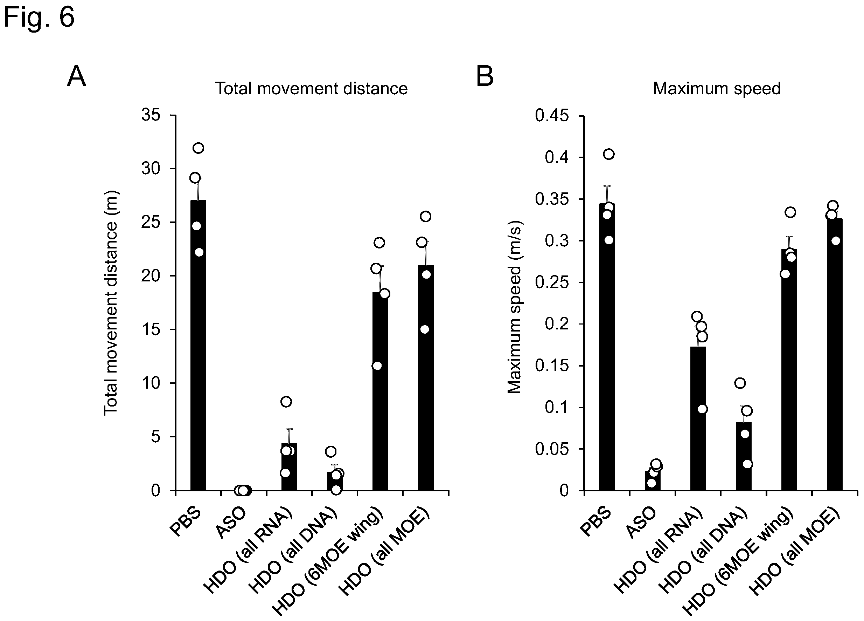

Figure 6 shows the results of evaluation of the motor function of mice at one hour after various nucleic acid agents were intraventricularly administered to the mice. The total movement distance (Figure 6A) and the maximum moving speed (Figure 6B) were improved noticeably with the groups treated with HDO (6MOE wing) and HDO (all MOE), compared with the groups treated with ASO, HDO (all RNA), and HDO (all DNA). This result has revealed that the HDOs comprising a 2'-O-MOE-RNA nucleoside have a very small effect for suppressing the motor function, and have very low toxicity.

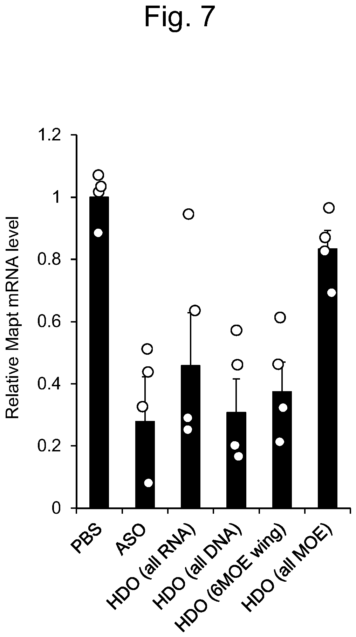

Figure 7 shows the Mapt mRNA expression level in the hippocampus at seven days after the intraventricular administration of various nucleic acid agents. The gene suppression effect was decreased with the group treated with HDO (all MOE), compared with the groups treated with ASO, HDO (all RNA), and HDO (all DNA). Meanwhile, the group treated with HDO (6MOE wing) exhibited the same gene suppression effect as the groups treated with ASO, HDO (all RNA), and HDO (all DNA). The above-described results have revealed that HDO (6MOE wing) can produce a high gene suppression effect without substantially inducing central nervous system toxicity.

### <Example 2: MOE modifications in HDO targeting BACE1 gene>

### (Purpose)

Through an in vivo experiment, the toxicity-reducing effect based on MOE modifications is investigated in terms of central nervous system toxicity observed in intraventricular administration of an HDO comprising: a first nucleic acid strand consisting of an ASO targeting the BACE1 gene; and a second nucleic acid strand having a base sequence complementary to the first nucleic acid strand.

### (Method)

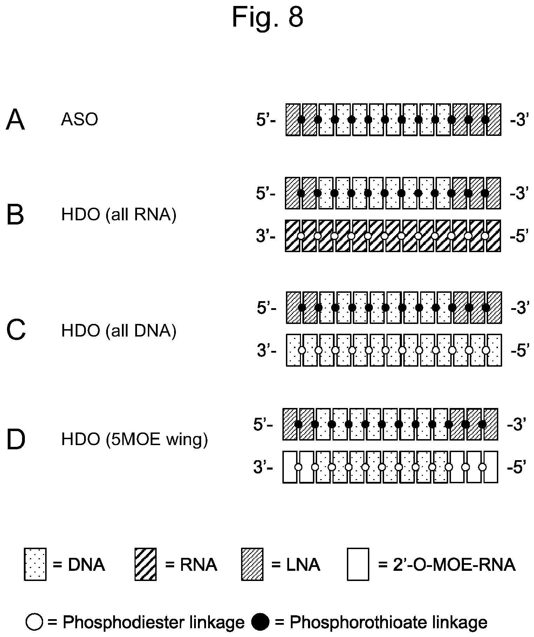

The base sequences and chemical modifications of the ASO, and the first nucleic acid strands and second nucleic acid strands constituting the HDO used in this Example are shown in Table 2 and Figure 8.

**[Table 2]**

| Table 2: ASO and HDO targeting the BACE1 gene | | | |
|---|---|---|---|
| | | Sequence (5'-3') | SEQ ID NO |
| ASO | ASO | g^t^a^t^t^g^c^t^g^a^g^g^a | 6 |
| HDO (all RNA) | ASO | g^t^a^t^t^g^c^t^g^a^g^g^a | 6 |
| | c(all RNA) | UCCUCAGCAAUAC | 7 |
| HDO (all DNA) | ASO | g^t^a^t^t^g^c^t^g^a^g^g^a | 6 |
| | c(all DNA) | tcctcagcaatac | 8 |
| HDO (5MOE wing) | ASO | g^t^a^t^t^g^c^t^g^a^g^g^a | 6 |
| | c(5MOE wing) | TC(5)C(5)tcagcaatAC(5) | 9 |

| | | | |
|---|---|---|---|
| Lower case letter: DNA; Upper case letter: RNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is an LNA/DNA gapmer antisense nucleic acid targeting a β-secretase 1 (beta-secretase 1, BACE1) mRNA of a mouse, has a base sequence complementary to part of the BACE1 mRNA, and has a structure wherein two LNA nucleosides at the 5' end, three LNA nucleosides at the 3' end, and eight DNA nucleosides therebetween are linked via a phosphorothioate linkage. All of HDO (all RNA), HDO (all DNA), and HDO (5MOE wing) used in this Example comprise the ASO as the first nucleic acid strand, and the second nucleic acid strand has a sequence complementary to the first nucleic acid strand. Specifically, the second nucleic acid strand (c(all RNA)) of HDO (all RNA) has a structure wherein the RNA nucleosides are linked via a phosphodiester linkage. The second nucleic acid strand (c(all DNA)) of HDO (all DNA) has a structure wherein the DNA nucleosides are linked via a phosphodiester linkage. The second nucleic acid strand (c(5MOE wing)) of HDO (5MOE wing) has a structure wherein three 2'-O-MOE-RNA nucleosides at the 5' end, two 2'-O-MOE-RNA nucleosides at the 3' end, and eight DNA nucleosides therebetween are linked via a phosphodiester linkage.

For ASO, HDO (all RNA), HDO (all DNA), and HDO (5MOE wing) described in Table 2, preparation of nucleic acids, in vivo experiments, evaluation of central nervous system toxicity, and evaluation of motor function were performed by the same methods as in Example 1. In this Example, however, the amount of the nucleic acid agent administered per mouse was 11.5 nmol/mouse.

### (Results)

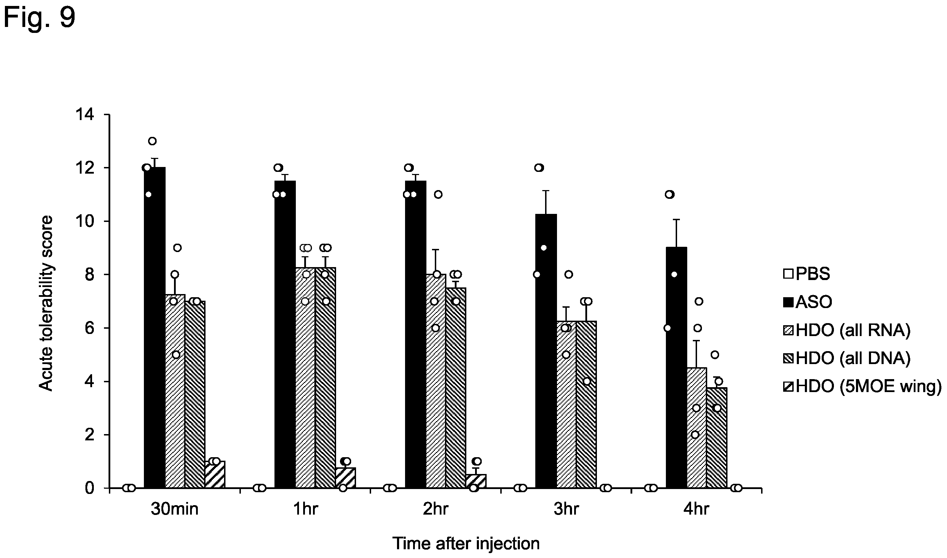

Figure 9 shows the results of evaluation of the central nervous system toxicity in mice to which various nucleic acid agents were intraventricularly administered. At any point of time of 30 minutes, 1 hour, 2 hours, 3 hours, and 4 hours after the administration of the nucleic acid agent, the acute tolerability score was decreased noticeably with the group treated with HDO (5MOE wing), compared with the groups treated with ASO, HDO (all RNA), and HDO (all DNA). This result has revealed that the HDOs comprising a 2'-O-MOE-RNA nucleoside achieves a noticeable decrease in central nervous system toxicity.

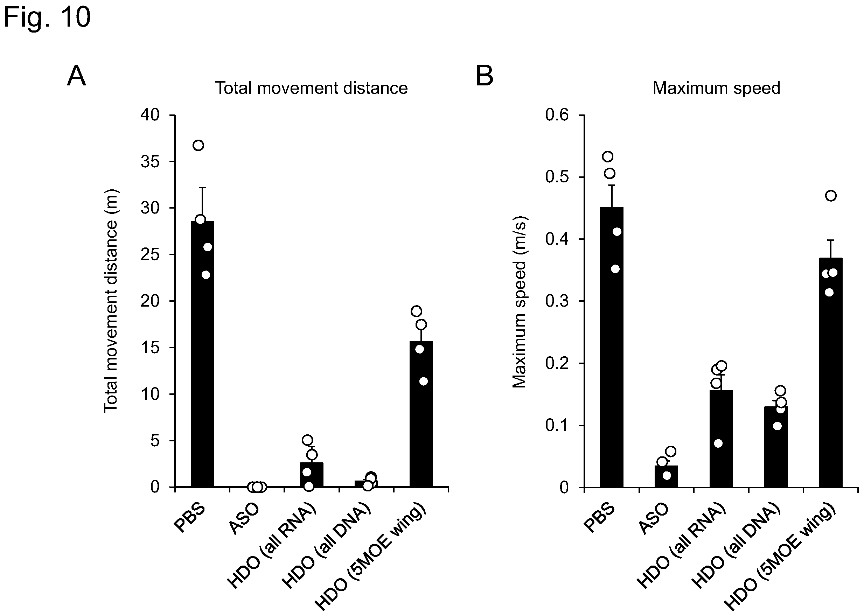

Figure 10 shows the results of evaluation of the motor function of mice at one hour after various nucleic acid agents were intraventricularly administered to the mice. The total movement distance (Figure 10A) and the maximum moving speed (Figure 10B) were improved noticeably with the group treated with HDO (5MOE wing), compared with the groups treated with ASO, HDO (all RNA), and HDO (all DNA). This result has revealed that the HDOs comprising a 2'-O-MOE-RNA nucleoside have an extremely small effect for suppressing the motor function, and have extremely low toxicity.

### <Example 3: MOE modifications in HDO targeting Malat1 gene>

### (Purpose)

Through an in vivo experiment, the toxicity-reducing effect based on MOE modifications is investigated in terms of central nervous system toxicity observed in intraventricular administration of an HDO comprising: a first nucleic acid strand consisting of an ASO targeting the Malat1 gene; and a second nucleic acid strand having a base sequence complementary to the first nucleic acid strand.

### (Method)

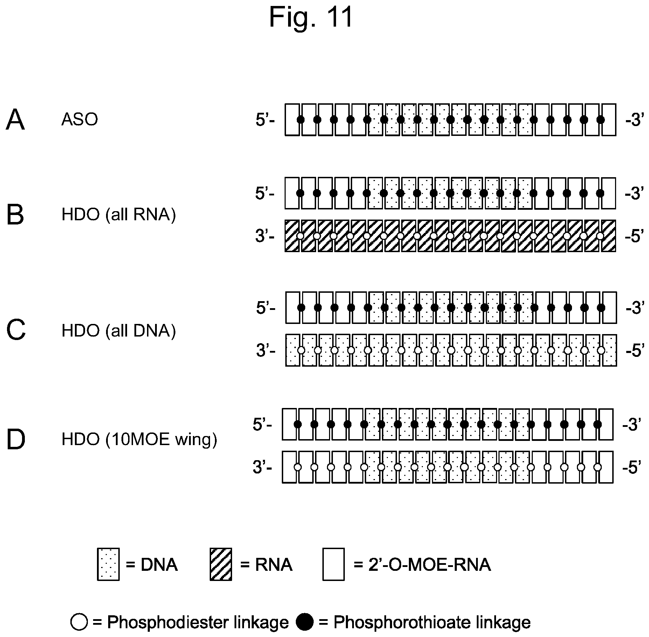

The base sequences and chemical modifications of the ASO, and first nucleic acid strands and second nucleic acid strands constituting the HDO used in this Example are shown in Table 3 and Figure 11.

**[Table 3]**

| Table 3: ASO and HDO targeting the Malat1 gene | | | |
|---|---|---|---|
| | | Sequence (5'-3') | SEQ ID NO |
| ASO | ASO | G^G^G^T^C(5)^a^g^c^t^g^c^c^a^a^t^G^C(5)^T^A^G | 10 |
| HDO (all RNA) | ASO | G^G^G^T^C(5)^a^g^c^t^g^c^c^a^a^t^G^C(5)^T^A^G | 10 |
| | c(all RNA) | CUAGCAWGGCAGCUGACCC | 11 |
| HDO (all DNA) | ASO | G^G^G^T^C(5)^a^g^c^t^g^c^c^a^a^t^G^C(5)^T^A^G | 10 |
| | c(all DNA) | ctagcattggcagctgaccc | 12 |
| HDO (10MOE wing) | ASO | G^G^G^T^C(5)^a^g^c^t^g^c^c^a^a^t^G^C(5)^T^A^G | 10 |
| | c(10MOE wing) | C(5)TAGC(5)attggcagctGAC(5)C(5)C(5) | 13 |

| | | | |
|---|---|---|---|
| Lower case letter: DNA; Upper case letter: RNA; Underlined lower case letter: LNA(c(5) represents 5-methylcytosine LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is a 2'-O-MOE-RNA/DNA gapmer antisense nucleic acid targeting a metastasis associated lung adenocarcinoma transcript 1 (Malat1) non-coding RNA of a mouse, has a base sequence complementary to part of the Malat1 ncRNA, and has a structure wherein five 2'-O-MOE-RNA nucleosides at the 5' end, five 2'-O-MOE-RNA nucleosides at the 3' end, and ten DNA nucleosides therebetween are linked via a phosphorothioate linkage. All of HDO (all RNA), HDO (all DNA), and HDO (10MOE wing) used in this Example comprise the ASO as the first nucleic acid strand, and the second nucleic acid strand has a sequence complementary to the first nucleic acid strand. Specifically, the second nucleic acid strand (c(all RNA)) of HDO (all RNA) has a structure wherein the RNA nucleosides are linked via a phosphodiester linkage. The second nucleic acid strand (c(all DNA)) of HDO (all DNA) has a structure wherein the DNA nucleosides are linked via a phosphodiester linkage. The second nucleic acid strand (c(10MOE wing)) of HDO (10MOE wing) has a structure wherein five 2'-O-MOE-RNA nucleosides at the 5' end, five 2'-O-MOE-RNA nucleosides at the 3' end, and ten DNA nucleosides therebetween are linked via a phosphodiester linkage.

For ASO, HDO (all RNA), HDO (all DNA), and HDO (10MOE wing) described in Table 3, preparation of nucleic acids, in vivo experiments, evaluation of central nervous system toxicity, and evaluation of motor function were performed by the same methods as in Example 1. In this Example, however, the amount of the nucleic acid agent administered per mouse was 13.86 nmol/mouse.

### (Results)

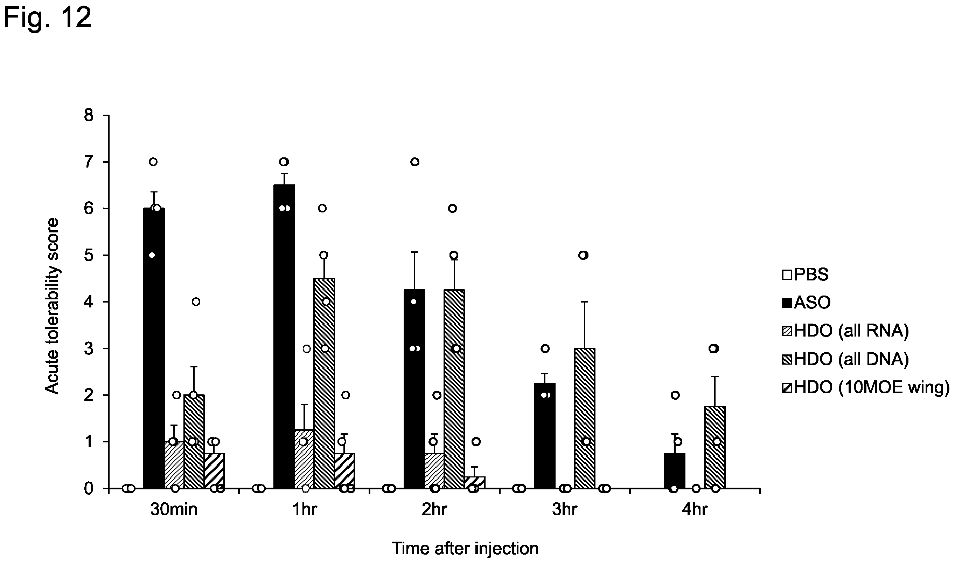

Figure 12 shows the results of evaluation of the central nervous system toxicity in mice to which various nucleic acid agents were intraventricularly administered. At any point of time of 30 minutes, 1 hour, 2 hours, 3 hours, and 4 hours after the administration of the nucleic acid agent, the acute tolerability score was decreased noticeably with the group treated with HDO (5MOE wing), compared with the groups treated with ASO and HDO (all DNA). In addition, at a point of time of 30 minutes, 1 hour, and 2 hours after the administration of the nucleic acid agent, the acute tolerability score was lower with the group treated with HDO (5MOE wing) than with the group treated with HDO (all RNA). This result has revealed that the HDOs comprising a 2'-O-MOE-RNA nucleoside achieve a noticeable decrease in central nervous system toxicity.

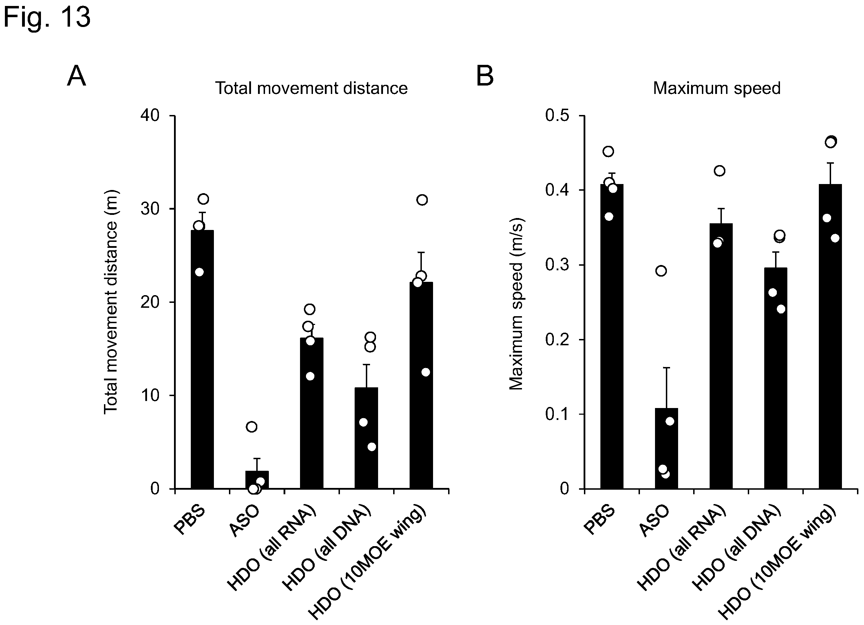

Figure 13 shows the results of evaluation of the motor function of mice at one hour after various nucleic acid agents were intraventricularly administered to the mice. The total movement distance (Figure 13A) and the maximum moving speed (Figure 13B) were improved noticeably with the group treated with HDO (10MOE wing), compared with the groups treated with ASO, HDO (all RNA), and HDO (all DNA). This result has revealed that the HDOs comprising a 2'-O-MOE-RNA nucleoside have an extremely small effect for suppressing the motor function, and have extremely low toxicity.

### <Example 4: Comparison of MOE modifications, 2'OMe modifications, and 2'F modifications>

### (Purpose)

Through an in vivo experiment, the toxicity-reducing effects based on MOE modification, 2'OMe modification, and 2'F modification are compared in terms of central nervous system toxicity observed in intraventricular administration of an HDO comprising: a first nucleic acid strand consisting of an ASO targeting the Mapt gene; and a second nucleic acid strand having a base sequence complementary to the first nucleic acid strand.

### (Method)

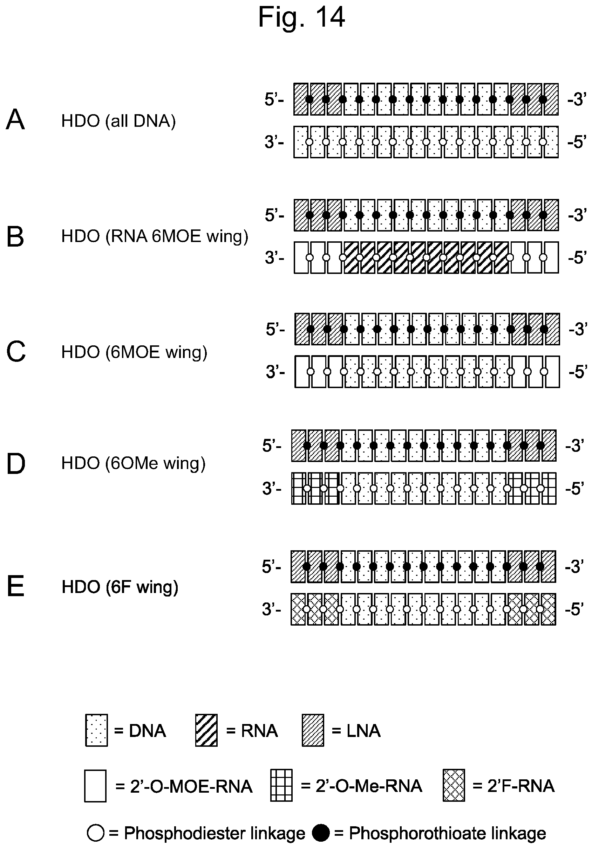

The base sequences and chemical modifications of the ASO, and first nucleic acid strands and second nucleic acid strands constituting the HDO used in this Example are shown in Table 4 and Figure 14.

**[Table 4]**

| Table 4: HDO targeting the Mapt gene | | | |
|---|---|---|---|
| | | Sequence (5'-3') | SEQ ID NO |
| HDO (all DNA) | ASO | a^t^a^g^t^c^a^c^t^c^t^g^g^t^g^a | 1 |
| | c(all DNA) | tcaccagagtgactat | 3 |
| HDO (RNA 6MOE wing) | ASO | a^t^a^g^t^c^a^c^t^c^t^g^g^t^g^a | 1 |
| | c(RNA 6MOE wing) | TC(5)ACCAGAGUGACTAT | 14 |
| HDO (6MOE wing) | ASO | a^t^a^g^t^c^a^c^t^c^t^g^g^t^g^a | 1 |
| | c(6MOE wing) | TC(5)AccagagtgacTAT | 4 |
| HDO (6OMe wing) | ASO | a^t^a^g^t^c^a^c^t^c^t^g^g^t^g^a | 1 |
| | c(6OMe wing) | U(M)C(M)A(M)ccagagtgacU(M)A(M)U(M) | 15 |
| HDO (6F wing) | ASO | a^t^a^g^t^c^a^c^t^c^t^g^g^t^g^a | 1 |
| | c(6Fwing) | U (F) C (F) A (F) ccagagtgacU (F) A (F) U (F) | 16 |

| | | | |
|---|---|---|---|
| Lower case letter: DNA; Upper case letter: RNA; Underlined lower case letter: LNA; Underlined upper case letter: 2'-O-MOE-RNA(C(5) represents 5-methylcytosine 2'-O-MOE-RNA; Upper case letter (M):2'-O-Me-RNA; Upper case letter (F):2'F-RNA; ^: Phosphorothioate linkage (PS linkage) | | | |

The ASO used in this Example is an LNA/DNA gapmer antisense nucleic acid targeting a Mapt mRNA, has a base sequence complementary to part of the Mapt mRNA, and has a structure wherein three LNA nucleosides at the 5' end, three LNA nucleosides at the 3' end, and ten DNA nucleosides therebetween are linked via a phosphorothioate linkage. All of HDO (all DNA), HDO (RNA 6MOE wing), HDO (6MOE wing), HDO (6OMe wing), and HDO (6F wing) used in this Example comprise the ASO as the first nucleic acid strand, and the second nucleic acid strand has a sequence complementary to the first nucleic acid strand. Specifically, the second nucleic acid strand (c(all DNA)) of HDO (all DNA) has a structure wherein the DNA nucleosides are linked via a phosphodiester linkage. The second nucleic acid strand (c(RNA 6MOE wing)) of HDO (RNA 6MOE wing) has a structure wherein three 2'-O-MOE-RNA nucleosides at the 5' end, three 2'-O-MOE-RNA nucleosides at the 3' end, and ten RNA nucleosides therebetween are linked via a phosphodiester linkage. The second nucleic acid strand (c(6MOE wing)) of HDO (6MOE wing) has a structure wherein three 2'-O-MOE-RNA nucleosides at the 5' end, three 2'-O-MOE-RNA nucleosides at the 3' end, and ten DNA nucleosides therebetween are linked via a phosphodiester linkage. The second nucleic acid strand (c(6OMe wing)) of HDO (6OMe wing) has a structure wherein three 2'-O-Me-RNA nucleosides at the 5' end, three 2'-O-Me-RNA nucleosides at the 3' end, and ten DNA nucleosides therebetween are linked via a phosphodiester linkage. The second nucleic acid strand (c(6F wing)) of HDO (6F wing) has a structure wherein three 2'F-RNA nucleosides at the 5' end, three 2'F-RNA nucleosides at the 3' end, and ten DNA nucleosides therebetween are linked via a phosphodiester linkage.

For HDO (RNA 6MOE wing), HDO (6MOE wing), HDO (6OMe wing), and HDO (6F wing) described in Table 4, preparation of nucleic acids, in vivo experiments, evaluation of central nervous system toxicity, and evaluation of motor function were performed by the same methods as in Example 1. In this Example, however, the amount of the nucleic acid agent administered per mouse was 19 nmol/mouse.

### (Results)

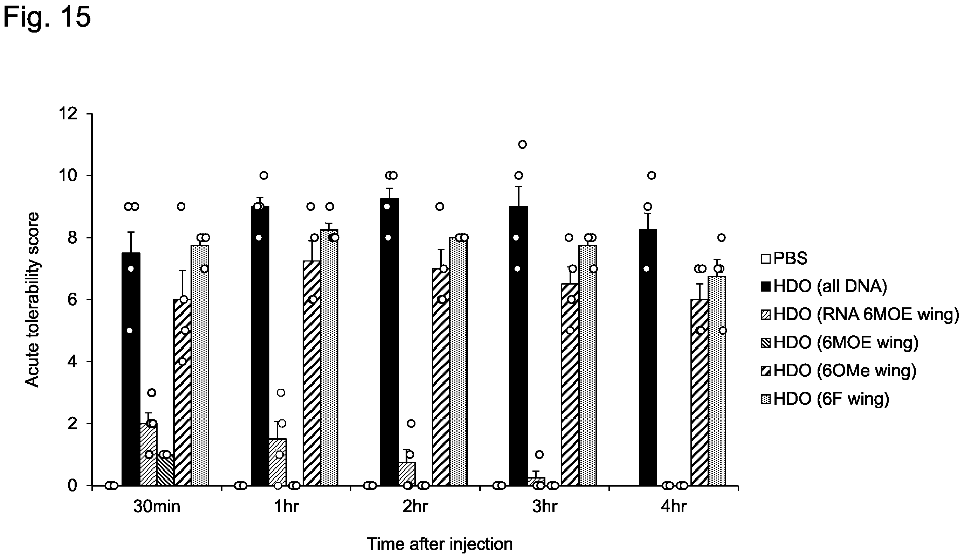

Figure 15 shows the results of evaluation of the central nervous system toxicity in mice to which various nucleic acid agents were intraventricularly administered. At any point of time of 30 minutes, 1 hour, 2 hours, 3 hours, and 4 hours after the administration of the nucleic acid agent, the acute tolerability score was decreased noticeably with the groups treated with HDO (RNA 6MOE wing) and HDO (6MOE wing), compared with the groups treated with HDO (all DNA), HDO (6OMe wing), and HDO (6F wing). Furthermore, the group treated with HDO (6MOE wing) exhibited a lower acute tolerability score than the group treated with HDO (RNA 6MOE wing). This result has revealed that the HDOs comprising a 2'-O-MOE-RNA nucleoside can achieve lower central nervous system toxicity than the HDO comprising a 2'-O-Me-RNA or 2'F-RNA nucleoside nucleoside.

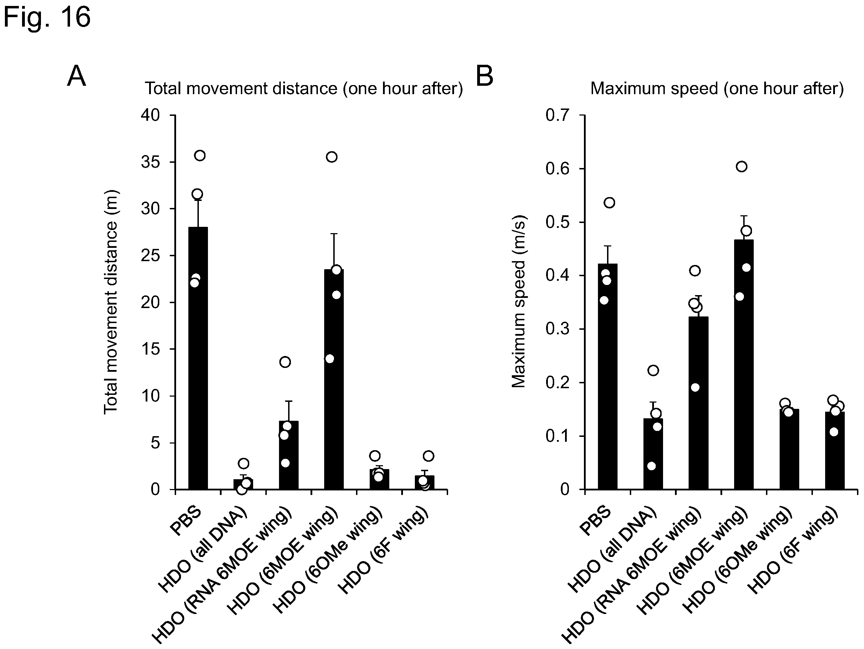

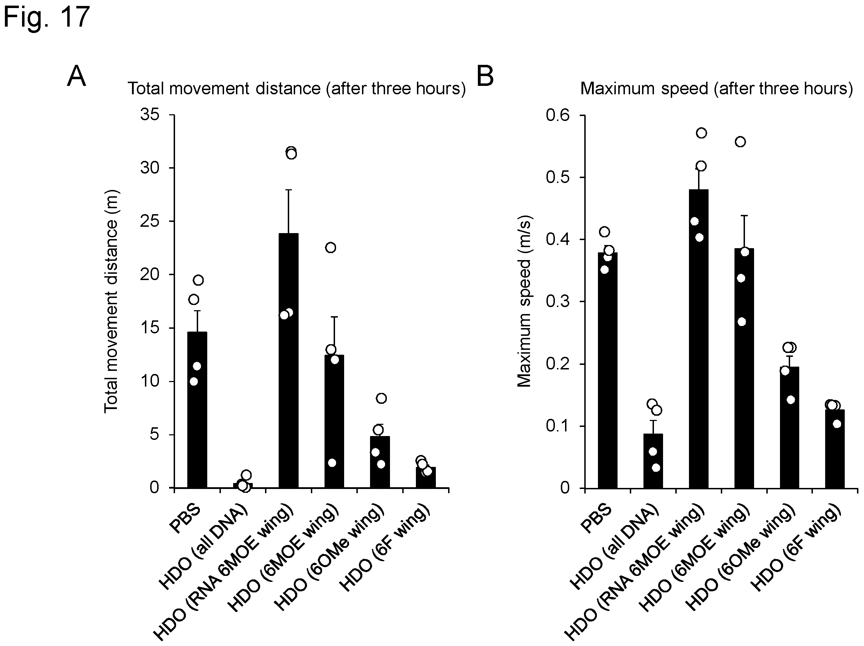

Figures 16 and 17 show the results of evaluation of the motor function of mice at 1 hour and 3 hours after various nucleic acid agents were intraventricularly administered to the mice. The total movement distance (Figure 16A and Figure 17A) and the maximum moving speed (Figure 16B and Figure 17B) were improved noticeably with the groups treated with HDO (RNA 6MOE wing) and HDO (6MOE wing), compared with the groups treated with HDO (all DNA), HDO (6OMe wing), and HDO (6F wing). This result has revealed that the HDOs comprising a 2'-O-MOE-RNA nucleoside have an extremely smaller effect for suppressing the motor function and extremely lower toxicity than the HDOs comprising a 2'-O-Me-RNA or 2'F-RNA nucleoside nucleoside.

### <Example 5: Substitutions of guanosine nucleosides>

### (Purpose)

Through an in vivo experiment, the toxicity-reducing effect based on the substitution of guanosine nucleoside in the second nucleic acid strand is examined in terms of central nervous system toxicity observed in intraventricular administration of an HDO comprising: a first nucleic acid strand consisting of an ASO targeting the Mapt gene; and a second nucleic acid strand having a base sequence complementary to the first nucleic acid strand.

### (Method)

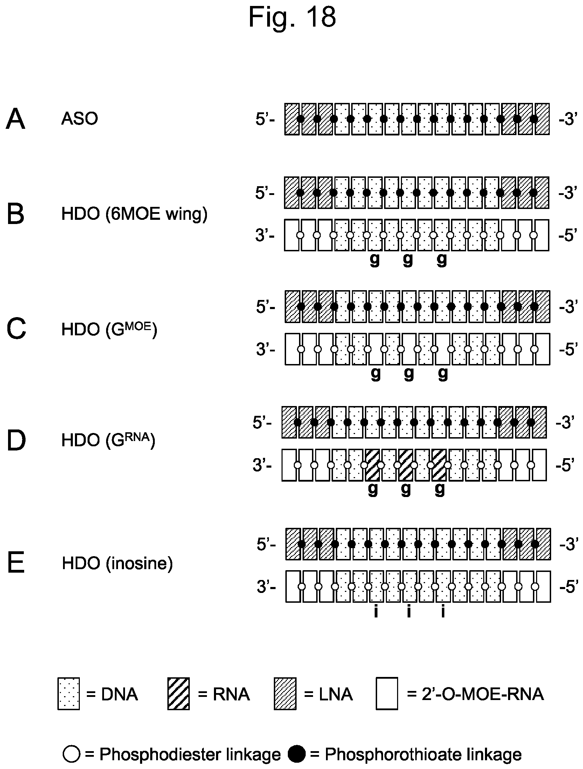

The base sequences and chemical modifications of the ASOs, and the first nucleic acid strands and second nucleic acid strands constituting the HDO used in this Example are shown in Table 5 and Figure 18.

**[Table 5]**

| Table 5: HDO targeting the Mapt gene | | | |
|---|---|---|---|
| | | Sequence (5'-3') | SEQ ID NO |
| ASO | ASO | a^t^a^g^t^c^a^c^t^c^t^g^g^t^g^a | 1 |
| HDO (6MOE wing) | ASO | a^t^a^g^t^c^a^c^t^c^t^g^g^t^g^a | 1 |
| | c(6MOE wing) | TC(5)AccagagtgacTAT | 4 |
| HDO (GMOE) | ASO | a^t^a^g^t^c^a^c^t^c^t^g^g^t^g^a | 1 |
| | c(G^{MOE}) | TC(5)AccaGaGtGacTAT | 17 |
| HDO (G^{RNA}) | ASO | a^t^a^g^t^c^a^c^t^c^t^g^g^t^g^a | 1 |
| | c(G^{RNA}) | TC(5)AccaGaGtGacTAT | 18 |
| HDO (inosine) | ASO | a^t^a^g^t^c^a^c^t^c^t^g^g^t^g^a | 1 |
| | c(inosine) | TC(5)AccaiaitiacTAT | 19 |

| | | | |
|---|---|---|---|
| Lower case letter: DNA (i represents inosine DNA); Upper case letter: RNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is an LNA/DNA gapmer antisense nucleic acid targeting a Mapt mRNA, has a base sequence complementary to part of the Mapt mRNA, and has a structure in which three LNA nucleosides at the 5' end, three LNA nucleosides at the 3' end, and ten DNA nucleosides therebetween are linked via a phosphorothioate linkage. All of HDO (6MOE wing), HDO (G^{MOE}), HDO (G^{RNA}), and HDO (inosine) used in this Example comprise the ASO as the first nucleic acid strand, and the second nucleic acid strand has a sequence complementary to the first nucleic acid strand. Specifically, the second nucleic acid strand (c(6MOE wing)) of HDO (6MOE wing) has a structure wherein three 2'-O-MOE-RNA nucleosides at the 5' end, three 2'-O-MOE-RNA nucleosides at the 3' end, and ten DNA nucleosides therebetween are linked via a phosphodiester linkage. In the second nucleic acid strand (c (G^{MOE})) of HDO (G^{MOE}), the DNA nucleoside comprising a guanine base in c(6MOE wing) is substituted with a 2'-O-MOE-RNA nucleoside. In the second nucleic acid strand (c (G^{RNA})) of HDO (G^{RNA}), the DNA nucleoside comprising a guanine base in c(6MOE wing) is substituted with an RNA nucleoside. In the second nucleic acid strand (c (inosine)) of HDO (inosine), a guanine base comprised in the DNA nucleoside in c(6MOE wing) is substituted with an inosine base.

For ASO, HDO (6MOE wing), HDO (G^{MOE}), HDO (G^{RNA}), and HDO (inosine) described in Table 5, preparation of nucleic acids, in vivo experiments, evaluation of central nervous system toxicity, and evaluation of motor function were performed by the same methods as in Example 1. In this Example, however, the amount of the nucleic acid agent administered per mouse was 18.86 nmol/mouse.

### (Results)

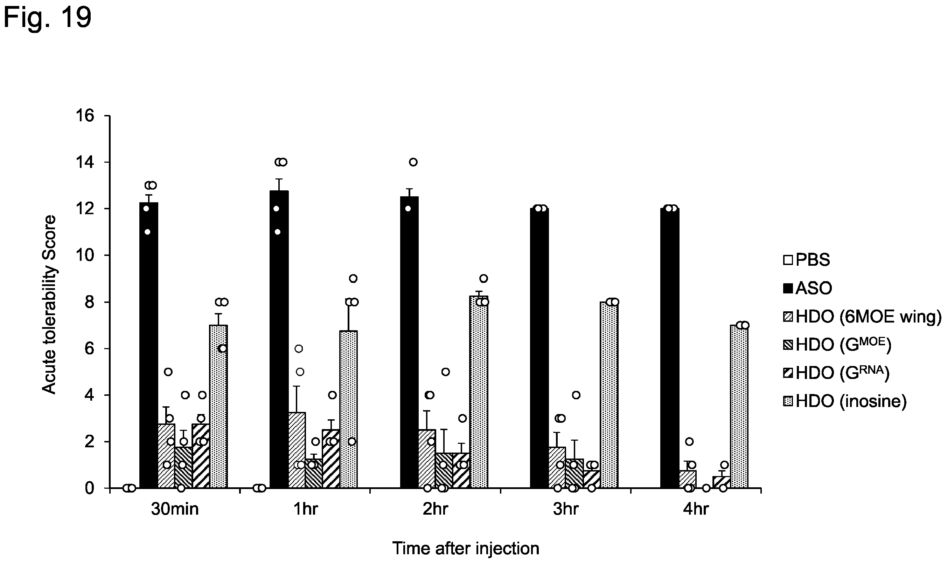

Figure 19 shows the results of evaluation of the central nervous system toxicity in mice to which various nucleic acid agents were intraventricularly administered. At any point of time of 30 minutes, 1 hour, 2 hours, 3 hours, and 4 hours after the administration of the nucleic acid agent, the groups treated with HDO (G^{MOE}) and HDO (G^{RNA}) exhibited an acute tolerability score the same as or lower than the group treated with HDO (6MOE wing).

### <Example 6: Various MOE modifications in HDOs targeting Mapt gene>

### (Purpose)

Through an in vivo experiment, the toxicity-reducing effect based on various MOE modifications is investigated in terms of central nervous system toxicity observed in intraventricular administration of an HDO comprising: a first nucleic acid strand consisting of an ASO targeting the Mapt gene; and a second nucleic acid strand having a base sequence complementary to the first nucleic acid strand.

### (Method)

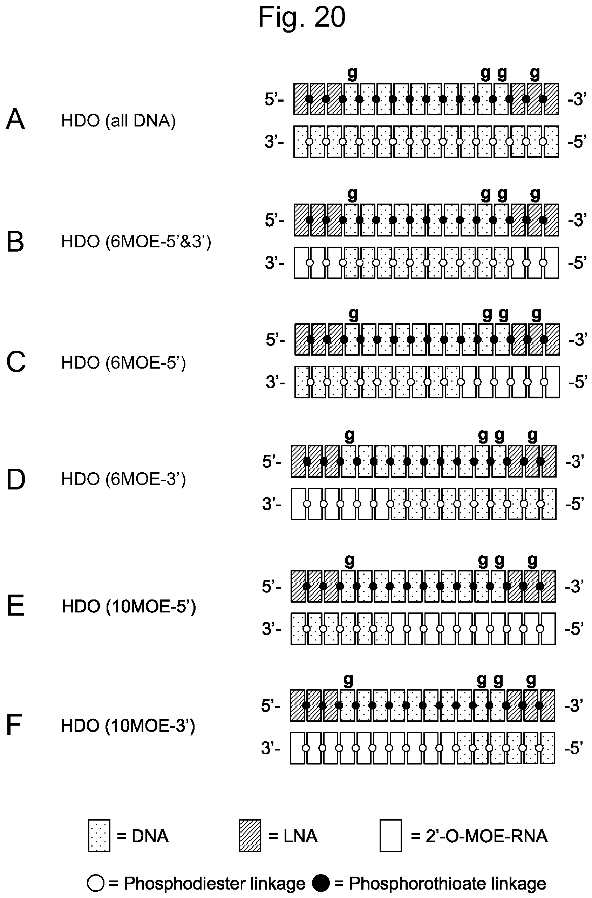

The base sequences and chemical modifications of the first nucleic acid strands and second nucleic acid strands constituting the HDO used in this Example are shown in Table 6 and Figure 20.

**[Table 6]**

| Table 6: HDO targeting the Mapt gene | | | |
|---|---|---|---|
| | | Sequence (5'-3') | SEQ ID NO |
| HDO (all DNA) | ASO | a^t^a^g^t^c^a^c^t^c^t^g^g^t^g^a | 1 |
| | c(all DNA) | tcaccagagtgactat | 3 |
| HDO (6MOE-5'&3') | ASO | a^t^a^g^t^c^a^c^t^c^t^g^g^t^g^a | 1 |
| | c(6MOE-5'&3') | TC(5)AccagagtgacTAT | 4 |
| HDO (6MOE-5') | ASO | a^t^a^g^t^c^a^c^t^c^t^g^g^t^g^a | 1 |
| | c(6MOE-5') | TC(5)AC(5)C(5)Agagtgactat | 20 |
| HDO (6MOE-3') | ASO | a^t^a^g^t^c^a^c^t^c^t^g^g^t^g^a | 1 |
| | c(6MOE-3') | tcaccagagtGAC(5)TAT | 21 |
| HDO (10MOE-5') | ASO | a^t^a^g^t^c^a^c^t^c^t^g^g^t^g^a | 1 |
| | c(10MOE-5') | TC(5)AC(5)C(5)AGAGTgactat | 22 |
| HDO (10MOE-3') | ASO | a^t^a^g^t^c^a^c^t^c^t^g^g^t^g^a | 1 |
| | c(10MOE-3') | tcaccaGAGTGAC(5)TAT | 23 |

| | | | |
|---|---|---|---|
| Lower case letter: DNA; Upper case letter: RNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is an LNA/DNA gapmer antisense nucleic acid targeting a Mapt mRNA, has a base sequence complementary to part of the Mapt mRNA, and has a structure wherein three LNA nucleosides at the 5' end, three LNA nucleosides at the 3' end, and ten DNA nucleosides therebetween are linked via a phosphorothioate linkage. All of HDO (all DNA), HDO (6MOE-5'&3'), HDO (6MOE-5'), HDO (6MOE-3'), HDO (10MOE-5'), and HDO (10MOE-3') used in this Example comprise the ASO as the first nucleic acid strand, and the second nucleic acid strand has a sequence complementary to the first nucleic acid strand. Specifically, the second nucleic acid strand (c(all DNA)) of HDO (all DNA) has a structure wherein the DNA nucleosides are linked via a phosphodiester linkage. The second nucleic acid strand (c(6MOE-5'&3')) of HDO (6MOE-5'&3') has a structure wherein three 2'-O-MOE-RNA nucleosides at the 5' end, three 2'-O-MOE-RNA nucleosides at the 3' end, and ten DNA nucleosides therebetween are linked via a phosphodiester linkage. The second nucleic acid strand (c(6MOE-5')) of HDO (6MOE-5') has a structure wherein six 2'-O-MOE-RNA nucleosides at the 5' end and ten DNA nucleosides at the 3' end are linked via a phosphodiester linkage. The second nucleic acid strand (c(6MOE-3')) of HDO (6MOE-3') has a structure wherein ten DNA nucleosides at the 5' end and six 2'-O-MOE-RNA nucleosides at the 3' end are linked via a phosphodiester linkage. The second nucleic acid strand (c(10MOE-5')) of HDO (10MOE-5') has a structure wherein ten 2'-O-MOE-RNA nucleosides at the 5' end and six DNA nucleosides at the 3' end are linked via a phosphodiester linkage. The second nucleic acid strand (c(10MOE-3')) of HDO (10MOE-3') has a structure wherein six DNA nucleosides at the 5' end and ten 2'-O-MOE-RNA nucleosides at the 3' end are linked via a phosphodiester linkage.

For HDO (all DNA), HDO (6MOE-5'&3'), HDO (6MOE-5'), HDO (6MOE-3'), HDO (10MOE-5'), and HDO (10MOE-3') described in Table 6, preparation of nucleic acids, in vivo experiments, evaluation of central nervous system toxicity, evaluation of motor function, and evaluation of the gene suppression effect were performed by the same methods as in Example 1. In this Example, however, the amount of the nucleic acid agent administered per mouse was 18.86 nmol/mouse.

### (Results)

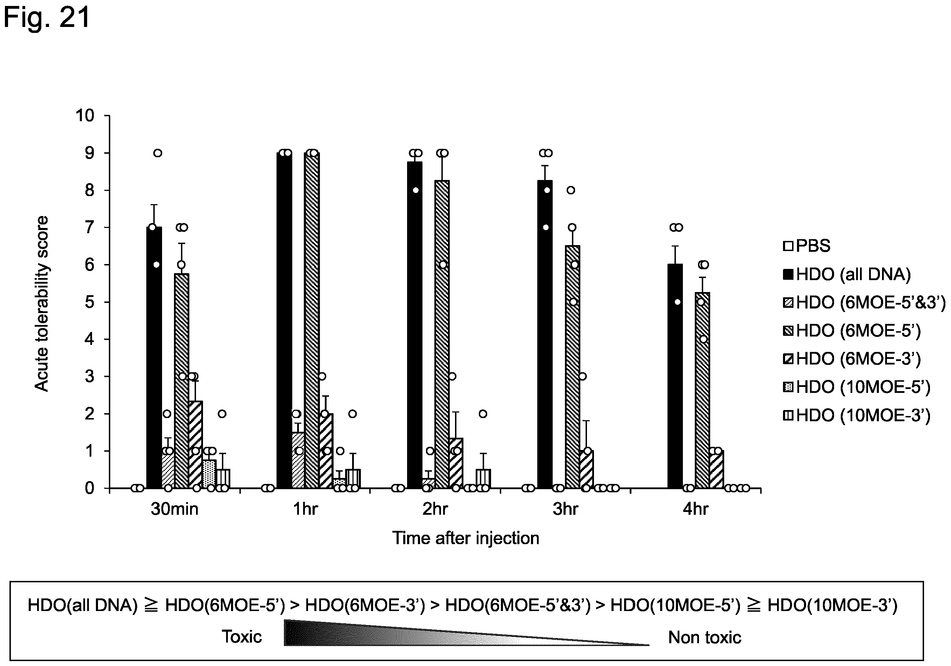

Figure 21 shows the results of evaluation of the central nervous system toxicity in mice to which various nucleic acid agents were intraventricularly administered. At any point of time of 30 minutes, 1 hour, 2 hours, 3 hours, and 4 hours after the administration of the nucleic acid agent, the acute tolerability score was decreased noticeably with the groups treated with HDO (6MOE-5'&3'), HDO (6MOE-5'), HDO (6MOE-3'), HDO (10MOE-5'), and HDO (10MOE-3'), compared with the group treated with HDO (all DNA). In particular, the acute tolerability scores for the groups treated with HDO (10MOE-5') and HDO (10MOE-3') were lowest among the groups treated with HDOs.

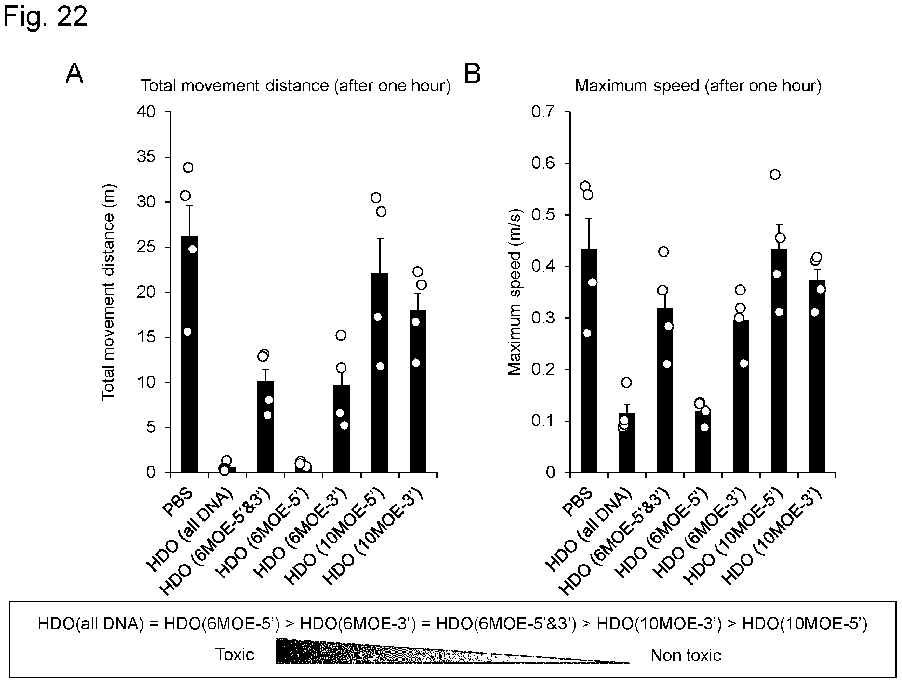

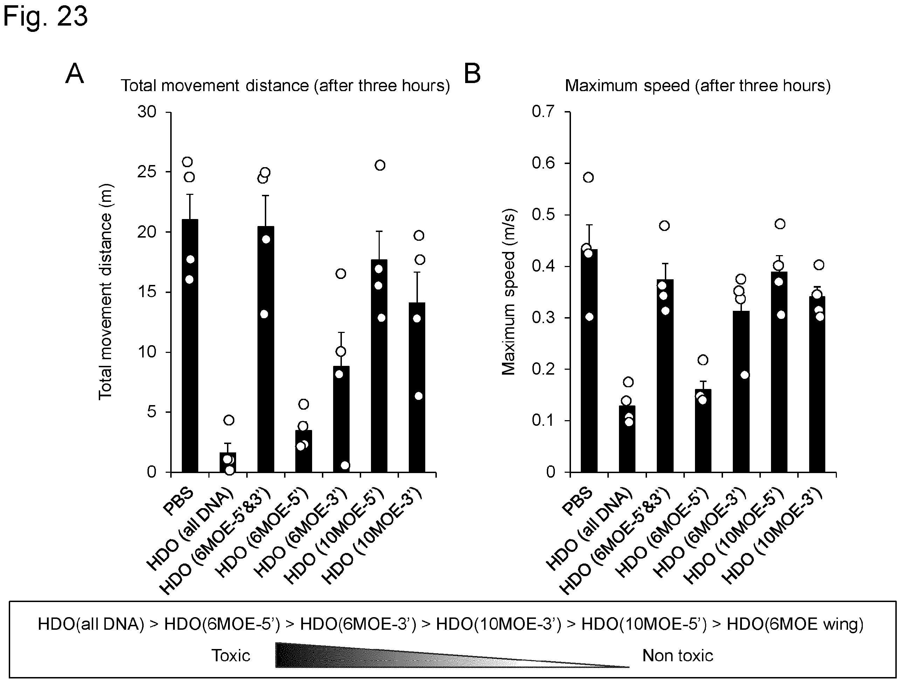

Figures 22 and 23 show the results of evaluation of the motor function of mice at one hour and three hours after various nucleic acid agents were intraventricularly administered to the mice. The total movement distance (Figure 22A and Figure 23A) and the maximum moving speed (Figure 22B and Figure 23B) were improved noticeably with the groups treated with HDO (6MOE-5'&3'), HDO (6MOE-5'), HDO (6MOE-3'), HDO (10MOE-5'), and HDO (10MOE-3'), compared with the group treated with HDO (all DNA). At one hour after the administration, the improvement effect of the groups treated with HDO (10MOE-5') and HDO (10MOE-3') was highest among the groups treated with HDOs. At three hours after the administration, the improvement effect of the group treated with HDO (6MOE-5'& 3') was highest among the groups treated with HDOs.

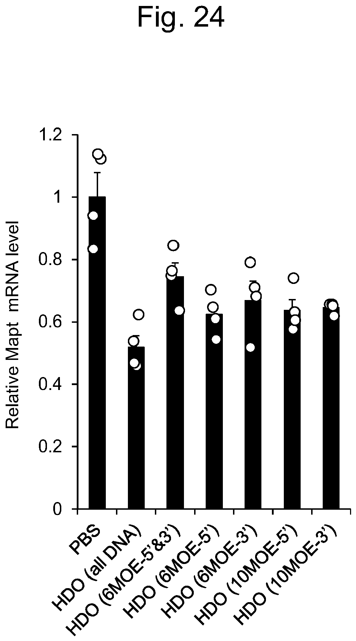

Figure 24 shows the Mapt mRNA expression level in the hippocampus at seven days after the intraventricular administration of various nucleic acid agents. The groups treated with HDO (all DNA), HDO (6MOE-5'&3'), HDO (6MOE-5'), HDO (6MOE-3'), HDO (10MOE-5'), and HDO (10MOE-3') exhibited a significant decrease in the expression level of Mapt mRNA, compared with the negative control treated with only PBS.

### <Example 7: Various MOE modifications in HDO targeting BACE1 gene>

### (Purpose)

Through an in vivo experiment, the toxicity-reducing effect based on various MOE modifications is investigated in terms of central nervous system toxicity observed in intraventricular administration of an HDO comprising: a first nucleic acid strand consisting of an ASO targeting the BACE1 gene; and a second nucleic acid strand having a base sequence complementary to the first nucleic acid strand.

### (Method)

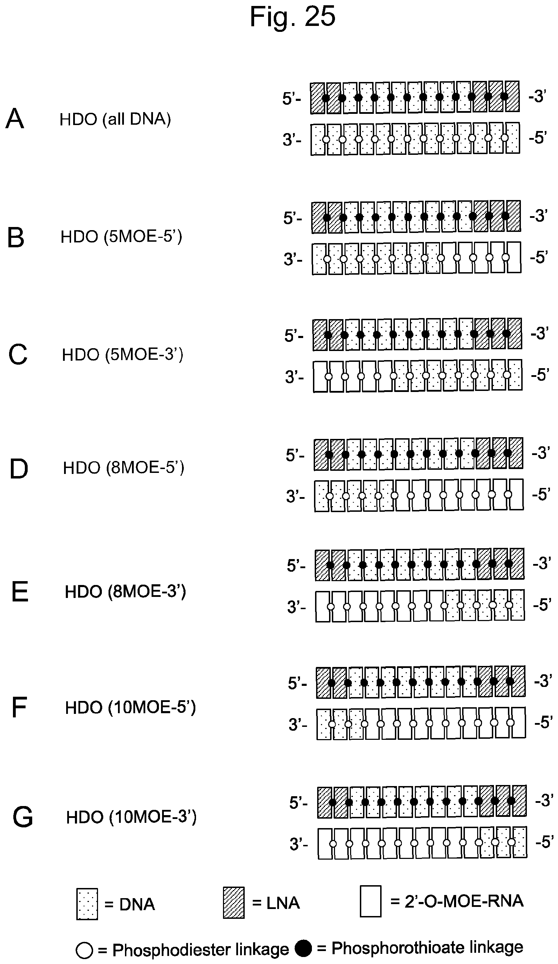

The base sequences and chemical modifications of the first nucleic acid strands and second nucleic acid strands constituting the HDOs used in this Example are shown in Table 7 and Figure 25.

**[Table 7]**

| Table 7: HDO targeting the BACE1 gene | | | |
|---|---|---|---|
| | | Sequence (5'-3') | SEQ ID NO |
| HDO (all DNA) | ASO | g^t^a^t^t^g^c^t^g^a^g^g^a | 6 |
| | c(all DNA) | tcctcagcaatac | 8 |
| HDO (5MOE-5') | ASO | g^t^a^t^t^g^c^t^g^a^g^g^a | 6 |
| | c(5MOE-5') | TC(5)C(5)TC(5)agcaatac | 24 |
| HDO (5MOE-3') | ASO | g^t^a^t^t^g^c^t^g^a^g^g^a | 6 |
| | c(5MOE-3') | tcctcagcAATAC(5) | 25 |
| HDO (8MOE-5') | ASO | g^t^a^t^t^g^c^t^g^a^g^g^a | 6 |
| | c(8MOE-5') | TC(5)C(5)TC(5)AGC(5)aatac | 26 |
| HDO (8MOE-3') | ASO | g^t^a^t^t^g^c^t^g^a^g^g^a | 6 |
| | c(8MOE-3') | tcctcAGC(5)AATAC(5) | 27 |
| HDO (10MOE-5') | ASO | g^t^a^t^t^g^c^t^g^a^g^g^a | 6 |
| | c(10MOE-5') | TC(5)C(5)TC(5)AGC(5)AAtac | 28 |
| HDO (10MOE-3') | ASO | g^t^a^t^t^g^c^t^g^a^g^g^a | 6 |
| | c(10MOE-3') | tccTC(5)AGC(5)AATAC(5) | 29 |

| | | | |
|---|---|---|---|
| Lower case letter: DNA; Upper case letter: RNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is an LNA/DNA gapmer antisense nucleic acid targeting BACE1 mRNA, has a base sequence complementary to part of BACE1 mRNA, and has a structure wherein two LNA nucleosides at the 5' end, three LNA nucleosides at the 3' end, and eight DNA nucleosides therebetween are linked via a phosphorothioate linkage. All of HDO (all DNA), HDO (5MOE-5'), HDO (5MOE-3'), HDO (8MOE-5'), HDO (8MOE-3'), HDO (10MOE-5'), and HDO (10MOE-3') used in this Example comprise the ASO as the first nucleic acid strand, and the second nucleic acid strand has a sequence complementary to the first nucleic acid strand. Specifically, the second nucleic acid strand (c(all DNA)) of HDO (all DNA) has a structure wherein the DNA nucleosides are linked via a phosphodiester linkage. The second nucleic acid strand (c(5MOE-5')) of HDO (5MOE-5') has a structure wherein five 2'-O-MOE-RNA nucleosides at the 5' end and eight DNA nucleosides at the 3' end are linked via a phosphodiester linkage. The second nucleic acid strand (c(5MOE-3')) of HDO (5MOE-3') has a structure wherein eight DNA nucleosides at the 5' end and five 2'-O-MOE-RNA nucleosides at the 3' end are linked via a phosphodiester linkage. The second nucleic acid strand (c(8MOE-5')) of HDO (8MOE-5') has a structure wherein eight 2'-O-MOE-RNA nucleosides at the 5' end and five DNA nucleosides at the 3' end are linked via a phosphodiester linkage. The second nucleic acid strand (c(8MOE-3')) of HDO (8MOE-3') has a structure wherein five DNA nucleosides at the 5' end and eight 2'-O-MOE-RNA nucleosides at the 3' end are linked via a phosphodiester linkage. The second nucleic acid strand (c(10MOE-5')) of HDO (10MOE-5') has a structure wherein ten 2'-O-MOE-RNA nucleosides at the 5' end and three DNA nucleosides at the 3' end are linked via a phosphodiester linkage. The second nucleic acid strand (c(10MOE-3')) of HDO (10MOE-3') has a structure wherein three DNA nucleosides at the 5' end and ten 2'-O-MOE-RNA nucleosides at the 3' end are linked via a phosphodiester linkage.

For HDO (all DNA), HDO (5MOE-5'), HDO (5MOE-3'), HDO (8MOE-5'), HDO (8MOE-3'), HDO (10MOE-5'), and HDO (10MOE-3') described in Table 7, preparation of nucleic acids, in vivo experiments, evaluation of central nervous system toxicity, and evaluation of motor function were performed by the same methods as in Example 1. In this Example, however, the amount of the nucleic acid agent administered per mouse was 11.5 nmol/mouse.

### (Results)

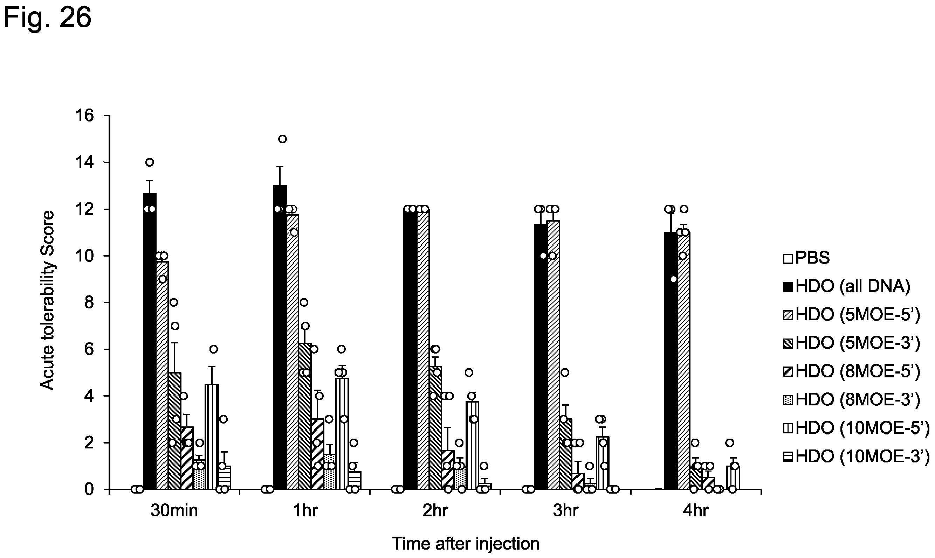

Figure 26 shows the results of evaluation of the central nervous system toxicity in mice to which various nucleic acid agents were intraventricularly administered. At any point of time of 30 minutes, 1 hour, 2 hours, 3 hours, and 4 hours after the administration of the nucleic acid agent, the acute tolerability score was decreased noticeably with the groups treated with HDO (5MOE-5'), HDO (5MOE-3'), HDO (8MOE-5'), HDO (8MOE-3'), HDO (10MOE-5'), and HDO (10MOE-3'), compared with the group treated with HDO (all DNA).

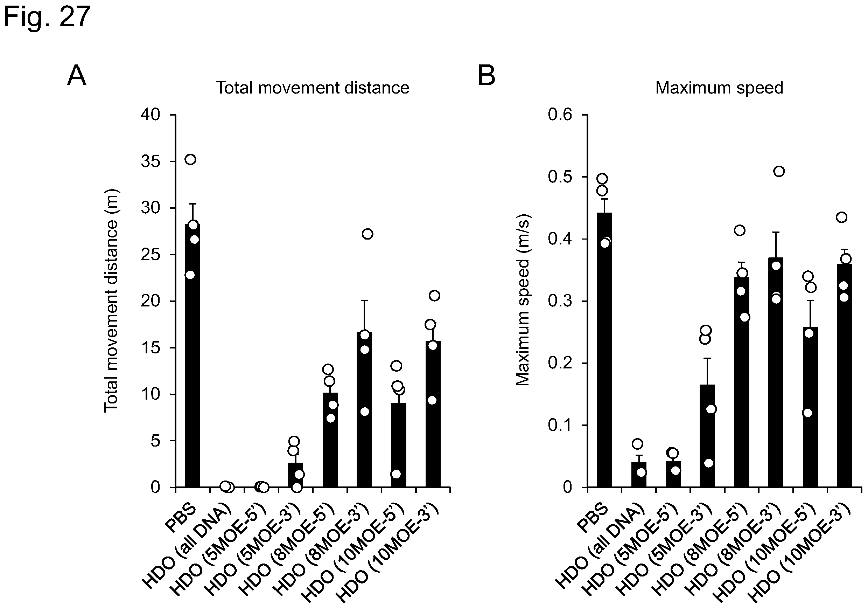

Figure 27 shows the results of evaluation of the motor function of mice at one hour after various nucleic acid agents were intraventricularly administered to the mice. The total movement distance (Figure 27A) and the maximum moving speed (Figure 27B) were improved noticeably with the groups treated with HDO (5MOE-5'), HDO (5MOE-3'), HDO (8MOE-5'), HDO (8MOE-3'), HDO (10MOE-5'), and HDO (10MOE-3'), compared with the group treated with HDO (all DNA).

### <Example 8: Various MOE modifications in HDO targeting Mapt gene>

### (Purpose)

Through an in vivo experiment, the toxicity-reducing effect based on various MOE modifications is investigated in terms of central nervous system toxicity observed in intraventricular administration of an HDO comprising: a first nucleic acid strand consisting of an ASO targeting the Mapt gene; and a second nucleic acid strand having a base sequence complementary to the first nucleic acid strand.

### (Method)

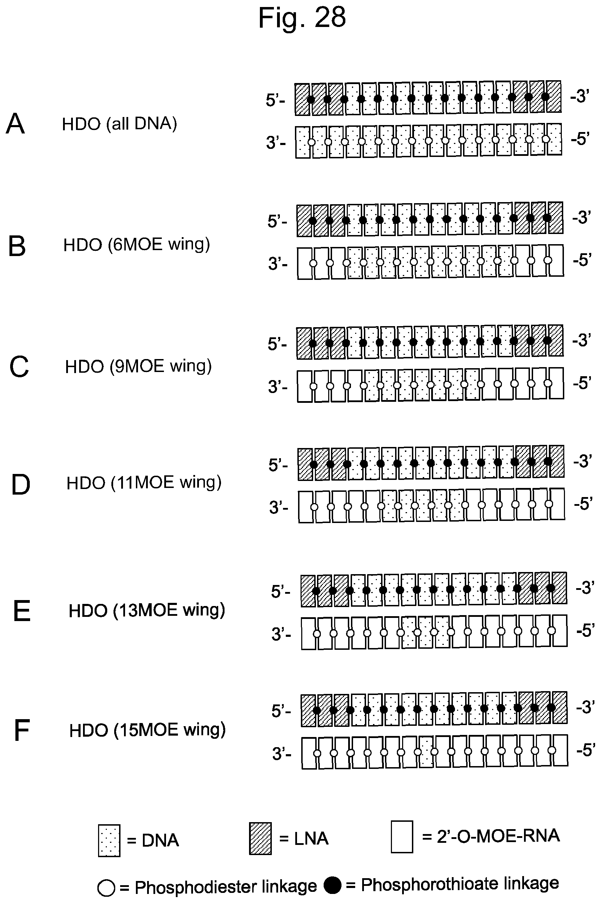

The base sequences and chemical modifications of the first nucleic acid strands and second nucleic acid strands constituting the HDOs used in this Example are shown in Table 8 and Figure 28.

**[Table 8]**

| Table 8: HDO targeting the Mapt gene | | | |
|---|---|---|---|
| | | Sequence (5'-3') | SEQ ID NO |
| HDO (all DNA) | ASO | a^t^a^g^t^c^a^c^t^c^t^g^g^t^g^a | 1 |
| | c(all DNA) | tcaccagagtgactat | 3 |
| HDO (6MOE wing) | ASO | a^t^a^g^t^c^a^c^t^c^t^g^g^t^g^a | 1 |
| | c(6MOE wing) | TC(5)AccagagtgacTAT | 4 |
| HDO (9MOE wing) | ASO | a^t^a^g^t^c^a^c^t^c^t^g^g^t^g^a | 1 |
| | c(9MOE wing) | TC(5)AC(5)C(5)agagtgaC(5)TAT | 30 |
| HDO (11MOE wing) | ASO | a^t^a^g^t^c^a^c^t^c^t^g^g^t^g^a | 1 |
| | c(11MOE wing) | TC(5)AC(5)C(5)AgagtgAC(5)TAT | 31 |
| HDO (13MOE wing) | ASO | a^t^a^g^t^c^a^c^t^c^t^g^g^t^g^a | 1 |
| | c(13MOE wing) | TC(5)AC(5)C(5)AGagtGAC(5)TAT | 32 |
| HDO (15MOE wing) | ASO | a^t^a^g^t^c^a^c^t^c^t^g^g^t^g^a | 1 |
| | c(15MOE wing) | TC(5)AC(5)C(5)AGAgTGAC(5)TAT | 33 |

| | | | |
|---|---|---|---|
| Lower case letter: DNA; Upper case letter: RNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is an LNA/DNA gapmer antisense nucleic acid targeting a Mapt mRNA, has a base sequence complementary to part of the Mapt mRNA, and has a structure wherein three LNA nucleosides at the 5' end, three LNA nucleosides at the 3' end, and ten DNA nucleosides therebetween are linked via a phosphorothioate linkage. All of HDO (all DNA), HDO (6MOE wing), HDO (9MOE wing), HDO (11MOE wing), HDO (13MOE wing), and HDO (15MOE wing) used in this Example comprise the ASO as the first nucleic acid strand, and the second nucleic acid strand has a sequence complementary to the first nucleic acid strand. Specifically, the second nucleic acid strand (c(all DNA)) of HDO (all DNA) has a structure wherein the DNA nucleosides are linked via a phosphodiester linkage. The second nucleic acid strand (c(6MOE wing)) of HDO (6MOE wing) has a structure wherein three 2'-O-MOE-RNA nucleosides at the 5' end, three 2'-O-MOE-RNA nucleosides at the 3' end, and ten DNA nucleosides therebetween are linked via a phosphodiester linkage. The second nucleic acid strand (c(9MOE wing)) of HDO (9MOE wing) has a structure wherein five 2'-O-MOE-RNA nucleosides at the 5' end, four 2'-O-MOE-RNA nucleosides at the 3' end, and seven DNA nucleosides therebetween are linked via a phosphodiester linkage. The second nucleic acid strand (c(11MOE wing)) of HDO (11MOE wing) has a structure wherein six 2'-O-MOE-RNA nucleosides at the 5' end, five 2'-O-MOE-RNA nucleosides at the 3' end, and five DNA nucleosides therebetween are linked via a phosphodiester linkage. The second nucleic acid strand (c(13MOE wing)) of HDO (13MOE wing) has a structure wherein seven 2'-O-MOE-RNA nucleosides at the 5' end, six 2'-O-MOE-RNA nucleosides at the 3' end, and three DNA nucleosides therebetween are linked via a phosphodiester linkage. The second nucleic acid strand (c(15MOE wing)) of HDO (15MOE wing) has a structure wherein eight 2'-O-MOE-RNA nucleosides at the 5' end, seven 2'-O-MOE-RNA nucleosides at the 3' end, and one DNA nucleoside therebetween are linked via a phosphodiester linkage.

For HDO (all DNA), HDO (6MOE wing), HDO (9MOE wing), HDO (11MOE wing), HDO (13MOE wing), and HDO (15MOE wing) described in Table 8, preparation of nucleic acids, in vivo experiments, evaluation of central nervous system toxicity, evaluation of motor function, and evaluation of the gene suppression effect were performed by the same methods as in Example 1. In this Example, however, the amount of the nucleic acid agent administered per mouse was 18.86 nmol/mouse.

### (Results)

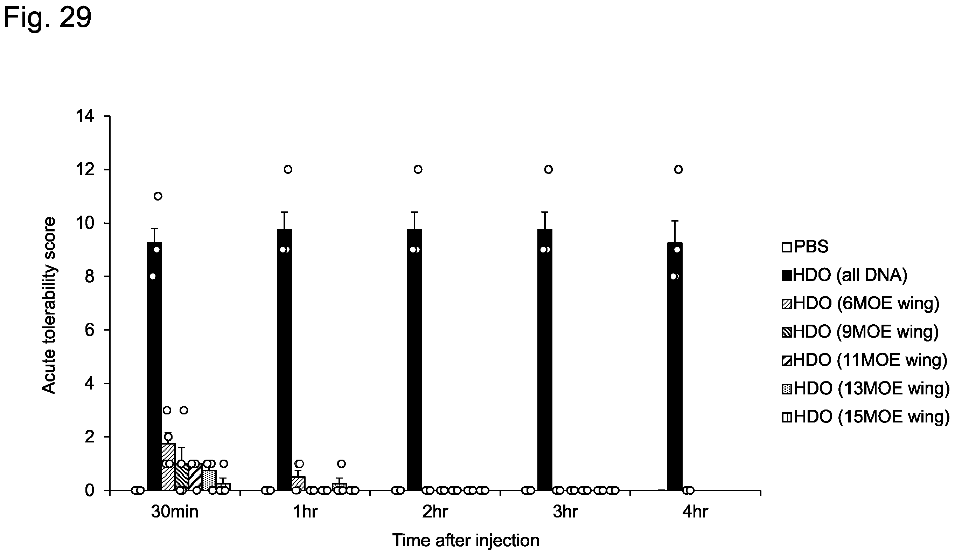

Figure 29 shows the results of evaluation of the central nervous system toxicity in mice to which various nucleic acid agents were intraventricularly administered. At any point of time of 30 minutes, 1 hour, 2 hours, 3 hours, and 4 hours after the administration of the nucleic acid agent, the acute tolerability score was decreased noticeably with the groups treated with HDO (6MOE wing), HDO (9MOE wing), HDO (11MOE wing), HDO (13MOE wing), and HDO (15MOE wing), compared with the group treated with HDO (all DNA).

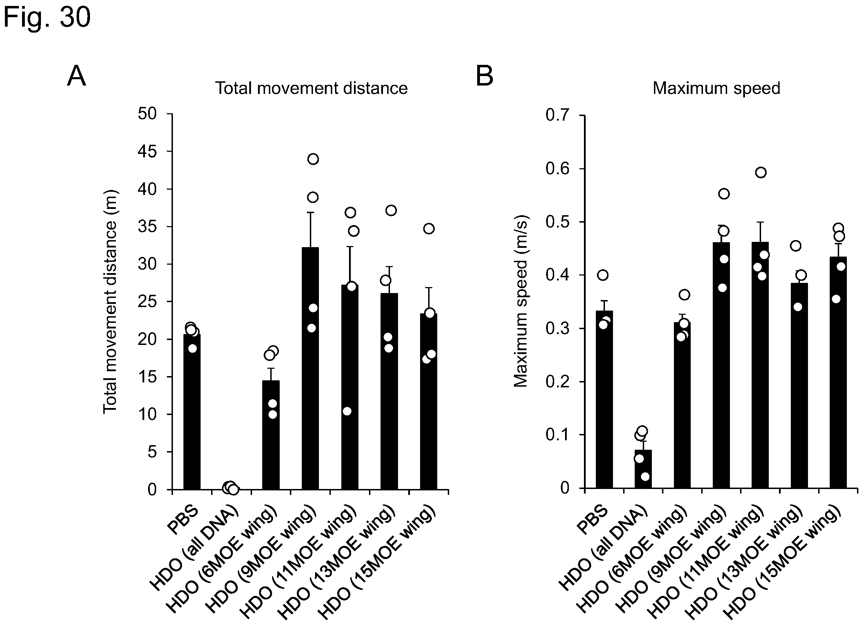

Figure 30 shows the results of evaluation of the motor function of mice at one hour after various nucleic acid agents were intraventricularly administered to the mice. The total movement distance (Figure 30A) and the maximum moving speed (Figure 30B) were improved noticeably with the groups treated with HDO (6MOE wing), HDO (9MOE wing), HDO (11MOE wing), HDO (13MOE wing), and HDO (15MOE wing), compared with the group treated with HDO (all DNA).

### <Example 9: Comparison of species of bases for MOE modifications>

### (Purpose)

Through an in vivo experiment, the toxicity-reducing effects depending on a difference in the kind of a nucleoside (nucleoside comprising an adenine base, guanine base, cytosine base, or thymine base) into which MOE modifications are introduced in the second nucleic acid strand are compared in terms of central nervous system toxicity observed in intraventricular administration of an HDO comprising: a first nucleic acid strand consisting of an ASO targeting the Mapt gene; and a second nucleic acid strand having a base sequence complementary to the first nucleic acid strand.

### (Method)

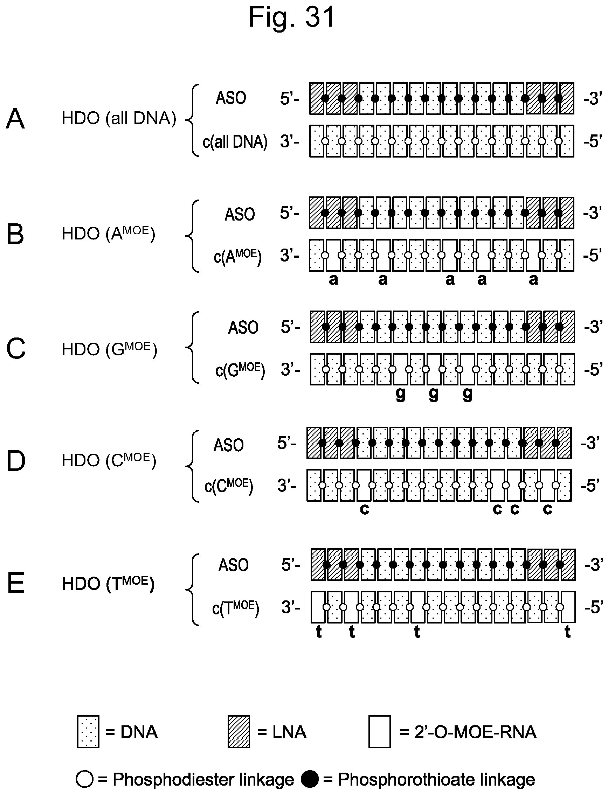

The base sequences and chemical modifications of the ASO, and first nucleic acid strands and second nucleic acid strands constituting the HDO used in this Example are shown in Table 9 and Figure 31.

**[Table 9]**

| Table 9: HDO targeting the Mapt gene | | | |
|---|---|---|---|
| | | Sequence (5'-3') | SEQ ID NO |
| HDO (all DNA) | ASO | a^t^a^g^t^c^a^c^t^c^t^g^g^t^g^a | 1 |
| | c(all DNA) | tcaccagagtgactat | 3 |
| HDO (AMOE) | ASO | a^t^a^g^t^c^a^c^t^c^t^g^g^t^g^a | 1 |
| | c(A^{MOE}) | tcAccAgAgtgActAt | 44 |
| HDO (GMOE) | ASO | a^t^a^g^t^c^a^c^t^c^t^g^g^t^g^a | 1 |
| | c(G^{MOE}) | tcaccaGaGtGactat | 45 |
| HDO (C^{MOE}) | ASO | a^t^a^g^t^c^a^c^t^c^t^g^g^t^g^a | 1 |
| | c(C^{MOE}) | tC(5)aC(5)C(5)agagtgaC(5)tat | 46 |
| HDO (T^{MOE}) | ASO | a^t^a^g^t^c^a^c^t^c^t^g^g^t^g^a | 1 |
| | c(T^{MOE}) | TcaccagagTgacTaT | 47 |

| | | | |
|---|---|---|---|
| Lower case letter: DNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is an LNA/DNA gapmer antisense nucleic acid targeting a Mapt mRNA, has a base sequence complementary to part of the Mapt mRNA, and has a structure wherein three LNA nucleosides at the 5' end, three LNA nucleosides at the 3' end, and ten DNA nucleosides therebetween are linked via a phosphorothioate linkage. All of HDO (all DNA), HDO (A^{MOE}), HDO (G^{MOE}), HDO (C^{MOE}), and HDO (T^{MOE}) used in this Example comprise the ASO as the first nucleic acid strand, and the second nucleic acid strand has a sequence complementary to the first nucleic acid strand. Specifically, the second nucleic acid strand (c(all DNA)) of HDO (all DNA) has a structure wherein the DNA nucleosides are linked via a phosphodiester linkage. The second nucleic acid strand (a(A^{MOE})) of HDO (A^{MOE}) has a structure wherein all of the DNA nucleosides comprising an adenine base are substituted with 2'-O-MOE-RNA nucleosides, and in the same manner, HDO (G^{MOE}), HDO (C^{MOE}), HDO (T^{MOE}) have a structure wherein all of the DNA nucleosides comprising a guanine base, cytosine base, and thymine base respectively are substituted with 2'-O-MOE-RNA nucleosides.

According to HDO (all DNA), HDO (A^{MOE}), HDO (G^{MOE}), HDO (C^{MOE}), and HDO (T^{MOE}) described in Table 9, preparation of nucleic acids, in vivo experiments, evaluation of central nervous system toxicity, and evaluation of motor function were performed by the same methods as in Example 1. In this Example, however, the amount of the nucleic acid agent administered per mouse was 19 nmol/mouse.

### (Results)

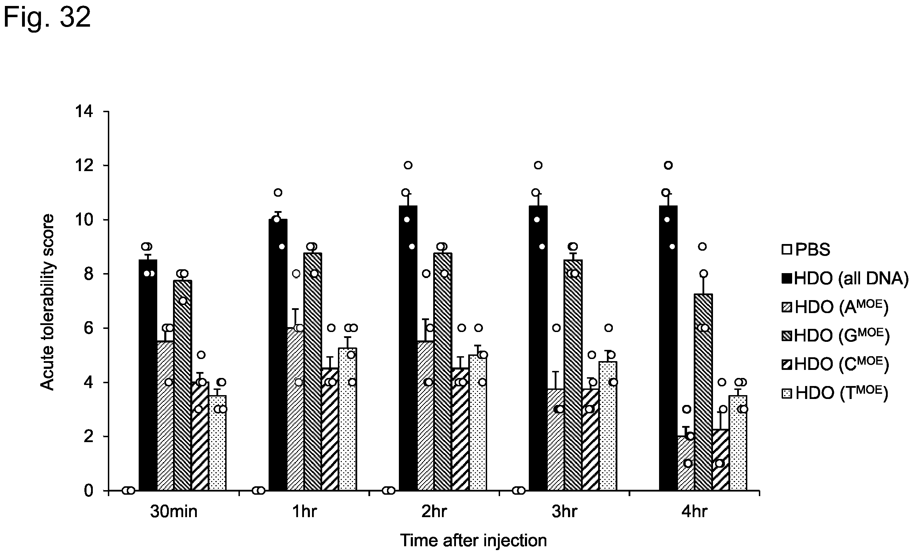

Figure 32 shows the results of evaluation of the central nervous system toxicity in mice to which various nucleic acid agents were intraventricularly administered. At any point of time of 30 minutes, 1 hour, 2 hours, 3 hours, and 4 hours after the administration of the nucleic acid agent, the acute tolerability score was decreased noticeably with the groups treated with HDO (A^{MOE}), HDO (C^{MOE}), and HDO (T^{MOE}), compared with the group treated with HDO (all DNA). The acute tolerability score was decreased slightly with the group treated with HDO (G^{MOE}), compared with the group treated with HDO (all DNA). This result has revealed that the HDOs wherein the nucleosides comprising an adenine base, cytosine base, or thymine base in the second nucleic acid strand are substituted with 2'-O-MOE-RNA nucleosides can reduce central nervous system toxicity, compared with the HDO wherein the second nucleic acid strand consists of only DNA nucleosides.

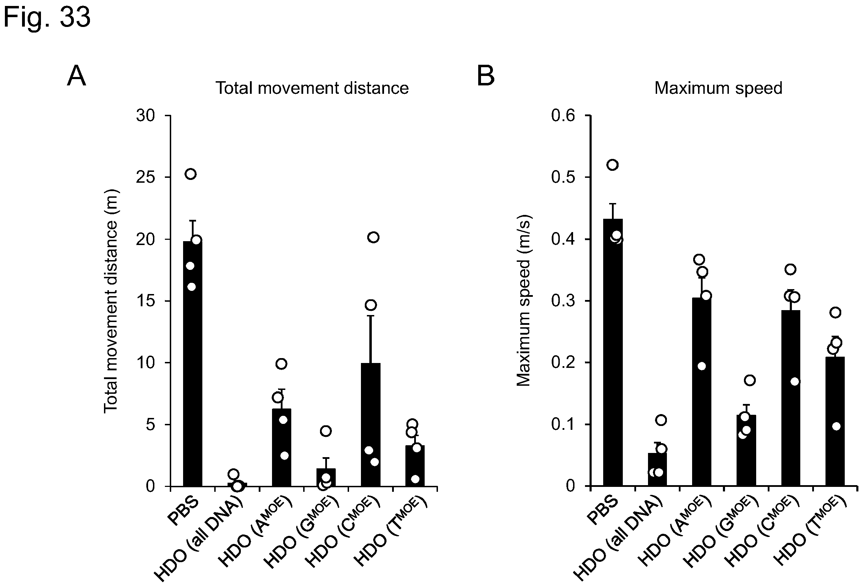

Figure 33 shows the results of evaluation of the motor function of mice at one hour and three hours after various nucleic acid agents were intraventricularly administered to the mice. The total movement distance (Figure 33A) and the maximum moving speed (Figure 33B) were improved noticeably with the groups treated with HDO (A^{MOE}), HDO (C^{MOE}), and HDO (T^{MOE}), compared with the group treated with HDO (all DNA). The total movement distance (Figure 33A) and the maximum moving speed (Figure 33B) were improved slightly with the group treated with HDO (G^{MOE}), compared with the group treated with HDO (all DNA). This result has revealed that the HDOs wherein the nucleosides comprising an adenine base, cytosine base, or thymine base in the second nucleic acid strand are substituted with 2'-O-MOE-RNA nucleosides can reduce central nervous system toxicity, compared with the HDO wherein the second nucleic acid strand consists of only DNA nucleosides.

<Example 10: Comparison of adjacent nucleic acids in MOE modifications>

### (Purpose)

Through an in vivo experiment, the toxicity-reducing effects based on the introduction of an MOE modification into a nucleoside comprising a cytosine base in the second nucleic acid strand and into a nucleoside adjacent thereto on the 5' side and/or 3' side thereof, are studied in terms of central nervous system toxicity observed in intraventricular administration of an HDO comprising: a first nucleic acid strand consisting of an ASO targeting the Mapt gene; and a second nucleic acid strand having a base sequence complementary to the first nucleic acid strand.

### (Method)

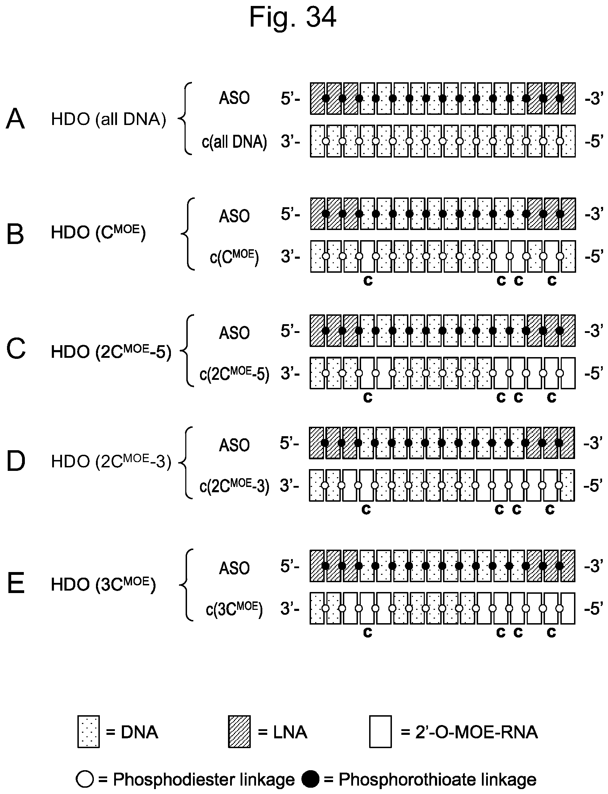

The base sequences and chemical modifications of the ASO, and first nucleic acid strands and second nucleic acid strands constituting the HDO used in this Example are shown in Table 10 and Figure 34.

**[Table 10]**

| Table 10: HDO targeting the Mapt gene | | | |
|---|---|---|---|
| | | Sequence (5'-3') | SEQ ID NO |
| HDO (all DNA) | ASO | a^t^a^g^t^c^a^c^t^c^t^g^g^t^g^a | 1 |
| | c(all DNA) | tcaccagagtgactat | 3 |
| HDO (CMOE) | ASO | a^t^a^g^t^c^a^c^t^c^t^g^g^t^g^a | 1 |
| | c(C^{MOE}) | tC(5)aC(5)C(5)agagtgaC(5)tat | 46 |
| HDO (2C^{MOE}-5) | ASO | a^t^a^g^t^c^a^c^t^c^t^g^g^t^g^a | 1 |
| | c(2C^{MOE}-5) | TC(5)AC(5)C(5)agagtgAC(5)tat | 48 |
| HDO (2C^{MOE}-3) | ASO | a^t^a^g^t^c^a^c^t^c^t^g^g^t^g^a | 1 |
| | c(2C^{MOE}-3) | tC(5)AC(5)C(5)AgagtgaC(5)Tat | 49 |
| HDO (3C^{MOE}) | ASO | a^t^a^g^t^c^a^c^t^c^t^g^g^t^g^a | 1 |
| | c(3C^{MOE}) | TC(5)AC(5)C(5)AgagtgAC(5)Tat | 50 |

| | | | |
|---|---|---|---|
| Lower case letter: DNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is an LNA/DNA gapmer antisense nucleic acid targeting a Mapt mRNA, has a base sequence complementary to part of the Mapt mRNA, and has a structure wherein three LNA nucleosides at the 5' end, three LNA nucleosides at the 3' end, and ten DNA nucleosides therebetween are linked via a phosphorothioate linkage. All of HDO (all DNA), HDO (C^{MOE}), HDO (2C^{MOE}-5), HDO (2C^{MOE}-3), and HDO (3C^{MOE}) used in this Example comprise the ASO as the first nucleic acid strand, and the second nucleic acid strand has a sequence complementary to the first nucleic acid strand. Specifically, the second nucleic acid strand (c(all DNA)) of HDO (all DNA) has a structure wherein the DNA nucleosides are linked via a phosphodiester linkage. The second nucleic acid strand (c(C^{MOE})) of HDO (C^{MOE}) has a structure wherein all of the DNA nucleosides comprising a cytosine base are substituted with 2'-O-MOE-RNA nucleosides. The second nucleic acid strand (c(2C^{MOE}-5)) of HDO (2C^{MOE}-5) has a structure wherein all of the DNA nucleosides comprising a cytosine base and the DNA nucleosides adjacent thereto on the 5' side thereof are substituted with 2'-O-MOE-RNA nucleosides. The second nucleic acid strand (c(2C^{MOE}-3)) of HDO (2C^{MOE}-3) has a structure wherein all of the DNA nucleosides comprising a cytosine base and the DNA nucleosides adjacent thereto on the 3' end side thereof are substituted with 2'-O-MOE-RNA nucleosides. The second nucleic acid strand (c(3C^{MOE})) of HDO (3C^{MOE}) has a structure wherein all of the DNA nucleosides comprising a cytosine base and the DNA nucleosides adjacent thereto on the 5' side and 3' side thereof are substituted with 2'-O-MOE-RNA nucleosides.

For HDO (all DNA), HDO (C^{MOE}), HDO (2C^{MOE}-5), HDO (2C^{MOE}-3), and HDO (3C^{MOE}) described in Table 10, preparation of nucleic acids, in vivo experiments, evaluation of central nervous system toxicity, and evaluation of motor function were performed by the same methods as in Example 1. In this Example, however, the amount of the nucleic acid agent administered per mouse was 19 nmol/mouse.

### (Results)

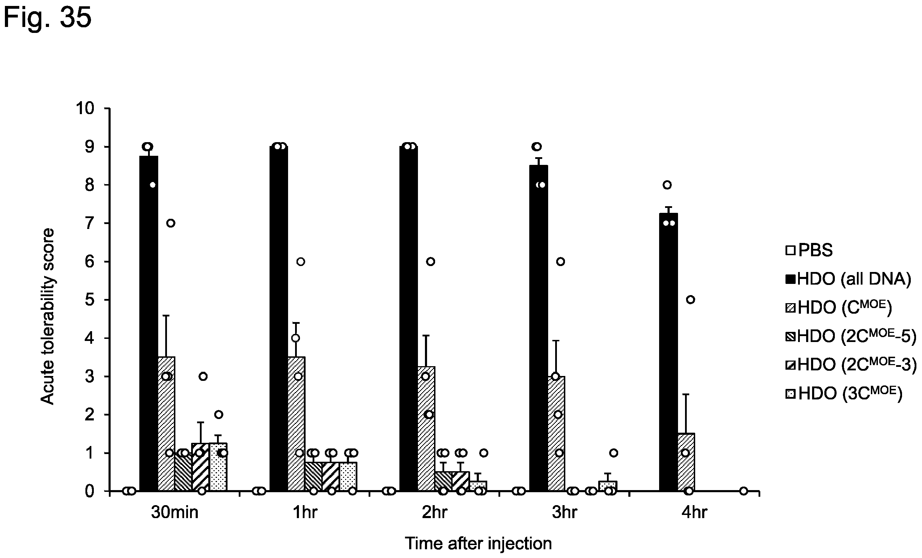

Figure 35 shows the results of evaluation of the central nervous system toxicity in mice to which various nucleic acid agents were intraventricularly administered. At any point of time of 30 minutes, 1 hour, 2 hours, 3 hours, and 4 hours after the administration of the nucleic acid agent, the acute tolerability score was decreased noticeably with the groups treated with HDO (2C^{MOE}-5), HDO (2C^{MOE}-3), and HDO (3C^{MOE}), compared with the groups treated with HDO (all DNA) and HDO (C^{MOE}). This result has revealed that the HDOs wherein the nucleosides comprising a cytosine base in the second nucleic acid strand and the nucleosides adjacent to the nucleosides are substituted with 2'-O-MOE-RNA nucleosides reduce central nervous system toxicity, compared with the HDO wherein the second nucleic acid strand consists of only DNA nucleosides.

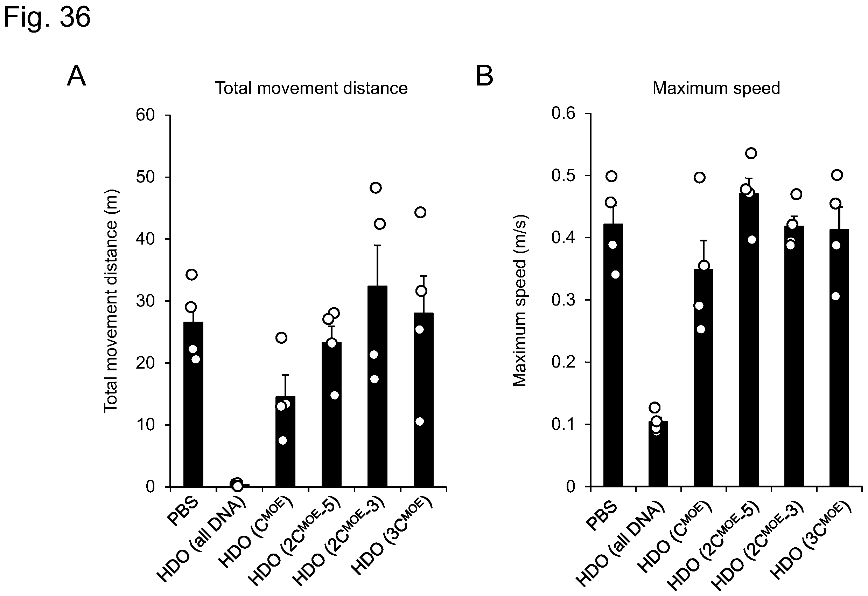

Figure 36 shows the results of evaluation of the motor function of mice at 1 hour and 3 hours after various nucleic acid agents were intraventricularly administered to the mice. The total movement distance (Figure 36A) and the maximum moving speed (Figure 36B) were improved noticeably with the groups treated with HDO (2C^{MOE}-5), HDO (2C^{MOE}-3), and HDO (3C^{MOE}), compared with the groups treated with HDO (all DNA) and HDO (C^{MOE}). This result has revealed that the HDOs wherein the nucleosides comprising a cytosine base in the second nucleic acid strand and the nucleosides adjacent to the nucleosides are substituted with 2'-O-MOE-RNA nucleosides can reduce central nervous system toxicity, compared with the HDO wherein the second nucleic acid strand consists of only DNA nucleosides.

### <Example 11: Stability of HDO in cerebrospinal fluid>

### (Purpose)

The stability of an HDO incubated in cerebrospinal fluid of a human or a rat is examined, wherein the HDO comprises: a first nucleic acid strand consisting of an ASO targeting the Mapt gene; and a second nucleic acid strand having a base sequence complementary to the first nucleic acid strand.

### (Method)

### (1) Preparation of nucleic acids

The base sequences and chemical modifications of the first nucleic acid strands and second nucleic acid strands constituting the HDOs used in this Example are shown in Table 11, Figure 37A, Figure 37B, Figure 38A, and Figure 38B.

**[Table 11]**

| Table 11: HDO targeting the Mapt gene | | | |
|---|---|---|---|
| | | Sequence (5'-3') | SEQ ID NO |
| ASO | ASO | a^t^a^g^t^c^a^c^t^c^t^g^g^t^g^a | 1 |
| HDO (ASO/cRNA) | ASO | a^t^a^g^t^c^a^c^t^c^t^g^g^t^g^a | 1 |
| | cRNA | U^C^A^CCAGAGUGAC^U^A^U | 2 |
| HDO (ASO/cDNA) | ASO | a^t^a^g^t^c^a^c^t^c^t^g^g^t^g^a | 1 |
| | cDNA | tAcAaAccagagtgacAtAaAt | 3 |

| | | | |
|---|---|---|---|
| Lower case letter: DNA; Upper case letter: RNA; Underlined lower case letter: LNA; ^: Phosphorothioate linkage (PS linkage) | | | |

The ASO used in this Example is an LNA/DNA gapmer antisense nucleic acid targeting a Mapt mRNA, has a base sequence complementary to part of the Mapt mRNA, and has a structure wherein three LNA nucleosides at the 5' end, three LNA nucleosides at the 3' end, and ten DNA nucleosides therebetween are linked via a phosphorothioate linkage. All of HDO (ASO/cRNA) and HDO (ASO/cDNA) used in this Example comprise the ASO as the first nucleic acid strand, and the second nucleic acid strand has a sequence complementary to the first nucleic acid strand. Specifically, the second nucleic acid strand (cRNA) of HDO (ASO/cRNA) has a structure wherein the RNA nucleosides are linked via three phosphorothioate linkages from the 5' end, three phosphorothioate linkages from the 3' end, and nine phosphodiester linkages therebetween. The second nucleic acid strand (cDNA) of HDO (ASO/cDNA) has a structure wherein the DNA nucleosides are linked via three phosphorothioate linkages from the 5' end, three phosphorothioate linkages from the 3' end, and nine phosphodiester linkages therebetween.

For HDO (ASO/cRNA) and HDO (ASO/cDNA) described in Table 11, nucleic acids were prepared by the same method as in Example 1.

### (2) Evaluation of stability of nucleic acid agent in cerebrospinal fluid

HDO (ASO/cRNA) and HDO (ASO/cDNA) were each prepared at 10 µM in an amount of 4 µL. With 4 µL of HDO, 16 µL of human cerebrospinal fluid or rat cerebrospinal fluid was mixed, and the resulting mixture was incubated in an incubator at 37°C. At each point of time after 10 minutes, 1 hour, and 6 hours, the mixture was introduced into liquid nitrogen to terminate the reaction.

Acrylamide gel (1 × TBE) at 16% was prepared and produced. The sample in an amount of 6 µL was loaded into the gel, and electrophoresed at 100V for 80 minutes. As controls, an ASO alone and a cRNA alone were simultaneously electrophoresed. Then, a solution was produced by diluting, with 1 × TBE, an aqueous GelRed (×10000) solution (Biotium) to a 1/10000 concentration. The gel was permeated with the solution for 10 minutes. Then, the gel was photographed with a ChemiDoc Touch imaging system (Bio-Rad Laboratories, Inc.).

### (Results)

Figure 37C shows the results of electrophoresis performed after HDO (ASO/cRNA) was incubated in human cerebrospinal fluid (human CSF) for 10 minutes and 6 hours. Figure 37D shows the results of quantification of the band intensity of the HDO double strand band in the results of electrophoresis in Figure 37C. The HDO (ASO/cRNA) in the human cerebrospinal fluid was mostly degraded when incubated for 10 minutes, and entirely degraded when incubated for 6 hours.

Figure 38C shows the results of electrophoresis performed after HDO (ASO/cRNA) or HDO (ASO/cDNA) was incubated in human cerebrospinal fluid or rat cerebrospinal fluid for 6 hours. In the human cerebrospinal fluid, HDO (ASO/cRNA) was degraded and unstable, but HDO (ASO/cDNA) was not degraded and was stable. In the rat cerebrospinal fluid, neither HDO (ASO/cRNA) nor HDO (ASO/cDNA) was degraded, and both of them were stable (Figure 38D).

### <Example 12: Stability of HDO in cerebrospinal fluid>

### (Purpose)

The stability of an HDO incubated in cerebrospinal fluid of a mouse, rat, monkey, or human is examined, wherein the HDO comprises: a first nucleic acid strand consisting of an ASO targeting the Mapt gene; and a second nucleic acid strand having a base sequence complementary to the first nucleic acid strand.

### (Method)

### (1) Preparation of nucleic acids

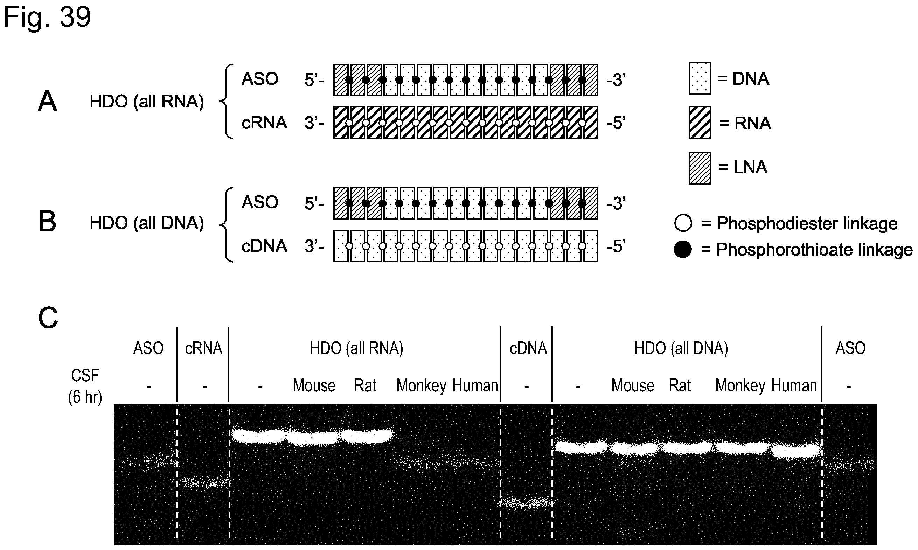

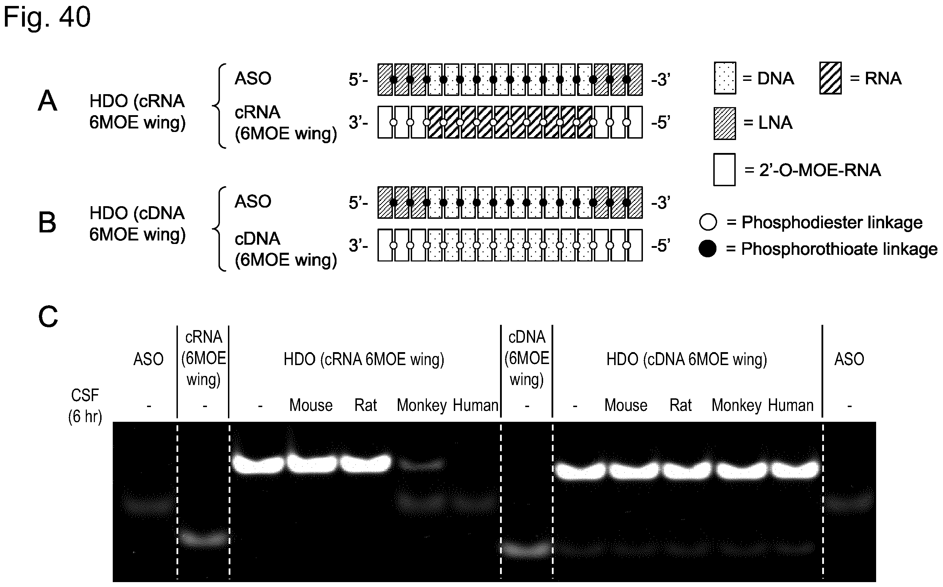

The base sequences and chemical modifications of the first nucleic acid strands and second nucleic acid strands constituting the HDOs used in this Example are shown in Table 12, Figure 39A, Figure 39B, Figure 40A, and Figure 40B.

**[Table 12]**

| Table 12: HDO targeting the Mapt gene | | | |
|---|---|---|---|
| | | Sequence (5'-3') | SEQ ID NO |
| HDO (all RNA) | ASO | a^t^a^g^t^c^a^c^t^c^t^g^g^t^g^a | 1 |
| | cRNA | UCACCAGAGUGACUAU | 2 |
| HDO (all DNA) | ASO | a^t^a^g^t^c^a^c^t^c^t^g^g^t^g^a | 1 |
| | cDNA | tcaccagagtgactat | 3 |
| HDO (cRNA 6MOE wing) | ASO | a^t^a^g^t^c^a^c^t^c^t^g^g^t^g^a | 1 |
| | cRNA(6MOE wing) | TC(5)ACCAGAGUGACTAT | 14 |
| HDO (cDNA 6MOE wing) | ASO | a^t^a^g^t^c^a^c^t^c^t^g^g^t^g^a | 1 |
| | cDNA(6MOE wing) | TC(5)AccagagtgacTAT | 4 |

| | | | |
|---|---|---|---|
| Lower case letter: DNA; Upper case letter: RNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is an LNA/DNA gapmer antisense nucleic acid targeting a Mapt mRNA, has a base sequence complementary to part of the Mapt mRNA, and has a structure wherein three LNA nucleosides at the 5' end, three LNA nucleosides at the 3' end, and ten DNA nucleosides therebetween are linked via a phosphorothioate linkage. All of HDO (all RNA), HDO (all DNA), HDO (cRNA 6MOE wing), and HDO (cDNA 6MOE wing) used in this Example comprise the ASO as the first nucleic acid strand, and the second nucleic acid strand has a sequence complementary to the first nucleic acid strand. Specifically, the second nucleic acid strand (cRNA) of HDO (all RNA) has a structure wherein the RNA nucleosides are linked via a phosphodiester linkage. The second nucleic acid strand (cDNA) of HDO (all DNA) has a structure wherein DNA nucleosides are linked via a phosphodiester linkage. The second nucleic acid strand (cRNA(6MOE wing)) of HDO (cRNA 6MOE wing) has a structure wherein three 2'-O-MOE-RNA nucleosides at the 5' end, three 2'-O-MOE-RNA nucleosides at the 3' end, and ten RNA nucleosides therebetween are linked via a phosphodiester linkage. The second nucleic acid strand (cDNA(6MOE wing)) of HDO (cDNA 6MOE wing) has a structure wherein three 2'-O-MOE-RNA nucleosides at the 5' end, three 2'-O-MOE-RNA nucleosides at the 3' end, and ten DNA nucleosides therebetween are linked via a phosphodiester linkage.

For HDO (all RNA), HDO (all DNA), HDO (cRNA 6MOE wing), and HDO (cDNA 6MOE wing) described in Table 12, preparation of nucleic acids and evaluation of stability in cerebrospinal fluid were performed by the same methods as in Example 11.

### (Results)

Figure 39C shows the results of electrophoresis performed after HDO (all RNA) and HDO (all DNA) were incubated in the cerebrospinal fluids of a mouse, rat, monkey, and human for 6 hours. HDO (all RNA) was not degraded and was stable in the cerebrospinal fluids of a mouse and a rat, but degraded and unstable in the cerebrospinal fluids of a monkey and a human. Compared with this, HDO (all DNA) was not degraded and was stable in the cerebrospinal fluid of any of a mouse, rat, monkey, and human.

Figure 40C shows the results of electrophoresis performed after HDO (cRNA 6MOE wing) and HDO (cDNA 6MOE wing) were incubated in the cerebrospinal fluids of a mouse, rat, monkey, and human for 6 hours. HDO (cRNA 6MOE wing) was not degraded and was stable in the cerebrospinal fluids of a mouse and a rat, but degraded and unstable in the cerebrospinal fluids of a monkey and a human. Compared with this, HDO (cDNA 6MOE wing) was not degraded and was stable in the cerebrospinal fluid of any of a mouse, rat, monkey, and human.

### <Example 13: Stability of HDO in cerebrospinal fluid>

### (Purpose)

The stability of an HDO in cerebrospinal fluid is examined, wherein the HDO comprises: a first nucleic acid strand consisting of an ASO targeting the Mapt gene; and a second nucleic acid strand having a base sequence complementary to the first nucleic acid strand, wherein part of the DNA nucleosides in the second nucleic acid strand are substituted with RNA nucleosides.

### (Method)

### (1) Preparation of nucleic acids

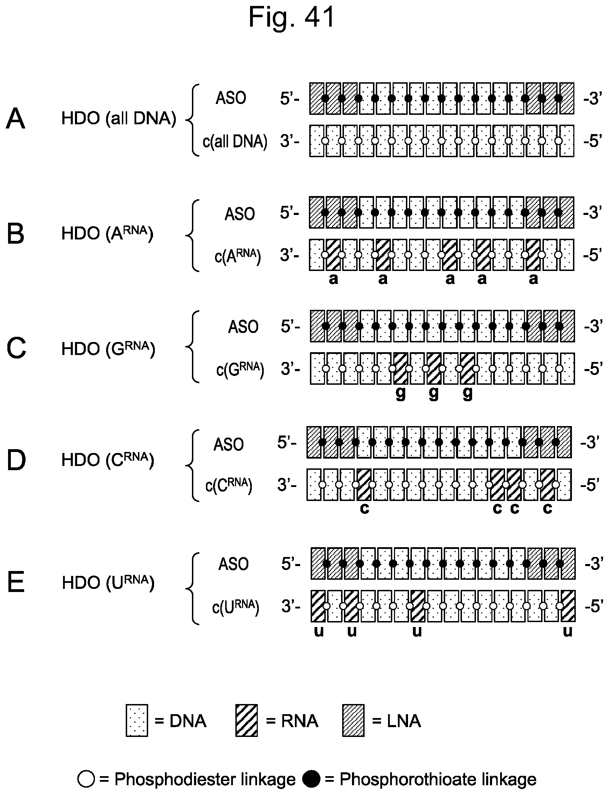

The base sequences and chemical modifications of the first nucleic acid strands and second nucleic acid strands constituting the HDOs used in this Example are shown in Table 13 and Figure 41.

**[Table 13]**

| Table 13: HDO targeting the Mapt gene | | | |
|---|---|---|---|
| | | Sequence (5'-3') | SEQ ID NO |
| HDO (all DNA) | ASO | **a^t^a^g^t^c^a^c^t^c^t^g^g^t^g^a** | 1 |
| | c(all DNA) | **tcaccagagtgactat** | 3 |
| HDO (ARNA) | ASO | **a^t^a^g^t^c^a^c^t^c^t^g^g^t^g^a** | 1 |
| | c(A^{RNA}) | **tcAccAgAgtgActAt** | 34 |
| HDO (G^{RNA}) | ASO | **a^t^a^g^t^c^a^c^t^c^t^g^g^t^g^a** | 1 |
| | c(G^{RNA}) | **tcaccaGaGtGactat** | 35 |
| HDO (CRNA) | ASO | **a^t^a^g^t^c^a^c^t^c^t^g^g^t^g^a** | 1 |
| | c(C^{RNA}) | **tCaCCagagtgaCtat** | 36 |
| HDO (URNA) | ASO | **a^t^a^g^t^c^a^c^t^c^t^g^g^t^g^a** | 1 |
| | c(U^{RNA}) | **UcaccagagUgacUaU** | 37 |

| | | | |
|---|---|---|---|
| Lower case letter: DNA; Upper case letter: RNA; Underlined lower case letter: LNA; ^: Phosphorothioate linkage (PS linkage) | | | |

The ASO used in this Example is an LNA/DNA gapmer antisense nucleic acid targeting a Mapt mRNA, has a base sequence complementary to part of the Mapt mRNA, and has a structure wherein three LNA nucleosides at the 5' end, three LNA nucleosides at the 3' end, and ten DNA nucleosides therebetween are linked via a phosphorothioate linkage. All of HDO (all DNA), HDO (A^{RNA}), HDO (G^{RNA}), HDO (C^{RNA}), and HDO (U^{RNA}) used in this Example comprise the ASO as the first nucleic acid strand, and the second nucleic acid strand has a sequence complementary to the first nucleic acid strand. Specifically, the second nucleic acid strand (c(all DNA)) of HDO (all DNA) has a structure wherein the DNA nucleosides are linked via a phosphodiester linkage. The second nucleic acid strand (c(A^{RNA})) of HDO (A^{RNA}) has a structure wherein all of the DNA nucleosides comprising an adenine base in c(all DNA) are substituted with RNA nucleosides. The second nucleic acid strand (c(G^{RNA})) of HDO (G^{RNA}) has a structure wherein all of the DNA nucleosides comprising a guanine base in c(all DNA) are substituted with RNA nucleosides. The second nucleic acid strand (c(C^{RNA})) of HDO (C^{RNA}) has a structure wherein all of the DNA nucleosides comprising a cytosine base in c(all DNA) are substituted with RNA nucleosides. The second nucleic acid strand (c(U^{RNA})) of HDO (U^{RNA}) has a structure wherein all of the DNA nucleosides comprising a thymine base in c(all DNA) are substituted with RNA nucleosides comprising a uracil base.

For HDO (all DNA), HDO (A^{RNA}), HDO (G^{RNA}), HDO (C^{RNA}), and HDO (U^{RNA}) described in Table 13, preparation of nucleic acids and evaluation of the stability in the cerebrospinal fluid were performed by the same methods as in Example 11.

### (Results)

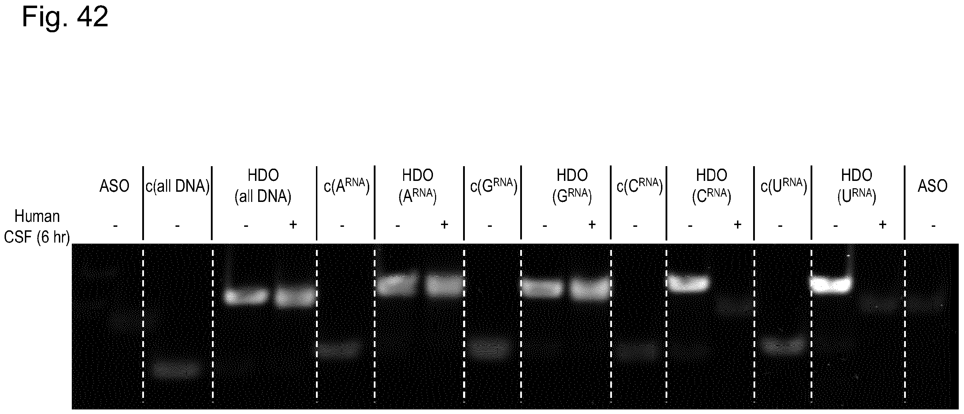

Figure 42 shows the results of electrophoresis performed after HDO (all DNA), HDO (A^{RNA}), HDO (G^{RNA}), HDO (C^{RNA}), and HDO (U^{RNA}) were incubated in the human cerebrospinal fluid for 6 hours. HDO (all DNA), HDO (A^{RNA}), and HDO (G^{RNA}) were not degraded, and were stable in the human cerebrospinal fluid. Meanwhile, HDO (C^{RNA}), and HDO (U^{RNA}) were degraded and unstable in the human cerebrospinal fluid.

### <Example 14: Stability of HDO in cerebrospinal fluid>

### (Purpose)

The stability of an HDO in cerebrospinal fluid is examined, wherein the HDO comprises: a first nucleic acid strand consisting of an ASO targeting the Mapt gene; and a second nucleic acid strand having a base sequence complementary to the first nucleic acid strand, wherein part of the DNA nucleosides in the second nucleic acid strand are substituted with RNA nucleosides.

### (Method)

### (1) Preparation of nucleic acids

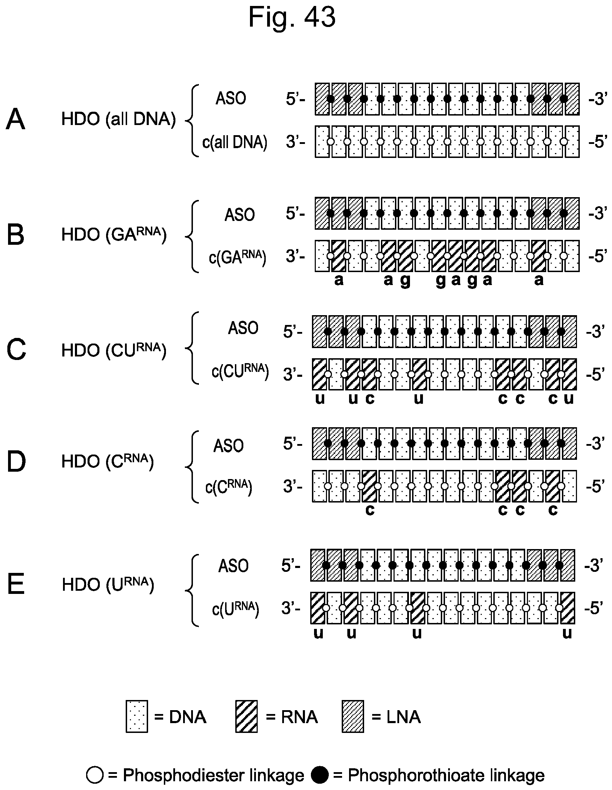

The base sequences and chemical modifications of the first nucleic acid strands and second nucleic acid strands constituting the HDOs used in this Example are shown in Table 14 and Figure 43.

**[Table 14]**

| Table 14: HDO targeting the Mapt gene | | | |
|---|---|---|---|
| | | Sequence (5'-3') | SEQ ID NO |
| HDO (all DNA) | ASO | **a^t^a^g^t^c^a^c^t^c^t^g^g^t^g^a** | 1 |
| | c(all DNA) | **tcaccagagtgactat** | 3 |
| HDO (GA^{RNA}) | ASO | **a^t^a^g^t^c^a^c^t^c^t^g^g^t^g^a** | 1 |
| | c(GA^{RNA}) | **tcAccAGAGtGActAt** | 38 |
| HDO (CU^{RNA}) | ASO | **a^t^a^g^t^c^a^c^t^c^t^g^g^t^g^a** | 1 |
| | c(CU^{RNA}) | **UCaCCagagUgaCUaU** | 39 |
| HDO (CRNA) | ASO | **a^t^a^g^t^c^a^c^t^c^t^g^g^t^g^a** | 1 |
| | c(C^{RNA}) | **tCaCCagagtgaCtat** | 36 |
| HDO (URNA) | ASO | **a^t^a^g^t^c^a^c^t^c^t^g^g^t^g^a** | 1 |
| | c(U^{RNA}) | **UcaccagagUgacUaU** | 37 |

| | | | |
|---|---|---|---|
| Lower case letter: DNA; Upper case letter: RNA; Underlined lower case letter: LNA; ^: Phosphorothioate linkage (PS linkage) | | | |

The ASO used in this Example is an LNA/DNA gapmer antisense nucleic acid targeting a Mapt mRNA, has a base sequence complementary to part of the Mapt mRNA, and has a structure wherein three LNA nucleosides at the 5' end, three LNA nucleosides at the 3' end, and ten DNA nucleosides therebetween are linked via a phosphorothioate linkage. All of HDO (all DNA), HDO (GA^{RNA}), HDO (CU^{RNA}), HDO (C^{RNA}), and HDO (U^{RNA}) used in this Example comprise the ASO as the first nucleic acid strand, and the second nucleic acid strand has a sequence complementary to the first nucleic acid strand. Specifically, the second nucleic acid strand (c(all DNA)) of HDO (all DNA) has a structure wherein the DNA nucleosides are linked via a phosphodiester linkage. The second nucleic acid strand (c(GA^{RNA})) of HDO (GA^{RNA}) has a structure wherein, in c(all DNA), all of the DNA nucleosides comprising a guanine base and the DNA nucleosides comprising an adenine base are substituted with RNA nucleosides. The second nucleic acid strand (c(CU^{RNA})) of HDO (CU^{RNA}) has a structure wherein, in c(all DNA), all of the DNA nucleosides comprising a cytosine base are substituted with RNA nucleosides, and all of the DNA nucleosides comprising a thymine base are substituted with RNA nucleosides comprising a uracil base. The second nucleic acid strand (c(C^{RNA})) of HDO (C^{RNA}) has a structure wherein all of the DNA nucleosides comprising a cytosine base in c(all DNA) are substituted with RNA nucleosides. The second nucleic acid strand (c(U^{RNA})) of HDO (U^{RNA}) has a structure wherein all of the DNA nucleosides comprising a thymine base in c(all DNA) are substituted with RNA nucleosides comprising a uracil base.

For HDO (all DNA), HDO (GA^{RNA}), HDO (CU^{RNA}), HDO (C^{RNA}), and HDO (U^{RNA}) described in Table 14, preparation of nucleic acids and evaluation of the stability in the cerebrospinal fluid were performed by the same methods as in Example 11.

### (Results)

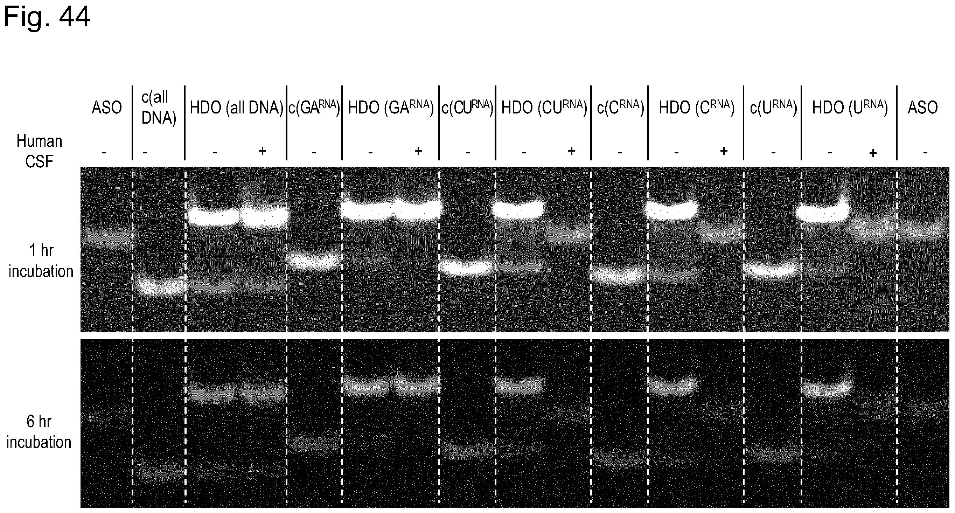

Figure 44 shows the results of electrophoresis performed after HDO (all DNA), HDO (GA^{RNA}), HDO (CU^{RNA}), HDO (C^{RNA}), and HDO (U^{RNA}) were incubated in human cerebrospinal fluid for one hour and 6 hours. HDO (all DNA) and HDO (GA^{RNA}) were not degraded, and were stable in the human cerebrospinal fluid. Meanwhile, HDO (CU^{RNA}), HDO (C^{RNA}), and HDO (U^{RNA}) were degraded and unstable in the human cerebrospinal fluid.

### <Example 15: Stability of HDO in cerebrospinal fluid>

### (Purpose)

The stability of an HDO in cerebrospinal fluid is examined, wherein the HDO comprises: a first nucleic acid strand consisting of an ASO targeting the Malat1 gene; and a second nucleic acid strand having a base sequence complementary to the first nucleic acid strand, wherein part of the DNA nucleosides in the second nucleic acid strand are substituted with RNA nucleosides.

### (Method)

### (1) Preparation of nucleic acids

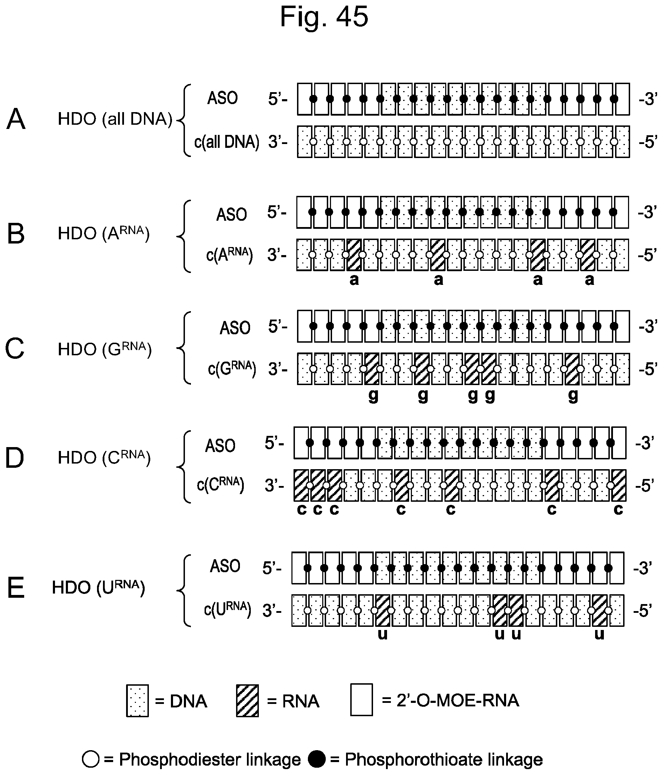

The base sequences and chemical modifications of the first nucleic acid strands and second nucleic acid strands constituting the HDOs used in this Example are shown in Table 15 and Figure 45.

**[Table 15]**

| Table 15: HDO targeting the Malat1gene | | | |
|---|---|---|---|
| | | Sequence (5'-3') | SEQ ID NO |
| HDO (all DNA) | ASO | **G^G^G^T^C(5)^a^g^c^t^g^c^c^a^a^t^G^C(5)^T^A^G** | 10 |
| | c(all DNA) | **ctagcattggcagctgaccc** | 12 |
| HDO (A^{RNA}) | ASO | **G^G^G^T^C(5)^a^g^c^t^g^c^c^a^a^t^G^C(5)^T^A^G** | 10 |
| | c(A^{RNA}) | **ctAgcAttggcAgctgAccc** | 40 |
| HDO (G^{RNA}) | ASO | **G^G^G^T^C(5)^a^g^c^t^g^c^c^a^a^t^G^C(5)^T^A^G** | 10 |
| | c(G^{RNA}) | **ctaGcattGGcaGctGaccc** | 41 |
| HDO (CRNA) | ASO | **G^G^G^T^C(5)^a^g^c^t^g^c^c^a^a^t^G^C(5)^T^A^G** | 10 |
| | c(C^{RNA}) | **CtagCattggCagCtgaCCC** | 42 |
| HDO (URNA) | ASO | **G^G^G^T^C(5)^a^g^c^t^g^c^c^a^a^t^G^C(5)^T^A^G** | 10 |
| | c(U^{RNA}) | **cUagcaUUggcagcUgaccc** | 43 |

| | | | |
|---|---|---|---|
| Lower case letter: DNA; Upper case letter: RNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is a 2'-O-MOE-RNA/DNA gapmer antisense nucleic acid targeting the Malat1 ncRNA, has a base sequence complementary to part of the Malat1 ncRNA, and has a structure wherein five 2'-O-MOE-RNA nucleosides at the 5' end, five 2'-O-MOE-RNA nucleosides at the 3' end, and ten DNA nucleosides therebetween are linked via a phosphorothioate linkage. All of HDO (all DNA), HDO (A^{RNA}), HDO (G^{RNA}), HDO (C^{RNA}), and HDO (U^{RNA}) used in this Example comprise the ASO as the first nucleic acid strand, and the second nucleic acid strand has a sequence complementary to the first nucleic acid strand. Specifically, the second nucleic acid strand (c(all DNA)) of HDO (all DNA) has a structure wherein the DNA nucleosides are linked via a phosphodiester linkage. The second nucleic acid strand (c(A^{RNA})) of HDO (A^{RNA}) has a structure wherein all of the DNA nucleosides comprising an adenine base in c(all DNA) are substituted with RNA nucleosides. The second nucleic acid strand (c(G^{RNA})) of HDO (G^{RNA}) has a structure wherein all of the DNA nucleosides comprising a guanine base in c(all DNA) are substituted with RNA nucleosides. The second nucleic acid strand (c(C^{RNA})) of HDO (C^{RNA}) has a structure wherein all of the DNA nucleosides comprising a cytosine base in c(all DNA) are substituted with RNA nucleosides. The second nucleic acid strand (c(U^{RNA})) of HDO (U^{RNA}) has a structure wherein all of the DNA nucleosides comprising a thymine base in c(all DNA) are substituted with RNA nucleosides comprising a uracil base.

For HDO (all DNA), HDO (A^{RNA}), HDO (G^{RNA}), HDO (C^{RNA}), and HDO (U^{RNA}) described in Table 15, preparation of nucleic acids and evaluation of the stability in the cerebrospinal fluid were performed by the same methods as in Example 11.

### (Results)

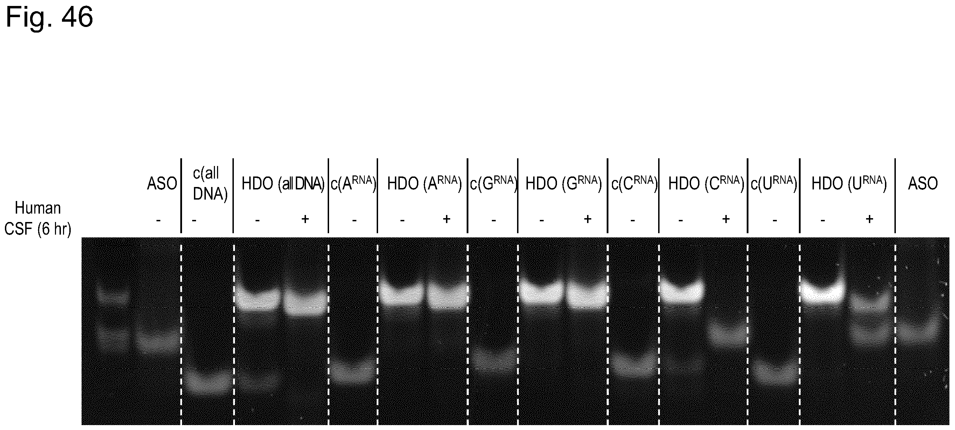

Figure 46 shows the results of electrophoresis performed after HDO (all DNA), HDO (A^{RNA}), HDO (G^{RNA}), HDO (C^{RNA}), and HDO (U^{RNA}) were incubated in the human cerebrospinal fluid for 6 hours. HDO (all DNA), HDO (A^{RNA}), and HDO (G^{RNA}) were not degraded, and were stable in the human cerebrospinal fluid. Meanwhile, HDO (C^{RNA}), and HDO (U^{RNA}) were degraded and unstable in the human cerebrospinal fluid.

### <Example 16: Evaluation of central nervous system toxicity in monkey>

### (Purpose)

The central nervous system toxicity in monkeys is evaluated, wherein an HDO comprising MOE modifications is intrathecally administered to the monkeys.

### (Method)

### (1) Preparation of nucleic acids

The base sequences and chemical modifications of the ASO, and the first nucleic acid strands and second nucleic acid strands constituting the HDO used in this Example are shown in Table 16 and Figures 47A to 47C.

**[Table 16]**

| Table 16: HDO targeting the Mapt gene | | | |
|---|---|---|---|
| | | Sequence (5'-3') | SEQ ID NO |
| ASO | ASO | **a^t^a^g^t^c^a^c^t^c^t^g^g^t^g^a** | 1 |
| HDO (RNA-MOE) | ASO | **a^t^a^g^t^c^a^c^t^c^t^g^g^t^g^a** | 1 |
| | c(RNA-MOE) | **TC(5)ACCAGAGUGACTAT** | 14 |
| HDO (DNA-MOE) | ASO | **a^t^a^g^t^c^a^c^t^c^t^g^g^t^g^a** | 1 |
| | c(DNA-MOE) | **TC(5)AccagagtgacTAT** | 4 |

| | | | |
|---|---|---|---|
| Lower case letter: DNA; Upper case letter: RNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

For ASO, HDO (RNA-MOE), and HDO (DNA-MOE) described in Table 16, nucleic acids were prepared by the same method as in Example 1.

### (2) Evaluation of central nervous system toxicity in monkey

To male cynomolgus monkeys (3 years old, 2 kg), ASO, HDO (RNA-MOE), and HDO (DNA-MOE) were administered. In the administration method, 5 mg of ASO or 0.94 µmol of HDO was intrathecally administered.

The behaviors of the monkeys were observed at 2 hours after the intrathecal administration (Figure 47D).

### (Results)

The results are shown in Figure 47E. The monkey to which an ASO was intrathecally administered exhibited serious quadriparesis and strong central nervous system toxicity. The monkey to which HDO (RNA-MOE) was intrathecally administered exhibited an approximately medium degree of paralysis in the lower limbs, but exhibited reduced central nervous system toxicity, compared with the monkey to which the ASO was intrathecally administered. The monkey to which HDO (DNA-MOE) was intrathecally administered did not exhibit paralysis, and exhibited a normal state of consciousness and a normal motor function.

### <Example 17: HDO having bulge structure>

### (Purpose)

An HDO comprising: a first nucleic acid strand consisting of an ASO targeting the Mapt gene; and a second nucleic acid strand having a base sequence complementary to the first nucleic acid strand is introduced into a neuronal cell line. The toxicity-reducing effect and the gene suppression effect by virtue of introducing a bulge structure into the second nucleic acid strand is investigated through an in vitro experiment.

### (Method)

### (1) Preparation of nucleic acids

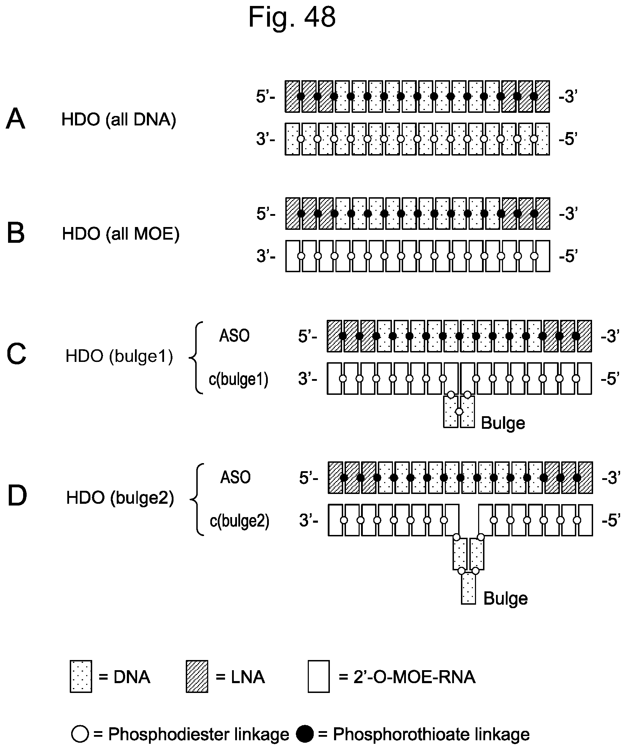

The base sequences and chemical modifications of the first nucleic acid strands and second nucleic acid strands constituting the HDOs used in this Example are shown in Table 17 and Figure 48.

**[Table 17]**

| Table 17: HDO having bulge structure | | | |
|---|---|---|---|
| | | Sequence (5'-3') | SEQ ID NO |
| HDO (all DNA) | ASO | **a^t^a^g^t^c^a^c^t^c^t^g^g^t^g^a** | 1 |
| | c(all DNA) | **tcaccagagtgactat** | 3 |
| HDO (all MOE) | ASO | **a^t^a^g^t^c^a^c^t^c^t^g^g^t^g^a** | 1 |
| | c(all MOE) | **TC(5)AC(5)C(5)AGAGTGAC(5)TAT** | 5 |
| HDO (bulge1) | ASO | **a^t^a^g^t^c^a^c^t^c^t^g^g^t^g^a** | 1 |
| | c(bulge1) | **TC(5)AC(5)C(5)AGAttGTGAC(5)TAT** | 51 |
| HDO (bulge2) | ASO | **a^t^a^g^t^c^a^c^t^c^t^g^g^t^g^a** | 1 |
| | c(bulge2) | **TC(5)AC(5)C(5)AGtttGTGAC(5)TAT** | 52 |

| | | | |
|---|---|---|---|
| Lower case letter:DNA;Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is an LNA/DNA gapmer antisense nucleic acid targeting a Mapt mRNA, has a base sequence complementary to part of the Mapt mRNA, and has a structure wherein three LNA nucleosides at the 5' end, three LNA nucleosides at the 3' end, and ten DNA nucleosides therebetween are linked via a phosphorothioate linkage. All of HDO (all DNA), HDO (all MOE), HDO (bulge1), and HDO (bulge2) used in this Example comprise the ASO as the first nucleic acid strand, and the second nucleic acid strand comprises a sequence complementary to the first nucleic acid strand. Specifically, the second nucleic acid strand (c(all DNA)) of HDO (all DNA) has a structure wherein the DNA nucleosides are linked via a phosphodiester linkage. The second nucleic acid strand (c(all MOE)) of HDO (all MOE) has a structure wherein the 2'-O-MOE-RNA nucleosides are linked via a phosphodiester linkage.

The second nucleic acid strand (c(bulge1)) of HDO (bulge1) comprises: a complementary region consisting of a base sequence complementary to the full length of the first nucleic acid strand; and a bulge structure positioned in the center of the complementary region. The complementary region has a structure wherein the 2'-O-MOE-RNA nucleosides are linked via a phosphodiester linkage. The bulge structure has a structure wherein two DNA nucleosides are linked via a phosphodiester linkage.

The second nucleic acid strand (c(bulge2)) of HDO (bulge2) comprises: a complementary region consisting of a base sequence complementary to the first nucleic acid strand; and a bulge structure placed in the center of the complementary region. The complementary region has a deletion of one base from the first nucleic acid strand, and at this position of deletion, a bulge structure is placed. The complementary region has a structure wherein the 2'-O-MOE-RNA nucleosides are linked via a phosphodiester linkage. The bulge structure has a structure wherein three DNA nucleosides are linked via a phosphodiester linkage.

For HDO (all DNA), HDO (all MOE), HDO (bulge1), and HDO (bulge2) described in Table 17, nucleic acids were prepared by the same method as in Example 1.

### (2) Evaluation of gene suppression effect

The HDO prepared in (1) was introduced into a human neuroblastoma-derived cell (BE (2)-M17 cell line) using a lipofection method (lipofectamine 2000).

Using an IsogenI kit (GeneDesign Inc.), RNA was extracted from the cells 48 hours after the introduction of the HDO. A cDNA was synthesized using Transcriptor Universal cDNA Master, DNase (Roche Diagnostics) in accordance with the protocol. Next, the resulting cDNA template was used to perform quantitative RT-PCR, whereby the expression levels of Mapt mRNA and Actb mRNA (the internal standard gene) were measured. Quantitative RT-PCR was performed with TaqMan (Roche Applied Science). Primers used in quantitative RT-PCR were products designed and manufactured by Thermo Fisher Scientific Inc. (formerly known as Life Technologies Corp.). Amplification was performed by repeating, 40 times, the following cycle: 95°C for 15 seconds, 60°C for 30 seconds, and 72°C for 1 second.

The ratio of the expression level of Mapt mRNA to the expression level of Actb mRNA (internal standard gene) was calculated, and a value standardized with respect to the value of the PBS-treated group was determined as a relative Mapt mRNA level.

### (3) Evaluation of cell toxicity

To evaluate the neurotoxicity at 48 hours after the introduction, the lactate dehydrogenase (LDH) activity in the cell supernatant was measured. The LDH activity was measured using Cytotoxicity LDH Assay Kit-WST (Dojindo Laboratories) in accordance with the attached protocol. A value standardized with respect to the LDH activity of a PBS-treated group was determined as a relative LDH release level.

### (Results)

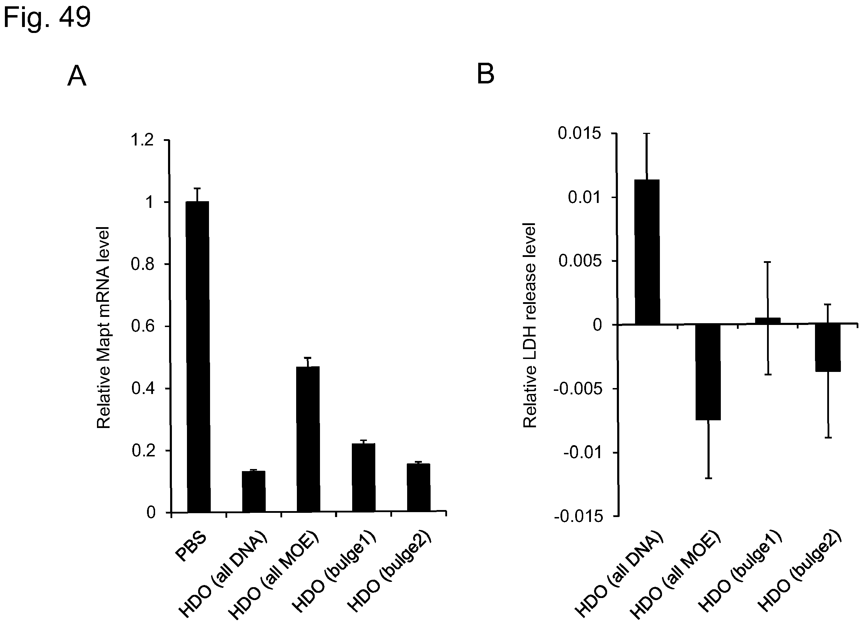

Figure 49A shows the results of evaluation of the target gene suppression effect of various nucleic acid agents in human neuroblastoma-derived cells (BE (2)-M17 cell line). The suppression effect was attenuated noticeably with the group treated with HDO (all MOE), compared with HDO (all DNA). In contrast, strong gene suppression effect comparable to HDO (all DNA) was produced in the administration groups of HDO (bulge1) and HDO (bulge2).

Figure 49B shows the results of evaluation of the LDH activity in the supernatant, as the cell toxicity exhibited when various nucleic acid agents were introduced into the cell. The group treated with HDO (all DNA) exhibited an increase in the LDH activity, but the administration groups of HDO (all MOE), HDO (bulge1), and HDO (bulge2) exhibited an LDH activity comparable to or below the PBS administration group, and the cell toxicity was decreased noticeably. This result has revealed that the HDOs comprising a 2'O-MOE-RNA nucleoside achieve a noticeable decrease in cell toxicity.

The above-described results have revealed that HDO (bulge1) and HDO (bulge2) can produce a strong gene suppression effect without inducing neuronal cell toxicity.

### <Example 18: Single-stranded HDO>

### (Purpose)

Through an in vivo experiment, the toxicity-reducing effect and the gene suppression effect that are based on the introduction of a bulge structure into the second nucleic acid strand, on the linkage between the first nucleic acid strand and the second nucleic acid strand via a linker, and on both the introduction and the linkage are investigated in terms of central nervous system toxicity observed in intraventricular administration of an HDO comprising: a first nucleic acid strand consisting of an ASO targeting the Mapt gene; and a second nucleic acid strand having a base sequence complementary to the first nucleic acid strand.

### (Method)

### (1) Preparation of nucleic acids

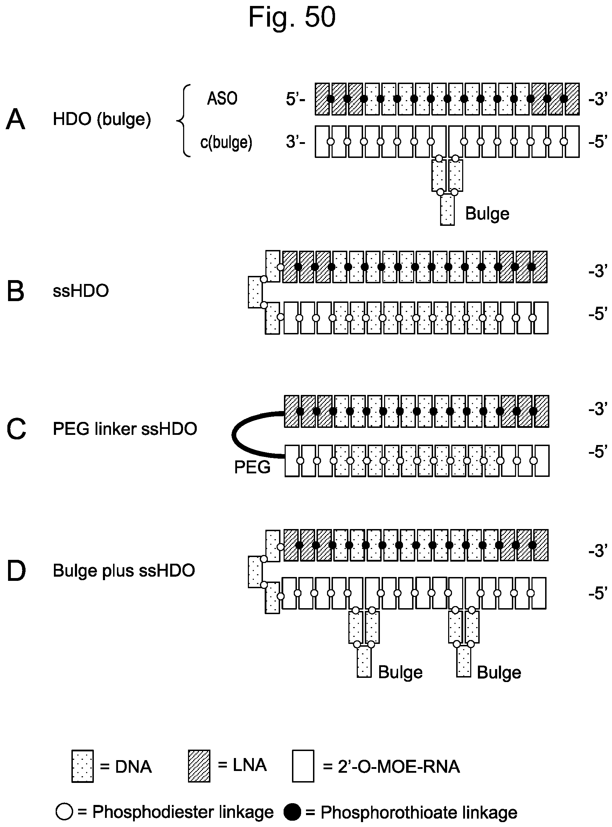

The base sequences and chemical modifications of the HDOs used in this Example are shown in Table 18 and Figure 50.

**[Table 18]**

| Table 18: Single-stranded HDO | | | |
|---|---|---|---|
| | | Sequence (5'-3') | SEQ ID NO |
| HDO (bulge) | ASO | a^t^a^g^t^c^a^c^t^c^t^g^g^t^g^a | 1 |
| | c(bulge) | **TC(5)AC(5)C(5)AGAtttGTGAC(5)TAT** | 53 |
| ssHDO | | TC(5)AccagagtgacTATaaaa^t^a^g^t^c^a^c^t ^c^t^g^g^t^g^a | 54 |
| PEG linker ssHDO | | TC(5)AccagagtgacTAT*a^t^a^g^t^c^a^c^t^c ^t^g^g^t^g^a | 55 |
| Bulge plus ssHDO | | TC(5)AC(5)C(5)tttAGAGTGtttAC(5)TATaaaa^ t^a^g^t^c^a^c^t^c^t^g^g^t^g^a | 56 |

| | | | |
|---|---|---|---|
| Lower case letter: DNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage); *: PEG linker | | | |

Any of HDO (bulge), ssHDO, PEG linker ssHDO, and Bulge plus ssHDO used in this Example comprises the first nucleic acid strand and the second nucleic acid strand. The first nucleic acid strand of any of HDO (bulge), ssHDO, PEG linker ssHDO, and Bulge plus ssHDO is an LNA/DNA gapmer antisense nucleic acid, has a base sequence complementary to part of Mapt mRNA, and has a structure wherein three LNA nucleosides at the 5' end, three LNA nucleosides at the 3' end, and ten DNA nucleosides therebetween are linked via a phosphorothioate linkage. The first nucleic acid strand and the second nucleic acid strand of HDO (bulge) are not linked via a linker, but in ssHDO, PEG linker ssHDO, and Bulge plus ssHDO, the 3' end of the second nucleic acid strand is bound to the 5' end of the first nucleic acid strand via a linker.

The second nucleic acid strand (c(bulge)) of HDO (bulge) comprises: a complementary region consisting of a base sequence complementary to the first nucleic acid strand; and a bulge structure. The complementary region has a structure wherein the 2'-O-MOE-RNA nucleosides are linked via a phosphodiester linkage. The bulge structure has a structure wherein three DNA nucleosides are linked via a phosphodiester linkage.

The second nucleic acid strand of ssHDO has a sequence complementary to the first nucleic acid strand, and has a structure wherein three 2'-O-MOE-RNA nucleosides, ten DNA nucleosides, and three 2'-O-MOE-RNA nucleosides are linked in this order from the 5' end via a phosphodiester linkage. The linker that links the first nucleic acid strand and the second nucleic acid strand in ssHDO consists of three DNA nucleosides linked via a phosphodiester linkage.

The structure of the second nucleic acid strand of PEG linker ssHDO is the same as the structure of the above-described second nucleic acid strand of the ssHDO. The linker that links the first nucleic acid strand and the second nucleic acid strand in PEG linker ssHDO consists of PEG (polyethylene glycol).

The second nucleic acid strand of Bulge plus ssHDO comprises: a complementary region consisting of a base sequence complementary to the first nucleic acid strand; and two bulge structures. The complementary region has a structure wherein the 2'-O-MOE-RNA nucleosides are linked via a phosphodiester linkage. The two bulge structures each has a structure wherein three DNA nucleosides are linked via a phosphodiester linkage. The linker that links the first nucleic acid strand and the second nucleic acid strand in Bulge plus ssHDO consists of three DNA nucleosides linked via a phosphodiester linkage.

For ssHDO, PEG linker ssHDO, and Bulge plus ssHDO described in Table 18, preparation of nucleic acids, in vivo experiments, evaluation of central nervous system toxicity, evaluation of motor function, and evaluation of the gene suppression effect are performed by the same methods as in Example 1.

### (Results)

In the mice to which HDO (bulge), ssHDO, PEG linker ssHDO, and Bulge plus ssHDO are intraventricularly administered, the acute tolerability score is greatly decreased at 30 minutes to 4 hours after the administration, compared with e.g., the mice to which HDO (all DNA) used Example 1 is administered. In addition, in mice to which HDO (bulge), ssHDO, PEG linker ssHDO, and Bulge plus ssHDO are intraventricularly administered, the motor function, e.g., the total movement distance or the maximum moving speed, is greatly improved at one hour after the administration, compared with mice and the like to which HDO (all DNA) used Example 1 is administered. Furthermore, in mice to which HDO (bulge), ssHDO, PEG linker ssHDO, and Bulge plus ssHDO are intraventricularly administered, the Mapt mRNA expression level is decreased in the hippocampus at 7 days after the administration, revealing an improvement in the target gene suppression effect, compared with e.g., mice to which HDO (all MOE) used in Example 1 is administered. The above-described results indicate that introducing a bulge structure into the second nucleic acid strand, and linking the first nucleic acid strand and the second nucleic acid strand in HDO via a linker can achieve both a reduction in central nervous system toxicity, and an excellent gene suppression effect. In addition, the results indicate that introducing both a bulge structure and a linker into HDO can achieve both a reduction in central nervous system toxicity and an excellent gene suppression effect.

### <Example 19: Investigation of nucleic acid species to be introduced into gap region of second nucleic acid strand>

### (Purpose)

In terms of central nervous system toxicity exhibited when an HDO targeting the Malat1 gene is intraventricularly administered, the influence of the nucleic acid species introduced into a region is examined, wherein the region consists of a base sequence that is complementary, in the second nucleic acid strand, to the central region (gap region) of the first nucleic acid strand (the complementary region is hereinafter referred to as a "gap region" also in the second nucleic acid strand). Specifically, in the gap region of the second nucleic acid strand, toxicity is compared through an in vivo experiment among an HDO wherein all of the nucleosides are RNA nucleosides, an HDO wherein all of the nucleosides are DNA nucleosides, and an HDO wherein the RNA nucleosides are placed at the positions of an adenine base and a guanine base, and the DNA nucleosides are placed at the positions of a cytosine base and a thymine base.

### (Method)

The base sequences and chemical modifications of the ASO, and first nucleic acid strands and second nucleic acid strands constituting the HDO used in this Example are shown in Table 19.

**[Table 19]**

| Table 19: HDO targeting the Malat1 gene | | | |
|---|---|---|---|
| | | Sequence (5'-3') | SEQ ID NO |
| ASO | ASO | G^G^G^T^C(5)^a^g^c^t^g^c^c^a^a^t^G^C(5) ^T^A^G | 10 |
| HDO (all DNA) | ASO | G^G^G^T^C(5)^a^g^c^t^g^c^c^a^a^t^G^C(5) ^T^A^G | 10 |
| | c(all DNA) | **ctagcattggcagctgaccc** | 11 |
| HDO (cRNA 10MOE) | ASO | G^G^G^T^C(5)^a^g^c^t^g^c^c^a^a^t^G^C(5) ^T^A^G | 10 |
| | c(RNA 10MOE) | **C(5)TAGC(5)AUUGGCAGCUGAC(5)C(5)C(5)** | 57 |
| HDO (cDNA 10MOE) | ASO | G^G^G^T^C(5)^a^g^c^t^g^c^c^a^a^t^G^C(5) ^T^A^G | 10 |
| | c(DNA 10MOE) | **C(5)TAGC(5)attggcagctGAC(5)C(5)C(5)** | 13 |
| HDO (agRNA 10MOE) | ASO | G^G^G^T^C(5)^a^g^c^t^g^c^c^a^a^t^G^C(5) ^T^A^G | 10 |
| | c(agRNA 10MOE) | **C(5)TAGC(5)AttGGcAGctGAC(5)C(5)C(5)** | 58 |

| | | | |
|---|---|---|---|
| Lower case letter: DNA; Upper case letter: RNA; Underlined lower case letter: LNA(c(5) represents 5-methylcytosineLNA; Underlined upper case letter: 2'-O-MOE-RNA(C(5) represents 5-methylcytosine 2'-O-MOE-RNA; ^: Phosphorothioate linkage (PS linkage) | | | |

HDO (all DNA), HDO (cRNA 10MOE), HDO (cDNA 10MOE), and HDO (agRNA 10MOE) used in this Example comprise the above-described common ASO as the first nucleic acid strand, and the second nucleic acid strand has a sequence complementary to the first nucleic acid strand. Specifically, the second nucleic acid strand (c(all DNA)) of HDO (all DNA) has a structure wherein the DNA nucleosides are linked via a phosphodiester linkage. In the second nucleic acid strand c(RNA 10MOE) of HDO (cRNA 10MOE), a region consisting of a base sequence complementary to the wing region of the first nucleic acid strand (the complementary region is hereinafter referred to as a "wing region" also in the second nucleic acid strand) is composed of 2'-O-MOE-RNA nucleosides, and the gap region is composed of RNA nucleosides. In the second nucleic acid strand c(DNA 10MOE) of HDO (cDNA 10MOE), the wing region is composed of 2'-O-MOE-RNA nucleosides, and the gap region is composed of a DNA nucleoside structure. In the second nucleic acid strand c(agRNA 10MOE) of HDO (agRNA 10MOE), the wing region is composed of 2'-O-MOE-RNA nucleosides, and in the gap region, nucleosides having an adenine base and a guanine base are RNA nucleosides, and nucleosides having a cytosine base and a thymine base are DNA nucleosides.

For the nucleic acid molecules described in Table 19, preparation of nucleic acids, in vivo experiments, evaluation of central nervous system toxicity, and evaluation of motor function were performed by the same methods as in Example 1. In this Example, however, the amount of the nucleic acid agent administered per mouse was 14 nmol/mouse.

### (Results)

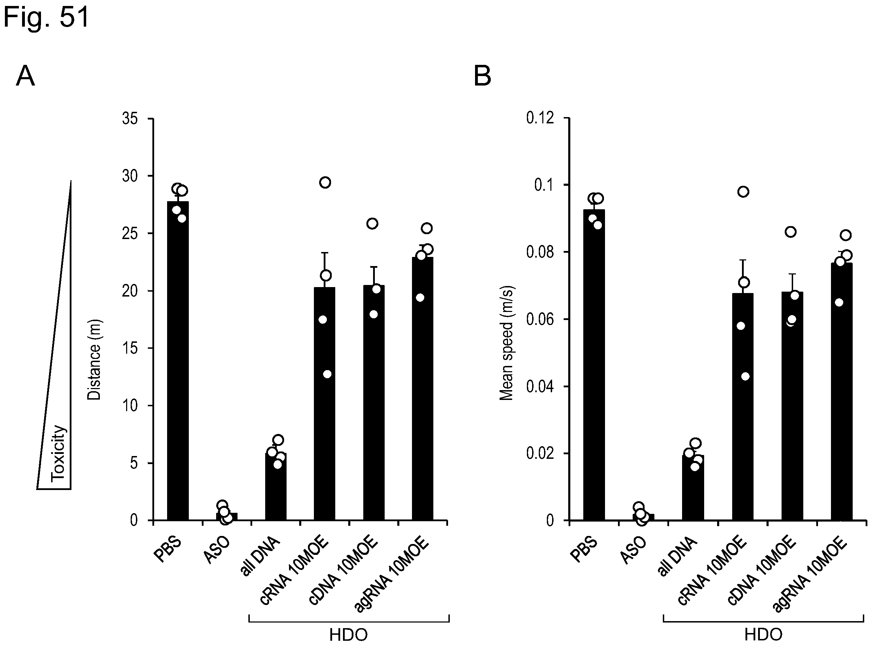

Figure 51 shows the results of evaluation of the motor function of mice at one hour after various nucleic acid agents were intraventricularly administered to the mice. The total movement distance (Figure 51A) and the average moving speed (Figure 51B) were improved noticeably in the administration groups of HDO (all DNA), HDO (cRNA 10MOE), HDO (cDNA 10MOE), and HDO (agRNA 10MOE), compared with the administration group of ASO. The total movement distance and the average moving speed were improved in the administration groups of HDO (cRNA 10MOE), HDO (cDNA 10MOE), and HDO (agRNA 10MOE), compared with the administration group of HDO (all DNA). Furthermore, the total movement distance and the average moving speed were improved in the administration groups of HDO (agRNA 10MOE), compared with the administration group of HDO (cRNA 10MOE) and HDO (cDNA 10MOE). This result has revealed that the HDO wherein the gap region of the second nucleic acid strand has RNA nucleosides at the positions of an adenine base and a guanine base, and has DNA nucleosides at the positions of a cytosine base and a thymine base can reduce central nervous system toxicity noticeably, compared with the single-stranded ASO and the other HDOs.

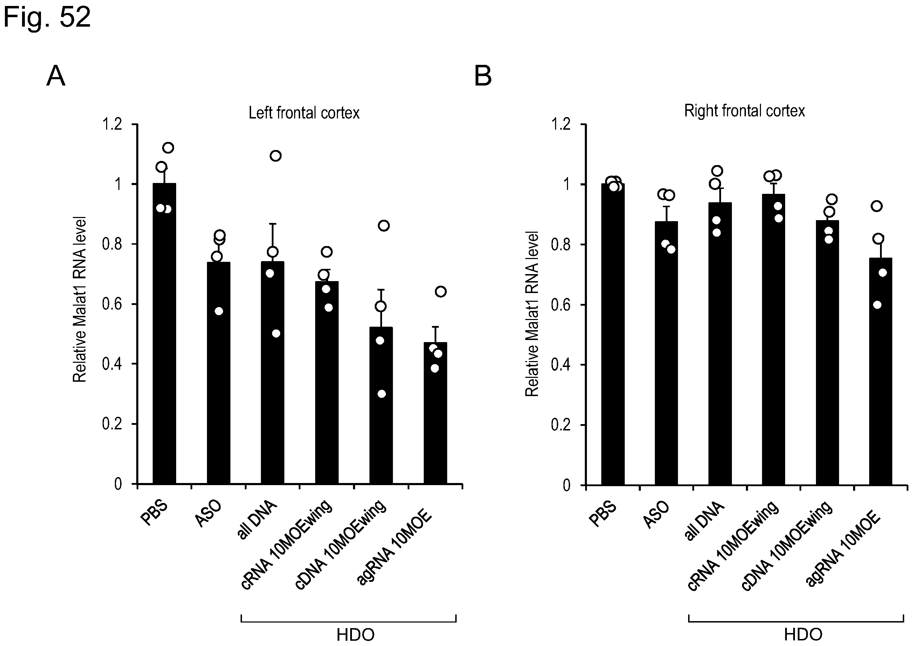

Figure 52 shows the Malat1 RNA expression level in the left and right frontal lobes at 7 days after the intraventricular administration of various nucleic acid agents. The administration group of HDO (agRNA 10MOE) exhibited an effect of suppressing the Malat1 RNA expression level noticeably, compared with the administration group of ASO and the administration group of the other HDOs. This result has revealed that the HDO comprising the second nucleic acid strand wherein the gap region has RNA nucleosides at the positions of an adenine base and a guanine base, and has DNA nucleosides at the positions of a cytosine base and a thymine base can achieve an excellent gene expression suppression effect, compared with the HDOs comprising the second nucleic acid strand wherein all the nucleosides in the gap region are DNA nucleosides or RNA nucleosides.

### <Example 20: Evaluation of double strand dissociation efficiency in brain tissue>

### (Purpose)

In a target tissue, the first nucleic acid strand can be dissociated from the second nucleic acid strand, and achieve an antisense effect on a target gene or a transcription product thereof. Accordingly, an HDO comprising the first nucleic acid strand and the second nucleic acid strand is incubated in the brain tissue homogenate of a mouse, and the double strand dissociation efficiency is examined.

### (Method)

### (1) Preparation of nucleic acids

In this Example, the same ASO, HDO (cRNA 10MOE), HDO (cDNA 10MOE), and HDO (agRNA 10MOE) as in Example 19 were used. The ASO and the HDOs were prepared by the same methods as in Example 19.

### (2) Evaluation of double-strand dissociation efficiency in brain tissue homogenate

For HDO (ASO/cRNA) and HDO (ASO/cDNA), each at 5.2 µL in an amount of 25 µM, 16.7 µL of a mouse brain homogenate solution and 28.1 µL of PBS were mixed, and the resulting mixture was incubated at 37°C in an incubator. After the incubation, protein kinase K was mixed in to terminate the reaction.

Acrylamide gel (1 × TBE) at 16% was prepared and produced. The sample in an amount of 9.6 µL was loaded to the gel, and electrophoresed at 100V for 120 minutes. As a control, an ASO alone was simultaneously electrophoresed. Then, a solution was produced by diluting, with 1 × TBE, an aqueous GelRed (× 10000) solution (Biotium) to a 1/10000 concentration. The gel was permeated with the solution for 10 minutes. Then, the gel was photographed with a ChemiDoc Touch imaging system (Bio-Rad Laboratories, Inc.).

### (Results)

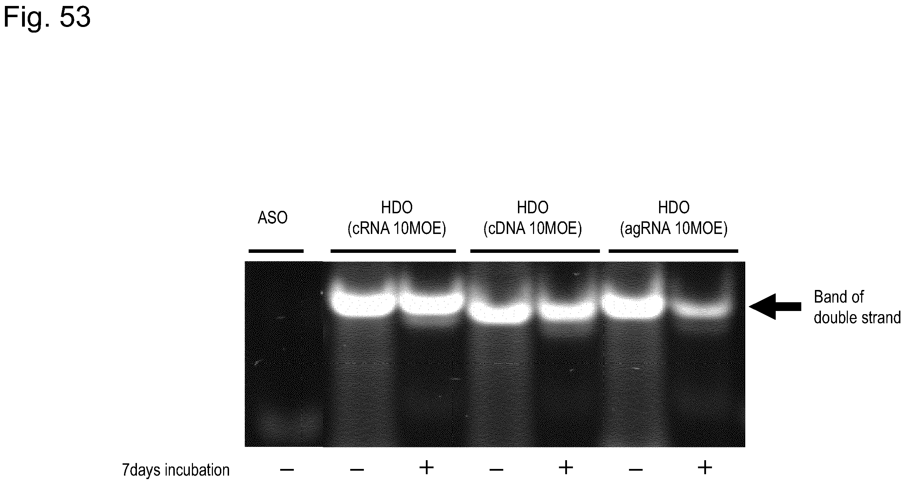

Figure 53 shows the results of electrophoresis performed after ASO, HDO (cRNA 10MOE), HDO (cDNA 10MOE) and HDO (agRNA 10MOE) were incubated in the mouse brain tissue homogenate for seven days. It has been revealed that, compared with HDO (cRNA 10MOE) and HDO (cDNA 10MOE), the double-stranded nucleic acids of HDO (agRNA 10MOE) (arrow in Figure 53) was noticeably decreased after the 7-day incubation, and has an extremely excellent double-strand separation capability in the brain tissue. Compared with the HDO comprising the second nucleic acid strand wherein all the nucleosides in the gap region are DNA nucleosides or RNA nucleosides, the HDO comprising the second nucleic acid strand wherein the gap region had RNA nucleosides at the positions of an adenine base and a guanine base, and had DNA nucleosides at the positions of a cytosine base and a thymine base had an extremely high double strand dissociation efficiency in the brain tissue, and strongly supported the result that the expression of a target gene was suppressed noticeably in the above-described Example 19.

### <Example 21: Investigation of nucleic acid modification to be introduced into wing region of second nucleic acid strand>

### (Purpose)

Through an in vivo experiment, the influence of the introduction of an MOE-modified nucleic acid or a natural nucleic acid (RNA or DNA) into the wing region and gap region of the second nucleic acid strand is compared in terms of central nervous system toxicity observed in intraventricular administration of the HDO targeting the Malat1 gene.

### (Method)

The base sequences and chemical modifications of the first nucleic acid strands and second nucleic acid strands constituting the HDOs used in this Example are shown in Table 20.

**[Table 20]**

| Table 20: HDO targeting the Malat1 gene | | | |
|---|---|---|---|
| | | Sequence (5'-3') | SEQ ID NO |
| HDO (all DNA) | ASO | G^G^G^T^C(5)^a^g^c^t^g^c^c^a^a^t^G^C (5)^T^A^G | 10 |
| | c(all DNA) | ctagcattggcagctgaccc | 12 |
| HDO (cRNA MOEwing) | ASO | G^G^G^T^C(5)^a^g^c^t^g^c^c^a^a^t^G^C (5)^T^A^G | 10 |
| | c(RNA MOEwing) | C(5)TAGC(5)AUUGGCAGCUGAC(5)C(5)C(5) | 57 |
| HDO (gapMOE RNA) | ASO | G^G^G^T^C(5)^a^g^c^t^g^c^c^a^a^t^G^C (5)^T^A^G | 10 |
| | c(gapMOE RNA) | CUAGCATTGGC(5)AGC(5)TGACCC | 59 |
| HDO (cDNA MOEwing) | ASO | G^G^G^T^C(5)^a^g^c^t^g^c^c^a^a^t^G^C (5)^T^A^G | 10 |
| | c(DNA MOE wing) | C(5)TAGC(5)attggcagctGAC(5)C(5)C(5) | 13 |
| HDO (gapDNA MOE) | ASO | G^G^G^T^C(5)^a^g^c^t^g^c^c^a^a^t^G^C (5)^T^A^G | 10 |
| | c(gapDNA MOE) | ctagcATTGGC(5)AGC(5)Tgaccc | 60 |
| HDO (agRNA MOEwing) | ASO | G^G^G^T^C(5)^a^g^c^t^g^c^c^a^a^t^G^C (5)^T^A^G | 10 |
| | c(agRNA MOEwing) | C(5)TAGC(5)AttGGcAGctGAC(5)C(5)C(5) | 58 |

| | | | |
|---|---|---|---|
| Lower case letter: DNA; Upper case letter: RNA; Underlined lower case letter: LNA(c(5) represents 5-methylcytosine LNA; Underlined upper case letter: 2'-O-MOE-RNA(C(5) represents 5-methylcytosine 2'-O-MOE-RNA; ^: Phosphorothioate linkage (PS linkage) | | | |

All of HDO (all DNA), HDO (cRNA MOEwing), HDO (gapMOE RNA), HDO (cDNA MOEwing), HDO (gapDNA MOE), and HDO (agRNA 1 0MOE) used in this Example comprise the ASO as the above-described common first nucleic acid strand, and the second nucleic acid strand has a sequence complementary to the first nucleic acid strand. Specifically, the second nucleic acid strand of HDO (all DNA) has a structure wherein the DNA nucleosides are linked via a phosphodiester linkage. In the second nucleic acid strand of HDO (cRNA MOEwing), the wing region is composed of 2'-O-MOE-RNA nucleosides, and the gap region is composed of an RNA nucleoside structure. In the second nucleic acid strand of HDO (gapMOE RNA), a region corresponding to the wing region is composed of RNA nucleosides, and a region corresponding to the gap region is composed of a 2'-O-MOE-RNA nucleoside structure. In the second nucleic acid strand of HDO (cDNA MOEwing), the wing region is composed of 2'-O-MOE-RNA nucleosides, and the gap region is composed of a DNA nucleoside structure. In the second nucleic acid strand of HDO (gapMOE DNA), a region corresponding to the wing region is composed of DNA nucleosides, and the gap region is composed of a 2'-O-MOE-RNA nucleoside structure. In the second nucleic acid strand of HDO (agRNA 10MOE), the wing region is composed of 2'-O-MOE-RNA nucleosides, and in the gap region, nucleosides having an adenine base and a guanine base are RNA nucleosides, and nucleosides having a cytosine base and a thymine base are DNA nucleosides.

For the nucleic acid molecules described in Table 21, preparation of nucleic acids, in vivo experiments, evaluation of central nervous system toxicity, and evaluation of motor function were performed by the same methods as in Example 1. In this Example, however, the amount of the nucleic acid agent administered per mouse was 28 nmol/mouse.

### (Results)

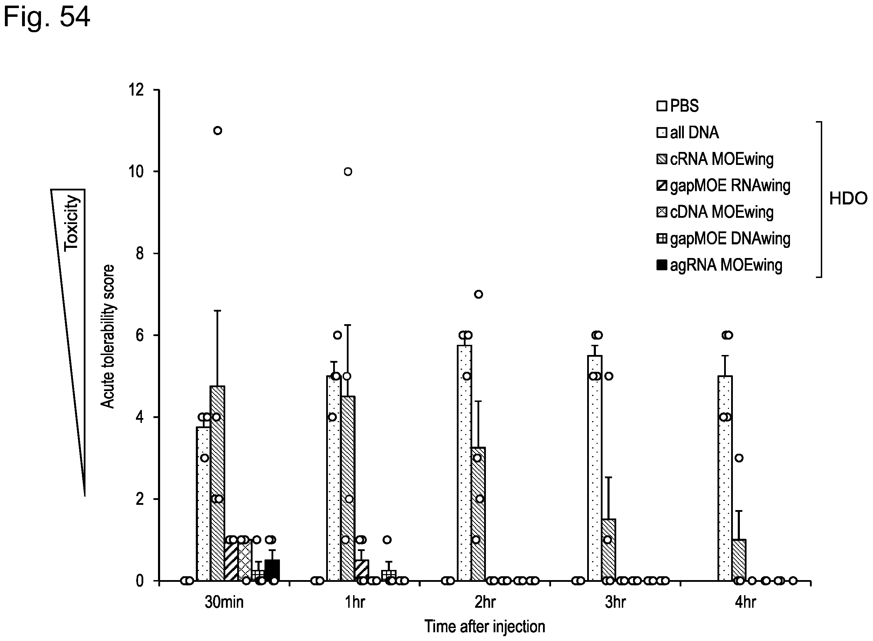

Figure 54 shows the results of evaluation of the central nervous system toxicity in mice to which various nucleic acid agents were intraventricularly administered. The acute tolerability score was decreased noticeably in the administration groups of HDO (cRNA MOEwing), HDO (gapMOE RNA), HDO (cDNA MOEwing), HDO (gapDNA MOE) and HDO (agRNA 10MOE), compared with the administration group of HDO (all DNA). The acute tolerability score was further decreased in the administration groups of HDO (gapMOE RNA), HDO (cDNA MOEwing), HDO (gapDNA MOE) and HDO (agRNA 10MOE), compared with the administration group of HDO (cDNA MOEwing).

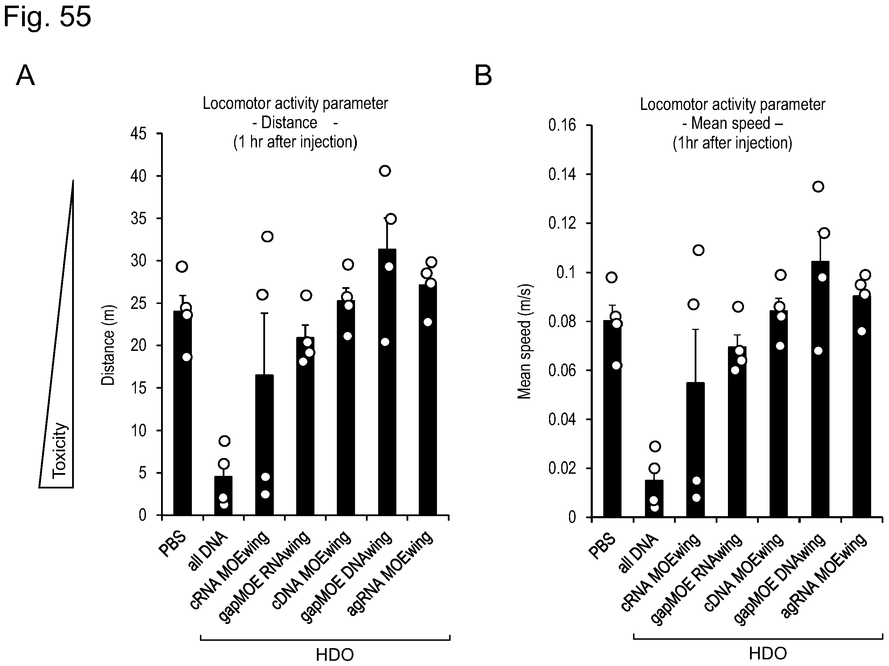

Figure 55 shows the results of evaluation of the motor function of mice at one hour after various nucleic acid agents were intraventricularly administered to the mice. The total movement distance (Figure 55A) and the average moving speed (Figure 55B) were improved noticeably in the administration groups of HDO (cRNA MOEwing), HDO (gapMOE RNA), HDO (cDNA MOEwing), HDO (gapDNA MOE) and HDO (agRNA 10MOE), compared with the administration group of HDO (all DNA). The total movement distance and the average moving speed were improved in the administration groups of HDO (gapMOE RNA), HDO (cDNA MOEwing), HDO (gapDNA MOE) and HDO (agRNA 10MOE), compared with the administration group of HDO (cDNA MOEwing).

This result has revealed that the HDOs wherein the second nucleic acid strand comprises an MOE modification in the wing region or the gap region can achieve reduced central nervous system toxicity, compared with the HDO wherein the second nucleic acid strand consists of DNA nucleosides.

### <Example 22: Investigation of nucleic acid species to be introduced into gap region of second nucleic acid strand: comparison of acute/delayed neurotoxicity>

### (Purpose)

Through an in vivo experiment, the toxicity-reducing effect is compared in terms of acute neurotoxicity observed within one day and delayed neurotoxicity observed one day or later after intraventricular administration of an HDO comprising: a first nucleic acid strand consisting of an ASO targeting the Mapt gene; and an MOE wing-modified second nucleic acid strand having a base sequence complementary to the first nucleic acid strand.

### (Method)

The base sequences and chemical modifications of the ASO, and first nucleic acid strands and second nucleic acid strands constituting the HDOs used in this Example are shown in Table 21.

**[Table 21]**

| Table 21: HDO targeting the Mapt gene | | | |
|---|---|---|---|
| | | Sequence (5'-3') | SEQ ID NO |
| ASO | | **a^t^a^g^t^c^a^c^t^c^t^g^g^t^g^a** | 1 |
| HDO (all DNA) | ASO | **a^t^a^g^t^c^a^c^t^c^t^g^g^t^g^a** | 1 |
| | c(all DNA) | **tcaccagagtgactat** | 3 |
| HDO (cRNA 6MOE) | ASO | **a^t^a^g^t^c^a^c^t^c^t^g^g^t^g^a** | 1 |
| | c(RNA 6MOE wing) | **TC(5)ACCAGAGUGACTAT** | 14 |
| HDO (cDNA 6MOE) | ASO | **a^t^a^g^t^c^a^c^t^c^t^g^g^t^g^a** | 1 |
| | c(6MOE wing) | **TC(5)AccagagtgacTAT** | 4 |
| HDO (agRNA 6MOE) | ASO | **a^t^a^g^t^c^a^c^t^c^t^g^g^t^g^a** | 1 |
| | c(agRNA 6MOE) | **TC(5)AccAGAGtGAcTAT** | 61 |

| | | | |
|---|---|---|---|
| Lower case letter: DNA; Upper case letter: RNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

HDO (all DNA), HDO (cRNA 6MOE), HDO (cDNA 6MOE), and HDO (agRNA 6MOE) used in this Example comprise the above-described ASO as the common first nucleic acid strand, and the second nucleic acid strand has a sequence complementary to the first nucleic acid strand. Specifically, the second nucleic acid strand (c(all DNA)) of HDO (all DNA) has a structure wherein the DNA nucleosides are linked via a phosphodiester linkage. The second nucleic acid strand of HDO (cRNA 6MOE) has a structure wherein three 2'-O-MOE-RNA nucleosides at the 5' end, three 2'-O-MOE-RNA nucleosides at the 3' end, and ten RNA nucleosides therebetween are linked via a phosphodiester linkage. The second nucleic acid strand of HDO (cDNA 6MOE) has a structure wherein three 2'-O-MOE-RNA nucleosides at the 5' end, three 2'-O-MOE-RNA nucleosides at the 3' end, and ten DNA nucleosides therebetween are linked via a phosphodiester linkage. The second nucleic acid strand of HDO (agRNA 6MOE) is composed of three 2'-O-Me-RNA nucleosides at the 5' end, three 2'-O-Me-RNA nucleosides at the 3' end, and a gap region therebetween, and in the gap region, nucleosides having an adenine base and a guanine base are RNA nucleosides, and nucleosides having a cytosine base and a thymine base are DNA nucleosides.

For ASO, HDO (all DNA), HDO (cRNA 6MOE), HDO (cDNA 6MOE), and HDO (agRNA 6MOE) described in Table 21, preparation of nucleic acids, in vivo experiments, evaluation of central nervous system toxicity, and evaluation of motor function were performed by the same methods as in Example 1. In this Example, however, the amount of the nucleic acid agent administered per mouse was 28 nmol/mouse.

### (Result 1: Acute neurotoxicity)

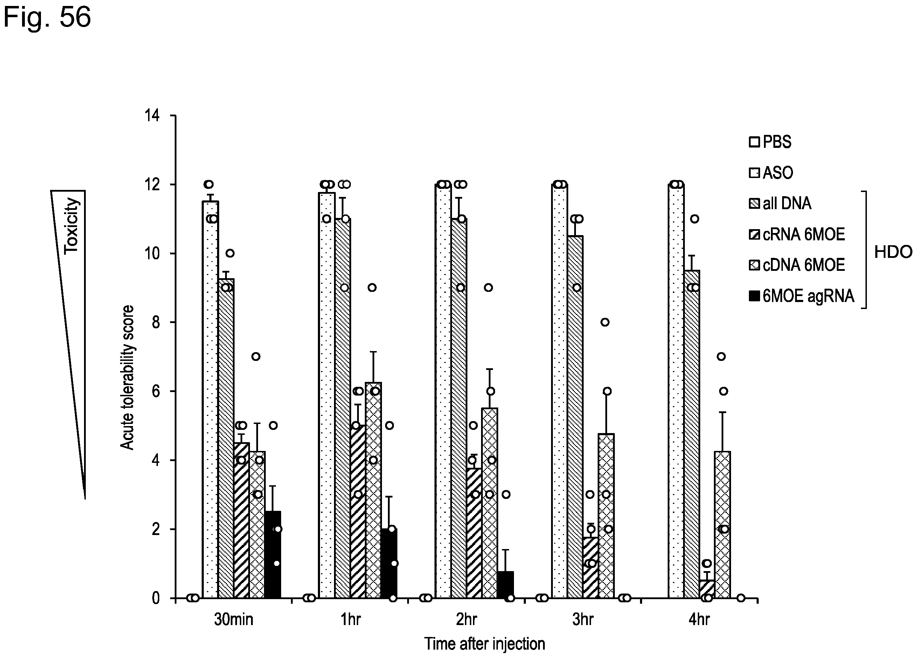

Figure 56 shows the results of evaluation of the acute central nervous system toxicity in mice one day after various nucleic acid agents were intraventricularly administered to the mice. At any point of time of 30 minutes, 1 hour, 2 hours, 3 hours, and 4 hours after the administration of the nucleic acid agent, the acute tolerability score was decreased noticeably in the administration groups of HDO (cRNA 6MOE), HDO (cDNA 6MOE), and HDO (agRNA 6MOE), compared with the administration groups of ASO and HDO (all DNA). Furthermore, the administration group of HDO (agRNA 6MOE) exhibited a lower acute tolerability score than the administration groups of HDO (cRNA 6MOE) and HDO (cDNA 6MOE). This result has revealed that the HDO wherein, in the gap region of the second nucleic acid strand, nucleosides having an adenine base and a guanine base are RNA nucleosides, and nucleosides having a cytosine base and a thymine base are DNA nucleosides can achieve an effect of reducing central nervous system toxicity noticeably, compared with the other HDOs.

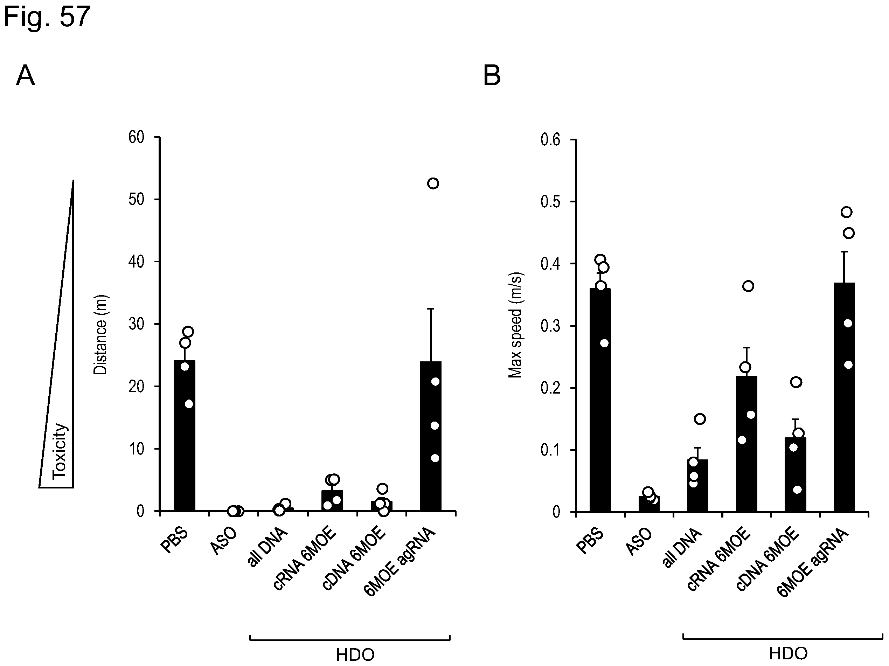

Figure 57 shows the results of evaluation of the motor function of mice at one hour after various nucleic acid agents were intraventricularly administered to the mice. The total movement distance (Figure 57A) and the maximum moving speed (Figure 57B) were improved in the administration groups of HDO (cRNA 6MOE), HDO (cDNA 6MOE), and HDO (agRNA 6MOE), compared with the administration groups of ASO and HDO (all DNA). Furthermore, the motor function was improved noticeably in the administration group of HDO (agRNA 6MOE), compared with the administration groups of HDO (cRNA 6MOE) and HDO (cDNA 6MOE). This result has revealed that the HDO wherein, in the gap region of the second nucleic acid strand, nucleosides having an adenine base and a guanine base are RNA nucleosides, and nucleosides having a cytosine base and a thymine base are DNA nucleosides has an extremely small influence on the motor function, and has extremely low toxicity, compared with the other HDOs.

### (Result 2: Delayed neurotoxicity)

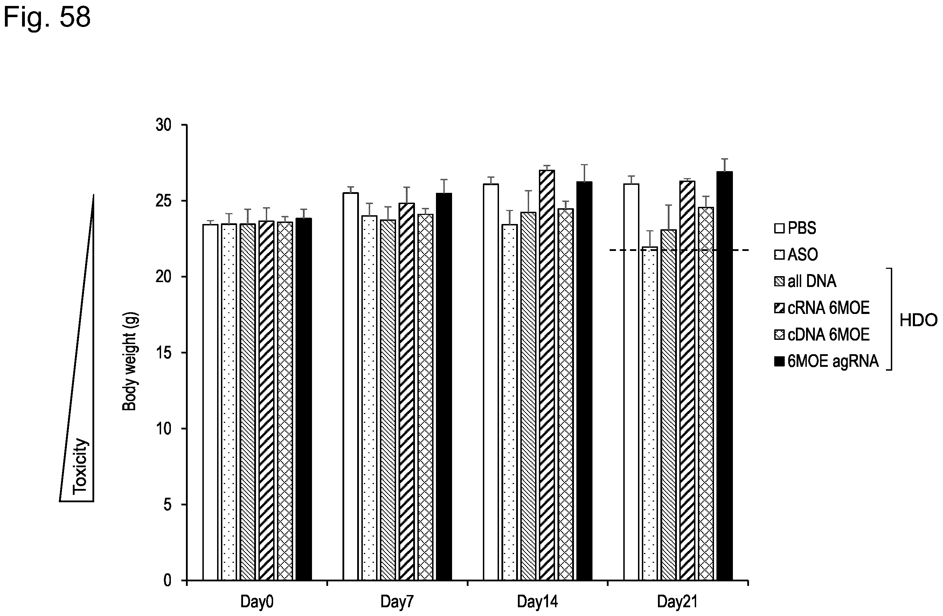

Figure 58 shows the results of evaluation of a decrease in the body weights of mice with respect to delayed central nervous system toxicity in the mice one day or later after various nucleic acid agents were intraventricularly administered to the mice. The body weights were measured 0, 7, 14, and 21 days after the administration, revealing that at any point of time of 7, 14, and 21 days after the administration, the administration groups of ASO and HDO (all DNA) exhibited a decrease in the body weight, compared with the administration group of PBS, and the administration groups of HDO (cRNA 6MOE), HDO (cDNA 6MOE), and HDO (agRNA 6MOE) exhibited reduction in the effect of reducing body weight, compared with the administration groups of ASO and HDO (all DNA).

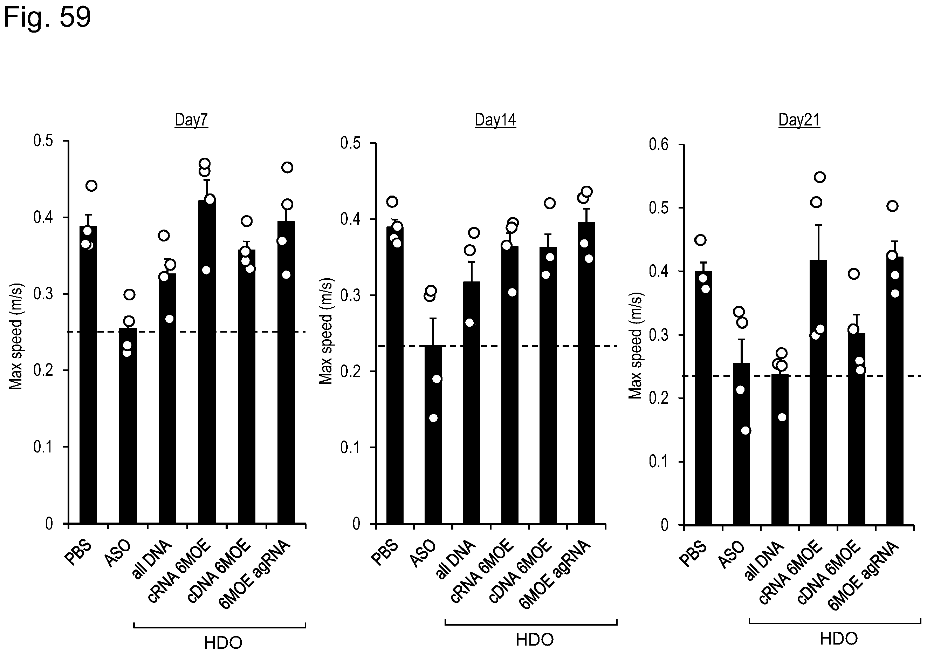

Figure 59 shows the results of evaluation of motor function of mice with respect to delayed central nervous system toxicity in the mice one day or later after various nucleic acid agents were intraventricularly administered to the mice. At any point of time of 7, 14, 21 days after the administration, the administration groups of HDO (cRNA 6MOE), HDO (cDNA 6MOE), and HDO (agRNA 6MOE) showed reduction in the effect of reducing the maximum moving speed, compared with the administration groups of ASO and HDO (all DNA).

### (Result 3: Target gene suppression effect)

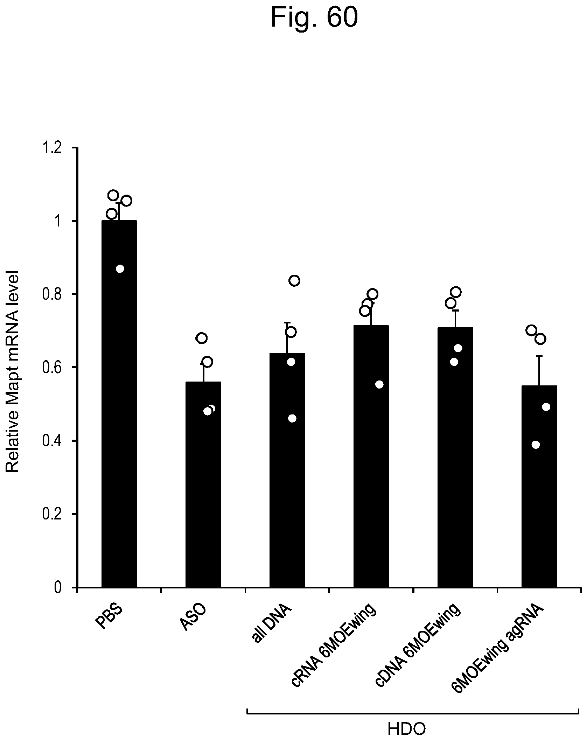

Figure 60 shows the Mapt mRNA expression level in the right frontal lobe 21 days after the intraventricular administration of various nucleic acid agents. The administration group of HDO (agRNA 6MOE) exhibited an effect of suppressing the Mapt mRNA expression level noticeably, compared with the administration groups of HDO (cRNA 6MOE) and HDO (cDNA 6MOE). This result has revealed that the HDO wherein, in the gap region of the second nucleic acid strand, nucleosides having an adenine base and a guanine base are RNA nucleosides, and nucleosides having a cytosine base and a thymine base are DNA nucleosides can achieve an excellent gene expression suppression effect, compared with the HDOs wherein the second nucleic acid strand consists of DNA nucleosides or RNA nucleosides.

### (Result 4: Evaluation of double strand dissociation efficiency in brain tissue homogenate)

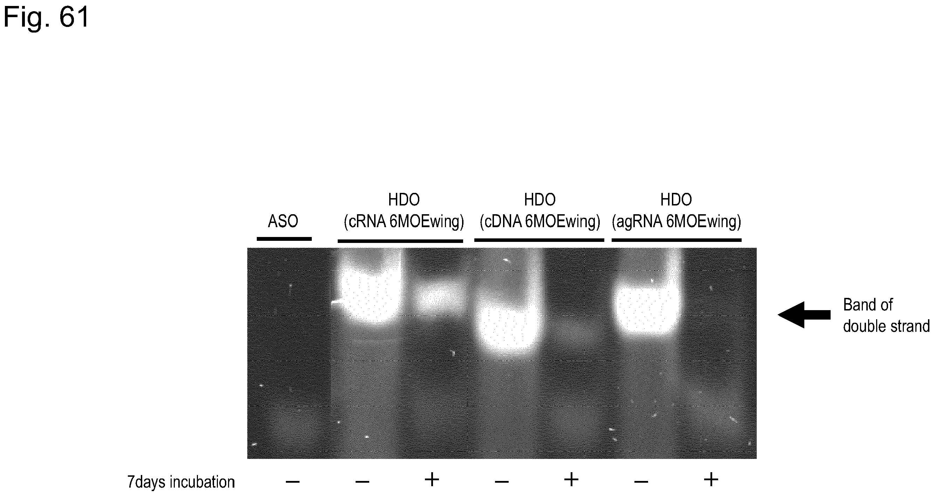

Figure 61 shows the results of electrophoresis performed after HDO (cRNA 6MOE), HDO (cDNA 6MOE), and HDO (agRNA 6MOE) were incubated in the mouse brain tissue for seven days, using the same method as in Example 20 for evaluation of the double strand dissociation efficiency of the nucleic acid agents in the brain tissue homogenate. HDO (agRNA 6MOE) exhibited a high double strand dissociation efficiency in the brain tissue homogenate, compared with HDO (cRNA 6MOE) and HDO (cDNA 6MOE). This result indicated that, compared with the HDO wherein the second nucleic acid strand consists of DNA nucleosides or RNA nucleoside, the HDO wherein, in the gap region of the second nucleic acid strand, nucleosides having an adenine base and a guanine base are RNA nucleosides, and nucleosides having a cytosine base and a thymine base are DNA nucleosides had an extremely high double strand dissociation efficiency in the brain tissue, and strongly supported the result that the expression of a target gene was noticeably suppressed in the above-described Result 3.

### <Example 23: Introduction of MOE modifications into the second nucleic acid strand of HDO comprising entirely MOE-modified ASO>

### (Purpose)

This Example is directed to an HDO comprising, as the first nucleic acid strand, an ASO that can control splicing for the Mecp2 gene as a target and in which all the nucleosides are composed of MOE-modified nucleosides (the ASO is hereinafter referred to as an "entirely MOE-modified ASO"). Through an in vivo experiment, it is investigated whether introducing an MOE modification into the second nucleic acid strand can reduce toxicity in terms of central nervous system toxicity observed in intraventricular administration of the HDO.

### (Method)

The base sequences and chemical modifications of the ASO, and first nucleic acid strands and second nucleic acid strands constituting the HDO used in this Example are shown in Table 22.

**[Table 22]**

| Table 22: ASO and HDO targeting the Mecp2 gene | | | |
|---|---|---|---|
| | | Sequence (5'-3') | SEQ ID NO |
| ASO | | **T^C(5)^T^A^C(5)^A^G^A^A^G^C(5)^A^A^G^G^T^G^T** | 62 |
| HDO (all DNA) | ASO | **T^C(5)^T^A^C(5)^A^G^A^A^G^C(5)^A^A^G^G^T^G^T** | 62 |
| | c(all DNA) | **acaccttgcttctgtaga** | 63 |
| HDO (cRDNA MOE) | ASO | **T^C(5)^T^A^C(5)^A^G^A^A^G^C(5)^A^A^G^G^T^G^T** | 62 |
| | c(cRDNA MOE) | **AC (5)AccttGcttctGtAGA** | 64 |

| | | | |
|---|---|---|---|
| Lower case letter: DNA; Upper case letter: RNA; Underlined lower case letter: LNA(c(5) represents 5-methylcytosine LNA; Underlined upper case letter: 2'-O-MOE-RNA(C(5) represents 5-methylcytosine 2'-O-MOE-RNA; ^: Phosphorothioate linkage (PS linkage) | | | |

The ASO used in this Example is a non-gapmer antisense nucleic acid targeting the MeCP2 (methyl-CpG binding protein 2) pre-mRNA, and has a base sequence complementary to part of the MECP2 pre-mRNA, and all of the nucleosides are 2'-O-MOE-RNA nucleosides linked via a phosphorothioate linkage.

HDO (all DNA) and HDO (cRDNA MOE) used in this Example comprise the above-described ASO as the common first nucleic acid strand, and the second nucleic acid strand has a sequence complementary to the first nucleic acid strand. Specifically, the second nucleic acid strand of HDO (all DNA) has a structure wherein the DNA nucleosides are linked via a phosphodiester linkage. The second nucleic acid strand of HDO (cRDNA MOE) is composed of three 2'-O-MOE-RNA nucleosides at the 5' end, three 2'-O-MOE-RNA nucleosides at the 3' end, and a gap region therebetween, and in the gap region, nucleosides having an adenine base and a guanine base are RNA nucleosides, and nucleosides having a cytosine base and a thymine base are DNA nucleosides.

For ASO, HDO (all DNA), and HDO (cRDNA MOE) described in Table 22, preparation of nucleic acids, in vivo experiments, evaluation of central nervous system toxicity, and evaluation of motor function were performed by the same methods as in Example 1. In this Example, however, the amount of the nucleic acid agent administered per mouse was 28 nmol/mouse.

### (Results)

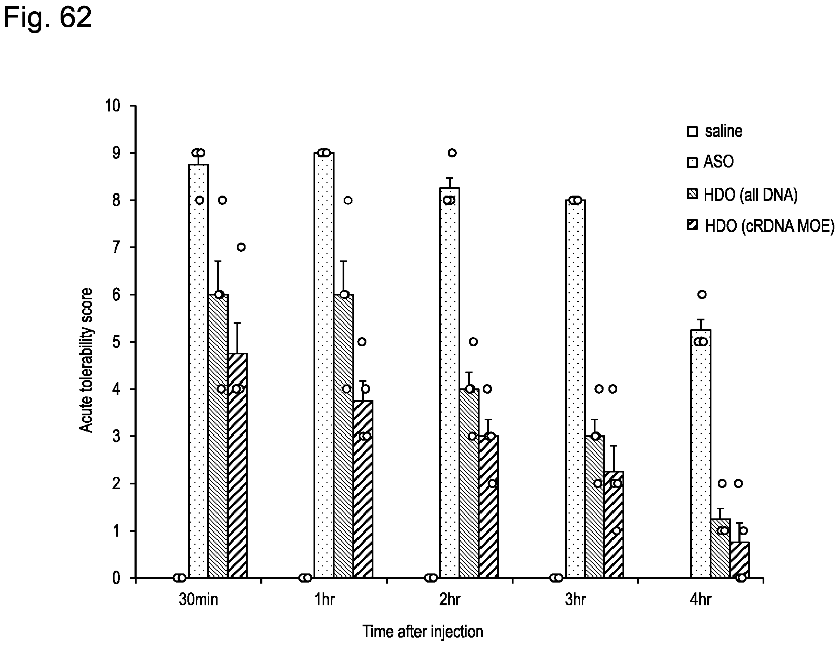

Figure 62 shows the results of evaluation of the acute central nervous system toxicity in mice to which various nucleic acid agents were intraventricularly administered. At any point of time of 30 minutes, 1 hour, 2 hours, 3 hours, and 4 hours after the administration of the nucleic acid agent, the acute tolerability score was decreased noticeably in the administration groups of HDO (all DNA) and HDO (cRDNA MOE), compared with the administration group of ASO. Furthermore, the administration group of HDO (cRDNA MOE) exhibited a lower acute tolerability score than the administration group of HDO (all DNA). This result has revealed that the HDO wherein the wing region of the second nucleic acid strand is composed of 2'-O-MOE-RNA nucleosides, and wherein, in the gap region, nucleosides having an adenine base and a guanine base are RNA nucleosides, and nucleosides having a cytosine base and a thymine base are DNA nucleosides can achieve low central nervous system toxicity, compared with the other HDOs.

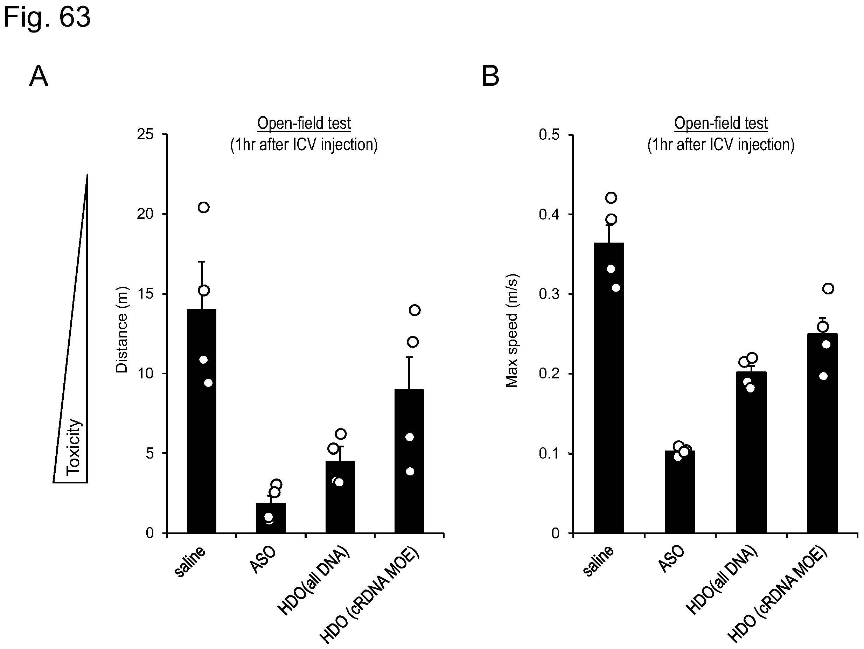

Figure 63 shows the results of evaluation of the motor function of mice at one hour after various nucleic acid agents were intraventricularly administered to the mice. The total movement distance (Figure 63A) and the maximum moving speed (Figure 63B) were improved in the administration groups of HDO (all DNA) and HDO (cRDNA MOE), compared with the administration group of ASO. Furthermore, the total movement distance and the maximum moving speed were improved in the administration groups of HDO (cRDNA MOE), compared with the administration group of HDO (all DNA). This result has revealed that the HDO wherein the wing region of the second nucleic acid strand is composed of 2'-O-MOE-RNA nucleosides, and wherein, in the gap region, nucleosides having an adenine base and a guanine base are RNA nucleosides, and nucleosides having a cytosine base and a thymine base are DNA nucleosides has an extremely small effect for suppressing the motor function, and has extremely low toxicity, compared with the other HDOs.

### <Example 24: Single-stranded HDO>

### (Purpose)

Through an in vitro experiment, the neuronal cell toxicity of a single-stranded HDO (ssHDO) in which the first nucleic acid strand consisting of an ASO targeting the Bace1 gene and the second nucleic acid strand having a base sequence complementary to the first nucleic acid strand are linked via a linker is investigated.

### (Method)

### (1) Preparation of nucleic acids

The base sequences and chemical modifications of HDOs and ssHDO used in this Example are shown in Table 23.

**[Table 23]**

| Table 23: ASO and HDO targeting the BACE1 gene | | | |
|---|---|---|---|
| | | Sequence (5'-3') | SEQ ID NO |
| (all DNA) | ASO | **g^t^a^t^t^g^c^t^g^a^g^g^a** | 6 |
| | c(all DNA) | **tcctcagcaatac** | 8 |
| ssHDO | | TC(5)C(5)aaaTC(5)AGC(5)AAcccTAC(5)aaaa g^t^a^t^t^g^c^t^g^a^g^g^a | 65 |

| | | | |
|---|---|---|---|
| Lower case letter: DNA; Upper case letter: RNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

### (2) Evaluation of gene suppression effect

The HDO prepared in (1) was introduced into human neuroblastoma-derived cells (Neuro 2a cell line) using a lipofection method (lipofectamine 2000).

Using an IsogenI kit (GeneDesign Inc.), RNA was extracted from the cell at 48 hours after the introduction of the HDO. A cDNA was synthesized using Transcriptor Universal cDNA Master, DNase (Roche Diagnostics) in accordance with the protocol. Next, the resulting cDNA template was used to perform quantitative RT-PCR, whereby the expression levels of Bace1 mRNA and Actb mRNA (the internal standard gene) were measured. Quantitative RT-PCR was performed with TaqMan (Roche Applied Science). Primers used in quantitative RT-PCR were products designed and manufactured by Thermo Fisher Scientific Inc. (formerly known as Life Technologies Corp.). Amplification was performed by repeating, 40 times, the following cycle: 95°C for 15 seconds, 60°C for 30 seconds, and 72°C for 1 second.

The ratio of the expression level of Bace1 mRNA to the expression level of Actb mRNA (internal standard gene) was calculated, and a value standardized with respect to the value of the PBS-treated group was determined as a relative Bace1 mRNA level.

### (3) Evaluation of cell toxicity

To evaluate the neuronal cell toxicity 48 hours after the introduction, the lactate dehydrogenase (LDH) activity in the cell supernatant was measured. The LDH activity was measured using Cytotoxicity LDH Assay Kit-WST (Dojindo Laboratories) in accordance with the attached protocol. A value standardized with respect to the LDH activity of a PBS-treated group was determined as a relative LDH release level.

### (Results)

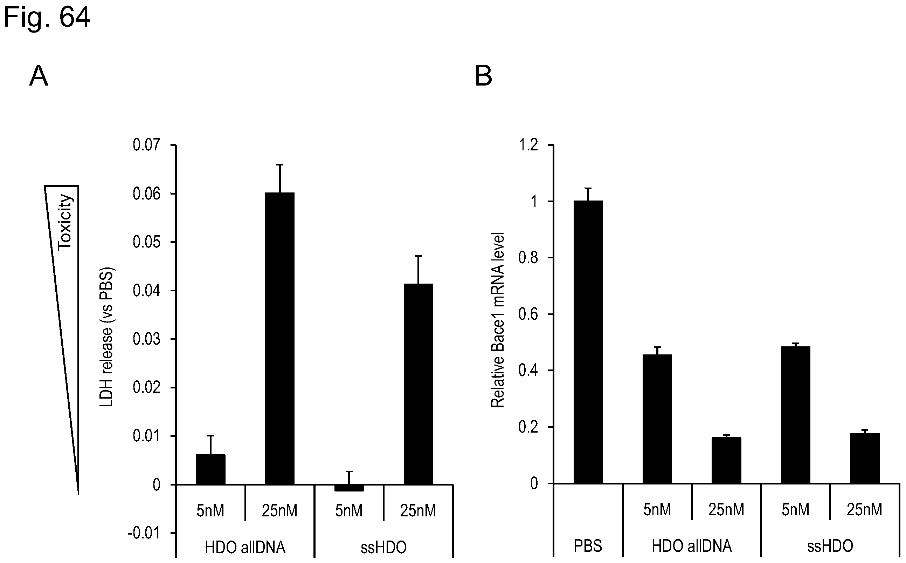

Figure 64B shows the results of evaluation of the target gene suppression effect of various nucleic acid agents in mouse neuroblastoma-derived cells (Neuro 2a cell line). Strong gene suppression effect which is comparable to those in the administration group of HDO (all DNA) was produced in the administration group of ssHDO.

Figure 64A shows the results of evaluation of the LDH activity in the supernatant as the cell toxicity when various nucleic acid agents were introduced into the cell. An increase in the LDH activity was suppressed, and the cell toxicity was decreased noticeably, in the cells to which ssHDO was administered at 5 nM and 25 nM, compared with the cells to which HDO (all DNA) was administered at 5 nM and 25 nM. This result has revealed that ssHDO comprising a 2'-MOE-RNA nucleoside achieves a noticeable decrease in the cell toxicity.

The above-described results have revealed that ssHDO achieves both a reduction in neurotoxicity and a strong gene suppression effect.

### <Example 25: Evaluation of central nervous system toxicity in monkey>

### (Purpose)

An HDO comprising MOE modifications is intrathecally administered to the monkeys, and the central nervous system toxicity is evaluated,.

### (Method)

### (1) Preparation of nucleic acids

The base sequences and chemical modifications of the ASO, and first nucleic acid strands and second nucleic acid strands constituting an HDO used in this Example are shown in Table 24.

**[Table 24]**

| Table 24: ASO and HDO targeting the Mapt gene | | | |
|---|---|---|---|
| | | Sequence (5'-3') | SEQ ID NO |
| ASO | | **a^t^a^g^t^c^a^c^t^c^t^g^g^t^g^a** | 1 |
| HDO (all DNA) | ASO | **a^t^a^g^t^c^a^c^t^c^t^g^g^t^g^a** | 1 |
| | c(all DNA) | **tcaccagagtgactat** | 3 |
| HDO (DNA MOE) | ASO | **a^t^a^g^t^c^a^c^t^c^t^g^g^t^g^a** | 1 |
| | c(DNA MOE) | **TC(5)AccagagtgacTAT** | 4 |
| HDO (RNA MOE) | ASO | **a^t^a^g^t^c^a^c^t^c^t^g^g^t^g^a** | 1 |
| | c(RNA MOE) | **TC(5)ACCAGAGUGACTAT** | 14 |
| HDO (DNARNA MOE) | ASO | **a^t^a^g^t^c^a^c^t^c^t^g^g^t^g^a** | 1 |
| | c(DNARNA MOE) | **TC(5)AccAGAGtGAcTAT** | 61 |

| | | | |
|---|---|---|---|
| Lower case letter: DNA; Upper case letter: RNA; Underlined lower case letter: LNA; Underlined upper case letter: 2'-O-MOE-RNA(C(5) represents 5-methylcytosine 2'-O-MOE-RNA; ^: Phosphorothioate linkage (PS linkage) | | | |

For ASO, HDO (all DNA), HDO (DNA MOE), HDO (RNA MOE), and HDO (DNARNA MOE) described in Table 24, nucleic acids were prepared by the same method as in Example 1.

### (2) Evaluation of central nervous system toxicity in monkey

To two male cynomolgus monkeys (3 years old, 2 kg), ASO, HDO (all DNA), HDO (DNA MOE), HDO (RNA MOE), and HDO (DNARNA MOE) were administered. In the administration method, 0.94 µmol of ASO or HDO was intrathecally administered by lumbar puncture.

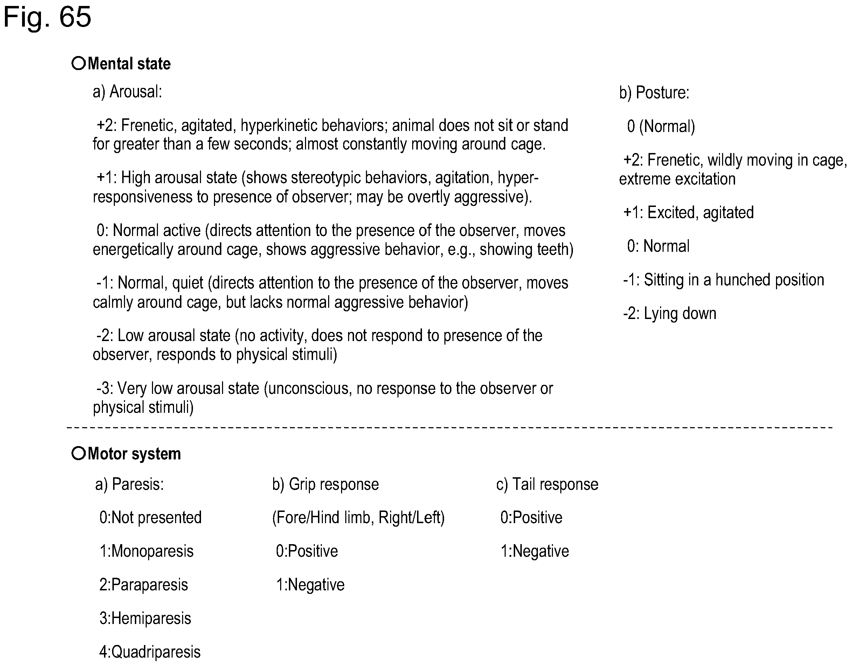

At 1 hour, 2 hours, and 3 hours after the intrathecal administration, the behaviors of the monkeys were evaluated by blinded evaluators on the basis of the modified FOB scores shown in Figure 65 for evaluation of central nervous system toxicity and on the basis of a three-minute video for measuring the spontaneous locomotion time or the number of jumps.

### (Results)

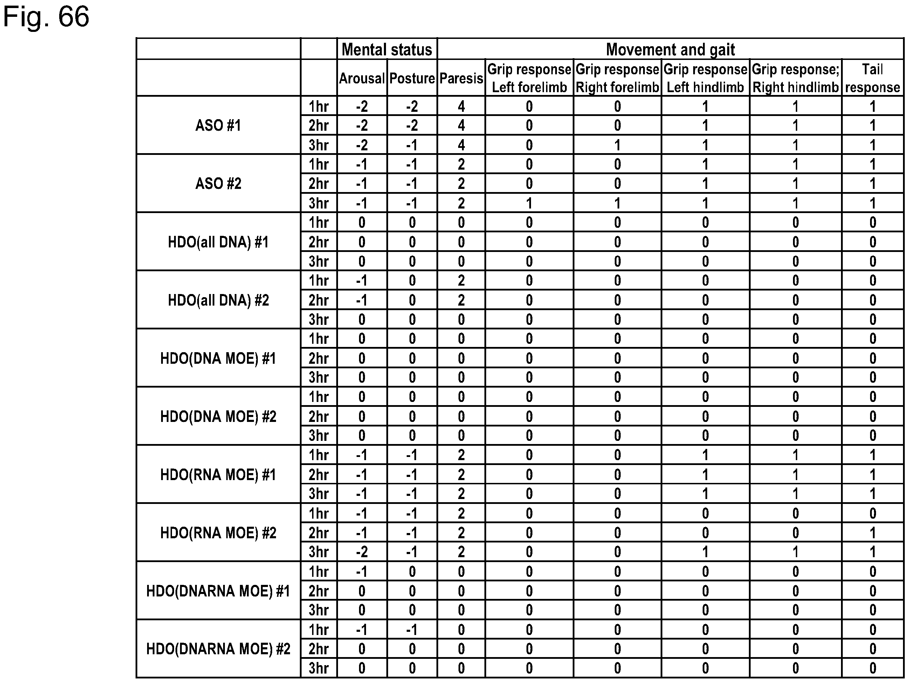

Figure 66 shows the results of evaluation of the acute central nervous system toxicity in monkeys to which various nucleic acid agents were intrathecally administered. At any point of time of 1 hour, 2 hours, and 3 hours after the administration of the nucleic acid agent, the modified FOB scores were decreased noticeably in the administration groups of HDO (all DNA), HDO (DNA MOE), HDO (RNA MOE), and HDO (DNARNA MOE), compared with the administration group of ASO. The modified FOB scores were decreased in the administration groups of HDO (DNA MOE) and HDO (DNARNA MOE), compared with the administration group of HDO (all DNA).

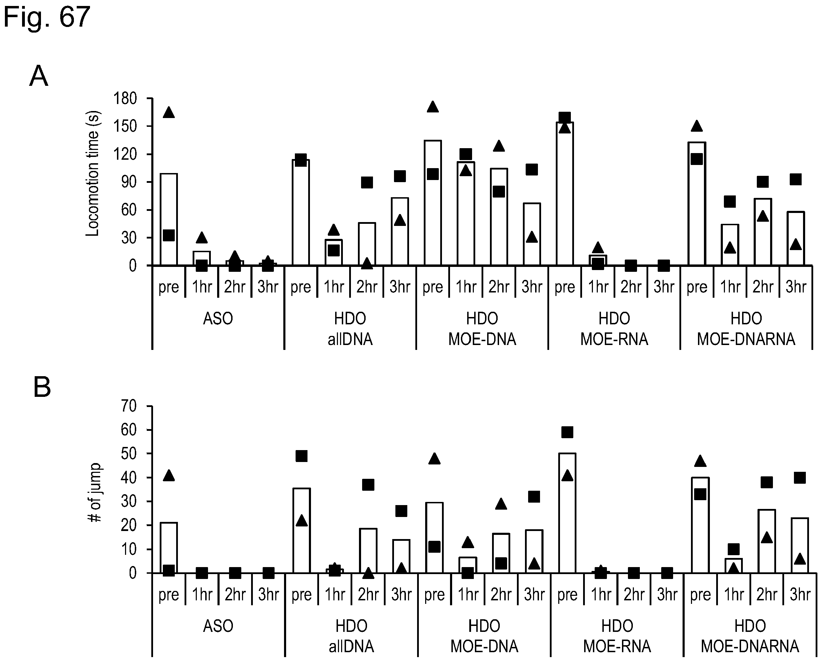

In addition, the results of a three-minute video for measuring the spontaneous locomotion time and the number of jumps are shown in Figure 67. The effect of decreasing the spontaneous locomotion time and the number of jumps was suppressed in the administration groups of HHDO (all DNA), HDO (DNA MOE), HDO (RNA MOE), and HDO (DNARNA MOE), compared with the administration group of ASO. Furthermore, the effect of decreasing the spontaneous locomotion time and the number of jumps was suppressed in the administration groups of HDO (DNA MOE) and HDO (DNARNA MOE), compared with the administration group of HDO (all DNA).

All publications, patents, and patent applications cited herein are incorporated herein by reference in their entirety.

## Claims

1. A double-stranded nucleic acid complex comprising a first nucleic acid strand and a second nucleic acid strand, wherein:
said first nucleic acid strand is capable of hybridizing to at least part of a target gene or a transcription product thereof, and has an antisense effect on said target gene or a transcription product thereof, and
said second nucleic acid strand comprises a base sequence complementary to said first nucleic acid strand, and comprises one or more 2'-modified nucleoside.

2. The double-stranded nucleic acid complex of claim 1, wherein said first nucleic acid strand is gapmer.

3. The double-stranded nucleic acid complex of claim 1, wherein all of nucleosides in said first nucleic acid strand are modified nucleosides.

4. The double-stranded nucleic acid complex of claim 3, wherein all of nucleosides in said first nucleic acid strand are 2'-modified nucleosides.

5. The double-stranded nucleic acid complex of claim 4, wherein all of nucleosides in said first nucleic acid strand are 2'-O-methoxyethyl-modified nucleosides.

6. The double-stranded nucleic acid complex of claim 1, wherein said second nucleic acid strand comprises one or consecutive two to ten 2'-modified nucleosides at the 5' end, and/or one or consecutive two to ten 2'-modified nucleosides at the 3' end.

7. The double-stranded nucleic acid complex of claim 1, wherein said second nucleic acid strand comprises one to seven 2'-modified nucleosides at positions other than 5' end and 3' end.

8. The double-stranded nucleic acid complex of claim 2, wherein said first nucleic acid strand comprises:
(1) a central region which comprises at least four consecutive deoxyribonucleosides,
(2) a 5'-wing region which comprises a non-natural nucleoside, positioned on the 5' end side of said central region, and
(3) a 3'-wing region which comprises a non-natural nucleoside, positioned on the 3' end side of said central region.

9. The double-stranded nucleic acid complex of claim 8, wherein said second nucleic acid strand comprises a 2'-modified nucleoside in a region which consists of a base sequence complementary to the 5'-wing region and/or the 3'-wing region in said first nucleic acid strand.

10. The double-stranded nucleic acid complex of claim 9, wherein all of the nucleosides in a region in said second nucleic acid strand which consists of a base sequence complementary to the 5'-wing region and/or the 3'-wing region in said first nucleic acid strand are 2'-modified nucleosides.

11. The double-stranded nucleic acid complex of claim 10, wherein all of the nucleosides which comprise a purine base in a region in said second nucleic acid strand which consists of a base sequence complementary to the central region in said first nucleic acid strand are ribonucleosides.

12. The double-stranded nucleic acid complex of claim 11, wherein all of the nucleosides which comprise a pyrimidine base in a region in said second nucleic acid strand which consists of a base sequence complementary to the central region in said first nucleic acid strand are deoxyribonucleosides.

13. The double-stranded nucleic acid complex of claim 12, wherein said 2'-modified nucleosides in said second nucleic acid strand are 2'-O-methoxyethyl-modified nucleosides or 2'-O-methyl-modified nucleosides.

14. The double-stranded nucleic acid complex of claim 8, wherein all of nucleosides in a region in said second nucleic acid strand which consists of a base sequence complementary to the central region in said first nucleic acid strand are:
(a) deoxyribonucleosides,
(b) deoxyribonucleosides and ribonucleosides,
(c) deoxyribonucleosides and 2'-modified nucleosides,
(d) ribonucleosides and 2'-modified nucleosides, or
(e) deoxyribonucleosides, ribonucleosides, and 2'-modified nucleosides.

15. The double-stranded nucleic acid complex of claim 14, wherein, in said second nucleic acid strand,
all of nucleosides in the regions which consist of a base sequence complementary to the 5'-wing region and the 3'-wing region in said first nucleic acid strand are 2'-modified nucleosides, and
all of nucleosides in the region which consists of a base sequence complementary to the central region in said first nucleic acid strand are deoxyribonucleosides.

16. The double-stranded nucleic acid complex of claim 8, wherein said second nucleic acid strand comprises at least four consecutive ribonucleosides complementary to the at least four consecutive deoxyribonucleosides in said central region in said first nucleic acid strand.

17. The double-stranded nucleic acid complex of claim 1, wherein the nucleosides in said second nucleic acid strand which are complementary to: (i) at least one guanosine nucleoside in said first nucleic acid strand,
(ii) the nucleoside which is adjacent to said guanosine nucleoside at the 5' end side,
(iii) the nucleoside which is adjacent to said guanosine nucleoside at the 3' end side, or
(iv) any combination of (i) to (iii)
are 2'-modified nucleosides.

18. The double-stranded nucleic acid complex of claim 1, wherein the nucleosides in said second nucleic acid strand which are complementary to: (i) at least one guanosine nucleoside in the 5'-wing region of said first nucleic acid strand,
(ii) the nucleoside which is adjacent to said guanosine nucleoside at the 5' end side,
(iii) the nucleoside which is adjacent to said guanosine nucleoside at the 3' end side, or
(iv) any combination of (i) to (iii)
are 2'-modified nucleosides.

19. The double-stranded nucleic acid complex of claim 1, wherein at least one nucleoside comprising adenine base or pyrimidine base in said second nucleic acid strand is a 2'-modified nucleoside and/or deoxyribonucleoside.

20. The double-stranded nucleic acid complex of claim 1, wherein at least one nucleoside comprising pyrimidine base in said second nucleic acid strand is 2'-modified nucleoside and/or deoxyribonucleoside.

21. The double-stranded nucleic acid complex of claim 1, wherein said second nucleic acid strand does not comprise a natural ribonucleoside comprising pyrimidine base.

22. The double-stranded nucleic acid complex of claim 18, wherein all of the nucleosides comprising pyrimidine base in said second nucleic acid strand are 2'-modified nucleosides and/or deoxyribonucleosides.

23. The double-stranded nucleic acid complex of claim 1, wherein 20% or more of nucleosides in said second nucleic acid strand are 2'-modified nucleosides.

24. The double-stranded nucleic acid complex of claim 1, wherein all of the nucleosides other than 2'-modified nucleosides in said second nucleic acid strand are deoxyribonucleosides.

25. The double-stranded nucleic acid complex of claim 1, wherein all of the nucleosides in a region in said second nucleic acid strand which consists of a base sequence complementary to the first nucleic acid strand are 2'-modified nucleosides.

26. The double-stranded nucleic acid complex of claim 1, wherein said 2'-modified nucleosides in said second nucleic acid strand are 2'-O-methoxyethyl-modified nucleosides and/or 2'-O-methyl-modified nucleosides.

27. The double-stranded nucleic acid complex of claim 1, wherein said second nucleic acid strand comprises at least one 2'-O-methyl-modified nucleoside, scpBNA nucleoside, AmNA nucleoside, or BNA-NC.

28. The double-stranded nucleic acid complex of claim 1, wherein said first nucleic acid strand is mixmer.

29. The double-stranded nucleic acid complex of claim 1, wherein said second nucleic acid strand comprises at least one bulge structure consisting of a base sequence not complementary to said first nucleic acid strand.

30. The double-stranded nucleic acid complex of claim 1, wherein said second nucleic acid strand comprises a non-complementary base, and/or an insertion sequence and/or a deletion of one or more bases, with respect to said first nucleic acid strand.

31. The double-stranded nucleic acid complex of claim 27, wherein said second nucleic acid strand comprises one to three said non-complementary bases.

32. The double-stranded nucleic acid complex of claim 30, wherein said insertion sequence consists of 1 to 8 bases.

33. The double-stranded nucleic acid complex of claim 30, wherein said deletion consists of consecutive 1 to 4 bases.

34. The double-stranded nucleic acid complex of claim 30, wherein said non-complementary base in said second nucleic acid strand forms a bulge structure, or wherein said second nucleic acid strand comprises, at the position of said deletion, a bulge structure consisting of a base sequence which is not complementary to said first nucleic acid strand.

35. The double-stranded nucleic acid complex of claim 29, wherein said bulge structure comprises a sugar-unmodified nucleoside, or all of nucleosides in said bulge structure are sugar-unmodified nucleosides.

36. The double-stranded nucleic acid complex of claim 29, wherein said bulge structure is 1 to 10 bases in length.

37. The double-stranded nucleic acid complex of claim 29, wherein, in said second nucleic acid strand, all of the nucleosides other than said bulge structure are 2'-modified nucleosides.

38. The double-stranded nucleic acid complex of claim 1, wherein said second nucleic acid strand is at least 8 bases in length.

39. The double-stranded nucleic acid complex of claim 1, wherein the base length of said second nucleic acid strand is shorter than the base length of the first nucleic acid strand.

40. The double-stranded nucleic acid complex of claim 1, wherein said second nucleic acid strand comprises at least one overhang region on the 5' end side and/or 3' end side of a region consisting of a base sequence complementary to said first nucleic acid strand.

41. The double-stranded nucleic acid complex of claim 40, wherein said overhang region is 1 to 20 bases in length.

42. The double-stranded nucleic acid complex of claim 40, wherein said overhang region comprises at least one deoxyribonucleoside and/or non-natural nucleoside.

43. The double-stranded nucleic acid complex of claim 1, wherein said first nucleic acid strand and said second nucleic acid strand are bound via a linker.

44. The double-stranded nucleic acid complex of claim 43, wherein:
the 5' end of said first nucleic acid strand and the 3' end of said second nucleic acid strand, and/or
the 3' end of said first nucleic acid strand and the 5' end of said second nucleic acid strand are bound by said linker.

45. The double-stranded nucleic acid complex of claim 43, wherein said linker is a cleavable or uncleavable linker.

46. The double-stranded nucleic acid complex of claim 43, wherein said linker consists of nucleic acids comprising natural nucleosides and/or non-natural nucleosides, or polyethylene glycol.

47. The double-stranded nucleic acid complex of claim 46, wherein said linker consisting of nucleic acids are 2 to 10 bases in length.

48. The double-stranded nucleic acid complex of claim 1, wherein said second nucleic acid strand is bound to a ligand.

49. The double-stranded nucleic acid complex of claim 48, wherein said ligand is any one or more selected from the group consisting of a small molecule, a peptide, a lipid, and a nucleic acid aptamer.

50. The double-stranded nucleic acid complex of claim 49, wherein said lipid is cholesterol or an analog thereof, or tocopherol or an analog thereof.

51. The double-stranded nucleic acid complex of claim 45, wherein said ligand is bound to the 5' end and/or 3' end of said second nucleic acid strand.

52. The double-stranded nucleic acid complex of claim 1, which is not bound to a ligand.

53. The double-stranded nucleic acid complex of claim 1, wherein all or a part of the internucleoside linkages in said first nucleic acid strand and/or said second nucleic acid strand are modified internucleoside linkages.

54. The double-stranded nucleic acid complex of claim 53, wherein said modified internucleoside linkage is a phosphorothioate linkage.

55. A pharmaceutical composition comprising the double-stranded nucleic acid complex of any one of claims 1 to 54 as an active ingredient.

56. The pharmaceutical composition of claim 55 for treating central nervous system disease in a subject.

57. The pharmaceutical composition of claim 55, wherein the pharmaceutical composition is administered by intraventricular administration or intrathecal administration.

58. The pharmaceutical composition of claim 57, wherein said intrathecal administration is suboccipital puncture or lumbar puncture.

59. The pharmaceutical composition of claim 57, wherein 0.1 mg to 200 mg of said double-stranded nucleic acid complex is administered.

60. The pharmaceutical composition of claim 55, wherein the pharmaceutical composition is administered by intravenous administration or subcutaneous administration.

61. The pharmaceutical composition of claim 60, wherein said double-stranded nucleic acid complex is administered at 0.1 mg/kg to 100 mg/kg.

62. The pharmaceutical composition of claim 55, wherein central nervous system toxicity is reduced.
